(19) Europäisches Patentamt
European Patent Office
Office européen des brevets

(11) EP 3 810 608 B1

(12) EUROPEAN PATENT SPECIFICATION

(45) Date of publication and mention of the grant of the patent:
**10.08.2022 Bulletin 2022/32**

(21) Application number: **19732991.5**

(22) Date of filing: **20.06.2019**

(51) International Patent Classification (IPC):
***C07D 471/04*** (2006.01) ***A61K 31/437*** (2006.01)
***A61K 31/444*** (2006.01) ***A61K 31/501*** (2006.01)
***A61P 25/28*** (2006.01)

(52) Cooperative Patent Classification (CPC):
**C07D 471/04; A61K 31/437; A61K 31/501**

(86) International application number:
**PCT/EP2019/066392**

(87) International publication number:
**WO 2019/243533 (26.12.2019 Gazette 2019/52)**

# (54) OGA INHIBITOR COMPOUNDS

PYRROLOPYRIDIN VERBINDUNGEN ALS OGA-INHIBITOREN

COMPOSÉS PYRROLOPYRIDINES COMME DES INHIBITEURS DE OGA

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **21.06.2018 EP 18382455**

(43) Date of publication of application:
**28.04.2021 Bulletin 2021/17**

(73) Proprietor: **Janssen Pharmaceutica NV**
**2340 Beerse (BE)**

(72) Inventors:
- **BARTOLOMÉ-NEBREDA, José Manuel**
  **28042 Madrid (ES)**
- **TRABANCO-SUÁREZ, Andrés, Avelino**
  **28042 Madrid (ES)**
- **LEENAERTS, Joseph, Elisabeth**
  **2340 Beerse (BE)**
- **OEHLRICH, Daniel**
  **2340 Beerse (BE)**
- **BUIJNSTERS, Petrus Jacobus Johannes Antonius**
  **2340 Beerse (BE)**
- **MARTINEZ LAMENCA, Carolina**
  **2340 Beerse (BE)**
- **VELTER, Adriana, Ingrid**
  **2340 Beerse (BE)**
- **VAN ROOSBROECK, Yves, Emiel, Maria**
  **2340 Beerse (BE)**

(74) Representative: **Garcia Prieto, Maria**
**Johnson & Johnson**
**Patent Law Department**
**Turnhoutseweg 30**
**2340 Beerse (BE)**

(56) References cited:
**WO-A1-03/009852**
**WO-A1-2016/030443**
**WO-A1-2017/106254**
**WO-A1-2017/144633**
**WO-A1-2018/055316**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 810 608 B1

## Description

FIELD OF THE INVENTION

The present invention relates to O-GlcNAc hydrolase (OGA) inhibitors, having the structure shown in Formula (I)

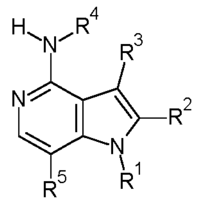

(I)

wherein the radicals are as defined in the specification. The invention is also directed to pharmaceutical compositions comprising such compounds, to processes for preparing such compounds and compositions, and to such compounds and compositions for use in the prevention and treatment of disorders in which inhibition of OGA is beneficial, such as tauopathies, in particular Alzheimer's disease or progressive supranuclear palsy; and neurodegenerative diseases accompanied by a tau pathology, in particular amyotrophic lateral sclerosis or frontotemporal lobe dementia caused by C9ORF72 mutations.

BACKGROUND OF THE INVENTION

O-GlcNAcylation is a reversible modification of proteins where N-acetyl-D-glucosamine residues are transferred to the hydroxyl groups of serine- and threonine residues yield O-GlcNAcylated proteins. More than 1000 of such target proteins have been identified both in the cytosol and nucleus of eukaryotes. The modification is thought to regulate a huge spectrum of cellular processes including transcription, cytoskeletal processes, cell cycle, proteasomal degradation, and receptor signaling.

O-GlcNAc transferase (OGT) and O-GlcNAc hydrolase (OGA) are the only two proteins described that add (OGT) or remove (OGA) O-GlcNAc from target proteins. OGA was initially purified in 1994 from spleen preparation and 1998 identified as antigen expressed by meningiomas and termed MGEA5, consists of 916 amino (102915 Dalton) as a monomer in the cytosolic compartment of cells. It is to be distinguished from ER- and Golgi-related glycosylation processes that are important for trafficking and secretion of proteins and different to OGA have an acidic pH optimum, whereas OGA display highest activity at neutral pH.

The OGA catalytic domain with its double aspartate catalytic center resides in then-terminal part of the enzyme which is flanked by two flexible domains. The C-terminal part consists of a putative HAT (histone acetyl transferase domain) preceded by a stalk domain. It has yet still to be proven that the HAT-domain is catalytically active.

O-GlcNAcylated proteins as well as OGT and OGA themselves are particularly abundant in the brain and neurons suggesting this modification plays an important role in the central nervous system. Indeed, studies confirmed that *O*-GlcNAcylation represents a key regulatory mechanism contributing to neuronal communication, memory formation and neurodegenerative disease. Moreover, it has been shown that OGT is essential for embryogenesis in several animal models and *ogt* null mice are embryonic lethal. OGA is also indispensible for mammalian development. Two independent studies have shown that OGA homozygous null mice do not survive beyond 24-48 hours after birth. *Oga* deletion has led to defects in glycogen mobilization in pups and it caused genomic instability linked cell cycle arrest in MEFs derived from homozygous knockout embryos. The heterozygous animals survived to adulthood however they exhibited alterations in both transcription and metabolism.

It is known that perturbations in O-GlcNAc cycling impact chronic metabolic diseases such as diabetes, as well as cancer. *Oga* heterozygosity suppressed intestinal tumorigenesis in an *Apc*-/+ mouse cancer model and the *Oga* gene (*MGEA5*) is a documented human diabetes susceptibility locus.

In addition, O-GlcNAc-modifications have been identified on several proteins that are involved in the development and progression of neurodegenerative diseases and a correlation between variations of O-GlcNAc levels on the formation of neurofibrillary tangle (NFT) protein by Tau in Alzheimer's disease has been suggested. In addition, O-GlcNAcylation of alpha-synuclein in Parkinson's disease has been described.

In the central nervous system six splice variants of tau have been described. Tau is encoded on chromosome 17 and consists in its longest splice variant expressed in the central nervous system of 441 amino acids. These isoforms differ by two N-terminal inserts (exon 2 and 3) and exon 10 which lie within the microtubule binding domain. Exon 10 is of considerable interest in tauopathies as it harbours multiple mutations that render tau prone to aggregation as described

below. Tau protein binds to and stabilizes the neuronal microtubule cytoskeleton which is important for regulation of the intracellular transport of organelles along the axonal compartments. Thus, tau plays an important role in the formation of axons and maintenance of their integrity. In addition, a role in the physiology of dendritic spines has been suggested as well.

Tau aggregation is either one of the underlying causes for a variety of so called tauopathies like PSP (progressive supranuclear palsy), Down's syndrome (DS), FTLD (frontotemporal lobe dementia), FTDP-17 (frontotemporal dementia with Parkinsonism-17), Pick's disease (PD), CBD (corticobasal degeneration), agryophilic grain disease (AGD), and AD (Alzheimer's disease). In addition, tau pathology accompanies additional neurodegenerative diseases like amyotrophic lateral sclerosis (ALS) or FTLD cause by C9ORF72 mutations. In these diseases, tau is post-translationally modified by excessive phosphorylation which is thought to detach tau from microtubules and makes it prone to aggregation. O-GlcNAcylation of tau regulates the extent of phosphorylation as serine or threonine residues carrying O-GlcNAc-residues are not amenable to phosphorylation. This effectively renders tau less prone to detaching from microtubules and reduces aggregation into neurotoxic tangles which ultimately lead to neurotoxicity and neuronal cell death. This mechanism may also reduce the cell-to-cell spreading of tau-aggregates released by neurons via along interconnected circuits in the brain which has recently been discussed to accelerate pathology in tau-related dementias. Indeed, hyperphosphorylated tau isolated from brains of AD-patients showed significantly reduced O-GlcNAcylation levels.

An OGA inhibitor administered to JNPL3 tau transgenic mice successfully reduced NFT formation and neuronal loss without apparent adverse effects. This observation has been confirmed in another rodent model of tauopathy where the expression of mutant tau found in FTD can be induced (tg4510). Dosing of a small molecule inhibitor of OGA was efficacious in reducing the formation of tau-aggregation and attenuated the cortical atrophy and ventricle enlargement.

Moreover, the O-GlcNAcylation of the amyloid precursor protein (APP) favours processing via the non-amyloidogenic route to produce soluble APP fragment and avoid cleavage that results in the AD associated amyloid-beta (A$\beta$) formation.

Maintaining O-GlcNAcylation of tau by inhibition of OGA represents a potential approach to decrease tau-phosphorylation and tau-aggregation in neurodegenerative diseases mentioned above thereby attenuating or stopping the progression of neurodegenerative tauopathy-diseases.

Some compounds are known in the art and have been disclosed to have OGA inhibitory activity: WO2012/117219 (Summit Corp. plc., published 7 September 2012) describes N-[[5-(hydroxymethyl)pyrrolidin-2-yl]methyl]alkylamide and N-alkyl-2-[5-(hydroxymethyl)pyrrolidin-2-yl]acetamide derivatives; WO2014/159234 (Merck Patent GMBH, published 2 October 2014) discloses mainly 4-phenyl or benzyl-piperidine and piperazine compounds substituted at the 1-position with an acetamido-thiazolylmethyl or acetamidoxazolylmethyl substituent; WO2016/0300443 (Asceneuron S.A., published 3 March 2016), WO2017/144633 and WO2017/0114639 (Asceneuron S.A., published 31 August 2017) disclose 1,4-disubstituted piperidines or piperazines; WO2017/144637 (Asceneuron S.A, published 31 August 2017) discloses more particular 4-substituted 1-[1-(1,3-benzodioxol-5-yl)ethyl]-piperazine; 1-[1-(2,3-dihydrobenzofuran-5-yl)ethyl]-; 1-[1-(2,3-dihydrobenzofuran-6-yl)ethyl]-; and 1-[1-(2,3-dihydro-1,4-benzodioxin-6-yl)ethyl]-piperazine derivatives; WO2017/106254 (Merck Sharp & Dohme Corp.) describes substituted N-[5-[(4-methylene-1-piperidyl)methyl]thiazol-2-yl]acetamide compounds; and WO2018/055316 (Centre Nat Rech Scient; Univ Orleans; Univ Francois Rabelai) describes (hetero)arylethynyl pyrrolopyridine compounds.

There is still a need for OGA inhibitor compounds with an advantageous balance of properties, for example with improved potency, good bioavailability, pharmacokinetics, and brain penetration, and/or better toxicity profile. It is accordingly an object of the present invention to provide compounds that overcome at least some of these problems.

SUMMARY OF THE INVENTION

The present invention is directed to compounds of Formula (I)

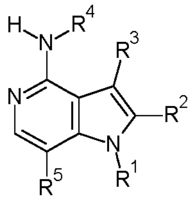

(I),

and the tautomers and the stereoisomeric forms thereof, wherein

$R^1$ is -$C_{1-6}$alkyl-C(O)-$NR^xR^y$, wherein

$R^x$ and $R^y$ are each independently selected from the group consisting of hydrogen and $C_{1-3}$alkyl; or $R^x$ and $R^y$ together with the nitrogen atom to which they are attached form a heterocyclyl ring selected from the group consisting of azetidinyl, pyrrolidinyl, piperidinyl, piperazinyl and morpholinyl;
$R^2$, $R^3$ and $R^5$ are each independently selected from the group consisting of hydrogen, halo and $C_{1-3}$alkyl;
$R^4$ is a monovalent radical selected from the group consisting of (a), (b), (c), (d), (e) and (f):

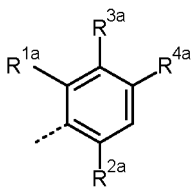 (a) 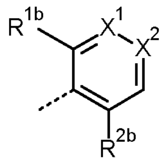 (b)

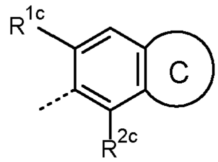 (c) 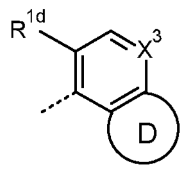 (d) 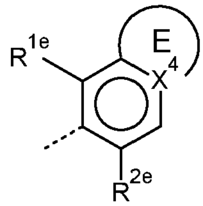 (e) 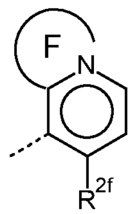 (f)

wherein
$R^{1a}$, $R^{2a}$, $R^{1b}$, and $R^{2b}$ are each independently selected from the group consisting of halo, $C_{1-3}$alkyl, monohalo$C_{1-3}$alkyl, polyhalo$C_{1-3}$alkyl, $C_{1-3}$alkyloxy, monohalo$C_{1-3}$alkyloxy, polyhalo$C_{1-3}$alkyloxy, and $C_{3-6}$cycloalkyl;
$R^{3a}$ is selected from the group consisting of hydrogen, halo, -C(O)-O$C_{1-3}$alkyl, -C(O)-NR'R", -N(R''')-C(O)-$C_{1-3}$alkyl;
$R^{4a}$ is selected from the group consisting of hydrogen, halo, -CN, $C_{1-3}$alkyl, monohalo$C_{1-3}$alkyl, polyhalo$C_{1-3}$alkyl, $C_{1-3}$alkyloxy, monohalo$C_{1-3}$alkyloxy, polyhalo$C_{1-3}$alkyloxy,
-C(O)-O$C_{1-3}$alkyl, -C(O)-NR'R", -N(R''')-C(O)-$C_{1-3}$alkyl, and Het; with the proviso that $R^{3a}$ and $R^{4a}$ are not simultaneously -C(O)-O$C_{1-3}$alkyl, -C(O)-NR'R", or -N(R''')-C(O)-$C_{1-3}$alkyl;
R' and R" are each independently selected from the group consisting of hydrogen and $C_{1-3}$alkyl; or R' and R" together with the nitrogen atom to which they are attached form a heterocyclyl ring selected from the group consisting of azetidinyl, pyrrolidinyl, piperidinyl, piperazinyl and morpholinyl;
R''' is selected from the group consisting of hydrogen and $C_{1-3}$alkyl;
Het is pyrazolyl or imidazolyl, optionally substituted with one or more independently selected $C_{1-3}$alkyl substituents;
$X^1$ and $X^2$ are each independently selected from N and CH, with the proviso that at least one of $X^1$ or $X^2$ is N;
$R^{1c}$, $R^{2c}$, $R^{1d}$, $R^{1e}$, $R^{2e}$, and $R^{2f}$ are each independently selected from the group consisting of halo, $C_{1-3}$alkyl, monohalo$C_{1-3}$alkyl, polyhalo$C_{1-3}$alkyl, $C_{1-3}$alkyloxy, monohalo$C_{1-3}$alkyl, polyhalo$C_{1-3}$alkyl, and $C_{3-6}$cycloalkyl;
$X^3$ represents CH or N;
$X^4$ represents C or N;
and each of the rings represented by

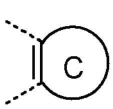, 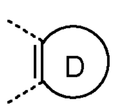, 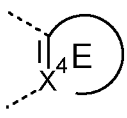, and 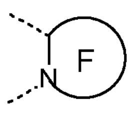,

form

(i) a 5- or 6-membered unsaturated heterocycle having one, two or three heteroatoms each independently selected from nitrogen and oxygen, and which is optionally substituted with one or more substituents, each independently selected from halo, $C_{1-3}$alkyl and oxo; or
(ii) a 5- or 6-membered aromatic heterocycle having one, two or three heteroatoms each independently selected from nitrogen, oxygen, and sulfur, and which is optionally substituted with one or more substituents, each

independently selected from halo, -CN, $C_{1-3}$alkyl, monohalo$C_{1-3}$alkyl, and polyhalo$C_{1-3}$alkyl;

and the pharmaceutically acceptable salts and the solvates thereof.

Illustrative of the invention is a pharmaceutical composition comprising a pharmaceutically acceptable carrier and any of the compounds described above. An illustration of the invention is a pharmaceutical composition made by mixing any of the compounds described above and a pharmaceutically acceptable carrier. Illustrating the invention is a process for making a pharmaceutical composition comprising mixing any of the compounds described above and a pharmaceutically acceptable carrier.

Another example of the invention is any of the compounds described above for use as a medicament, in particular in preventing or treating a tauopathy, more in particular a tauopathy selected from the group consisting of Alzheimer's disease, progressive supranuclear palsy, Down's syndrome, frontotemporal lobe dementia, frontotemporal dementia with Parkinsonism-17, Pick's disease, corticobasal degeneration, and agryophilic grain disease; or a neurodegenerative disease accompanied by a tau pathology, in particular a neurodegenerative disease selected from amyotrophic lateral sclerosis or frontotemporal lobe dementia caused by C9ORF72 mutations, in a subject in need thereof.

DETAILED DESCRIPTION OF THE INVENTION

The present invention is directed to compounds of Formula (I), as defined herein before, and pharmaceutically acceptable addition salts and solvates thereof. The compounds of Formula (I) are inhibitors of O-GlcNAc hydrolase (OGA) and may be useful in the prevention or treatment of tauopathies, in particular a tauopathy selected from the group consisting of Alzheimer's disease, progressive supranuclear palsy, Down's syndrome, frontotemporal lobe dementia, frontotemporal dementia with Parkinsonism-17, Pick's disease, corticobasal degeneration, and agryophilic grain disease; or maybe useful in the prevention or treatment of neurodegenerative diseases accompanied by a tau pathology, in particular a neurodegenerative disease selected from amyotrophic lateral sclerosis or frontotemporal lobe dementia caused by C9ORF72 mutations.

In a particular embodiment, the invention is directed to compounds of Formula (I) as defined hereinbefore, and the tautomers and the stereoisomeric forms thereof, wherein $R^1$ is -$C_{1-3}$alkyl-C(O)-$NR^xR^y$, wherein

$R^x$ and $R^y$ are each independently selected from the group consisting of hydrogen and $C_{1-3}$alkyl; or $R^x$ and $R^y$ together with the nitrogen atom to which they are attached form a heterocyclyl ring selected from the group consisting of azetidinyl, pyrrolidinyl, piperidinyl, piperazinyl and morpholinyl;
$R^2$, $R^3$ and $R^5$ are each independently selected from the group consisting of hydrogen, halo and $C_{1-3}$alkyl;
$R^4$ is a monovalent radical selected from the group consisting of (a), (b), (d) and (f), wherein
$R^{1a}$, $R^{2a}$, $R^{1b}$, and $R^{2b}$ are each independently selected from the group consisting of halo, $C_{1-3}$alkyl, monohalo$C_{1-3}$alkyl, polyhalo$C_{1-3}$alkyl, $C_{1-3}$alkyloxy, monohalo$C_{1-3}$alkyloxy, polyhalo$C_{1-3}$alkyloxy, and $C_{3-6}$cycloalkyl;
$R^{3a}$ is hydrogen;
$R^{4a}$ is selected from the group consisting of hydrogen, halo, -CN, $C_{1-3}$alkyl, monohalo$C_{1-3}$alkyl, polyhalo$C_{1-3}$alkyl, $C_{1-3}$alkyloxy, monohalo$C_{1-3}$alkyloxy, and polyhalo$C_{1-3}$alkyloxy;
$X^1$ and $X^2$ are each independently selected from N and CH, with the proviso that at least one of $X^1$ or $X^2$ is N;
$R^{1d}$ and $R^{2f}$ are each independently selected from $C_{1-3}$alkyl;
$X^3$ represents CH;
and each of the rings represented by

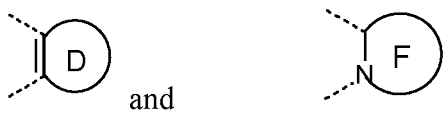

and

form

(i) a 5- or 6-membered unsaturated heterocycle having one or two heteroatoms each independently selected from nitrogen and oxygen, and which is optionally substituted with one or more substituents, each independently selected from halo and $C_{1-3}$alkyl; or
(ii) a 5- or 6-membered aromatic heterocycle having one, two or three heteroatoms each independently selected from nitrogen and oxygen, and which is optionally substituted with one or more substituents, each independently selected from $C_{1-3}$alkyl; and the pharmaceutically acceptable salts and the solvates thereof.

In a particular embodiment, the invention is directed to compounds of Formula (I) as referred to herein, and the tautomers and the stereoisomeric forms thereof, wherein

$R^1$ is -$C_{1-3}$alkyl-C(O)-$NR^xR^y$, wherein
$R^x$ and $R^y$ are each independently selected from the group consisting of hydrogen and $C_{1-3}$alkyl; or $R^x$ and $R^y$ together with the nitrogen atom to which they are attached form a heterocyclyl ring selected from the group consisting of azetidinyl, pyrrolidinyl, piperidinyl, piperazinyl and morpholinyl;
$R^2$, $R^3$ and $R^5$ are each independently selected from the group consisting of hydrogen, halo and $C_{1-3}$alkyl;
$R^4$ is a monovalent radical selected from the group consisting of (a), (b), (d) and (f), wherein
$R^{1a}$, $R^{2a}$, $R^{1b}$, and $R^{2b}$ are each independently selected from the group consisting of halo, $C_{1-3}$alkyl, polyhalo$C_{1-3}$alkyl, $C_{1-3}$alkyloxy, and polyhalo$C_{1-3}$alkyloxy;
$R^{3a}$ is hydrogen;
$R^{4a}$ is selected from the group consisting of hydrogen, halo, -CN, $C_{1-3}$alkyl, polyhalo$C_{1-3}$alkyl, $C_{1-3}$alkyloxy, and polyhalo$C_{1-3}$alkyloxy;
$X^1$ and $X^2$ are each independently selected from N and CH, with the proviso that at least one of $X^1$ or $X^2$ is N;
$R^{1d}$ and $R^{2f}$ are each independently selected from $C_{1-3}$alkyl;
$X^3$ represents CH;
and each of the rings represented by

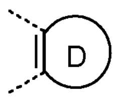 and 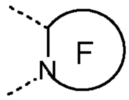

form

(i) a 5- or 6-membered unsaturated heterocycle having one or two nitrogen atoms, and which is optionally substituted with one or more substituents, each independently selected from halo and $C_{1-3}$alkyl; or
(ii) a 5- or 6-membered aromatic heterocycle having one or two nitrogen atoms, and which is optionally substituted with one or more substituents, each independently selected from $C_{1-3}$alkyl;

and the pharmaceutically acceptable salts and the solvates thereof.

In a particular embodiment, the invention is directed to compounds of Formula (I), as referred to herein, wherein $R^1$ is -$C_{1-3}$alkyl-C(O)-$NR^xR^y$, wherein -$NR^xR^y$ is selected from the group consisting of -$NH_2$, -$NHCH_3$, -$NH(CH_3)_2$, -$N(CH_3)(CH_2CH_3)$, azetidin-1-yl, and pyrrolidin-1-yl.
In an additional embodiment, the invention is directed to compounds of Formula (I), as referred to herein, wherein $R^2$, $R^3$ and $R^5$ are each independently selected from hydrogen and methyl.
In a further embodiment, the invention is directed to compounds of Formula (I), as referred to herein, wherein $R^{1a}$, $R^{2a}$, $R^{1b}$, and $R^{2b}$ are each independently selected from the group consisting of fluoro, chloro, methyl, isopropyl, $CF_3$, -$OCH_3$ and -$OCF_3$.
In another embodiment, the invention is directed to compounds of Formula (I), as referred to herein, wherein $R^{4a}$ is selected from the group consisting of hydrogen, fluoro, -CN, $CF_3$, and -$OCF_3$.
In another embodiment, the invention is directed to compounds of Formula (I), as referred to herein, wherein $X^1$ is N and $X^2$ is CH, or $X^1$ is CH and $X^2$ is N, or $X^1$ and $X^2$ are both N.
In a further embodiment, the invention is directed to compounds of Formula (I), as referred to herein, wherein $R^{1d}$, and $R^{2f}$ are each independently methyl or isopropyl.
In an additional embodiment, the invention is directed to compounds of Formula (I), as referred to herein, wherein $R^4$ is selected from the group consisting of

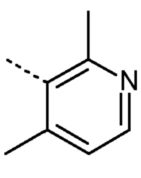 , 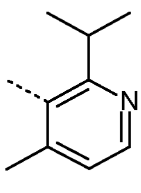 , 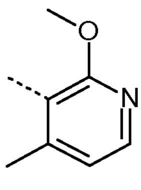 , 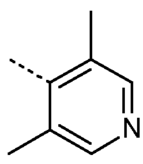 ,

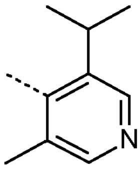 , 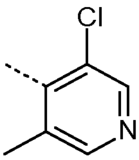 , 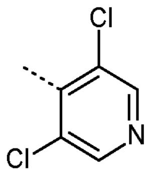 , 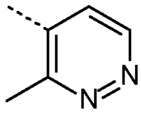 ,

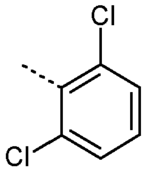 , 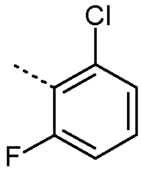 , 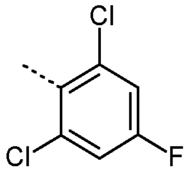 , 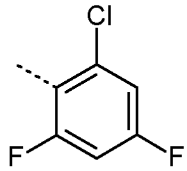 ,

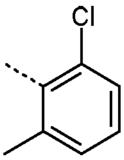 , 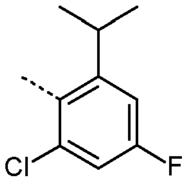 , 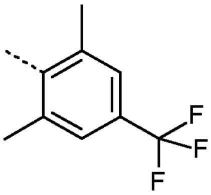 , 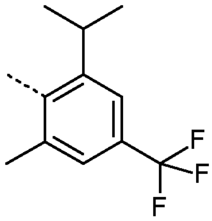 ,

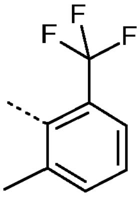 , 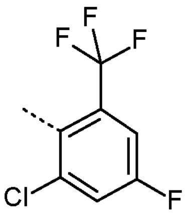 , 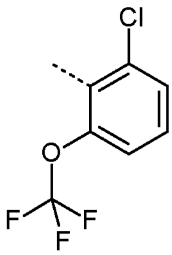 , 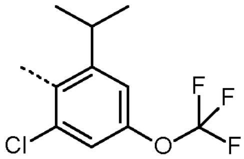 ,

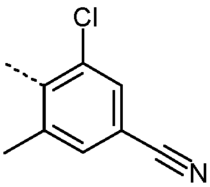 , 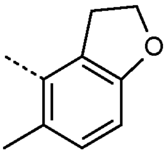 , 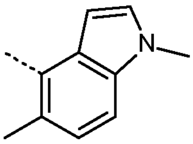 , and 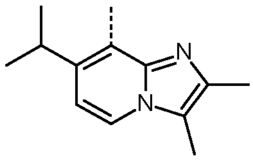 .

In an additional embodiment, the invention is directed to compounds of Formula (I), as referred to herein, wherein $R^4$ is selected from the group consisting of

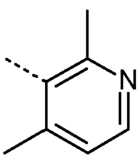 , 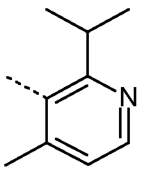 , 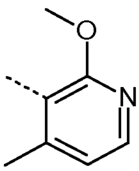 , 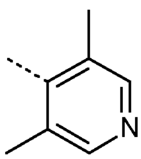 ,

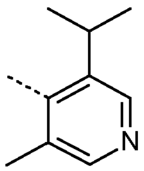, 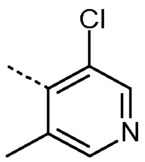, 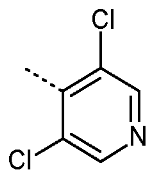, and 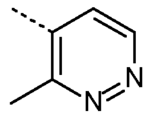.

In an additional embodiment, the invention is directed to compounds of Formula (I), as referred to herein, wherein $R^4$ is selected from the group consisting of

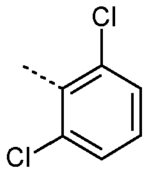, 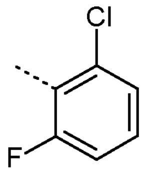, 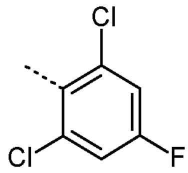, 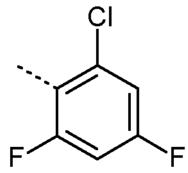,

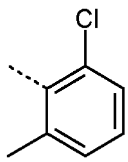, 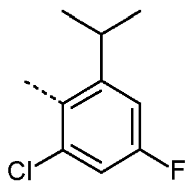, 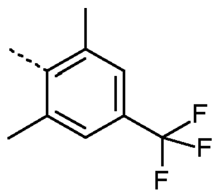, 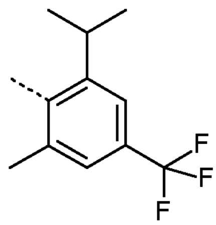,

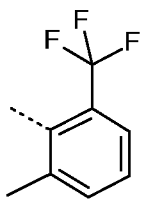, 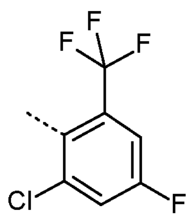, 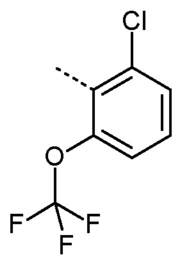, 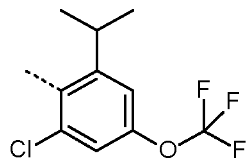, and

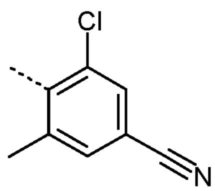.

In an additional embodiment, the invention is directed to compounds of Formula (I), as referred to herein, wherein $R^4$ is selected from the group consisting of

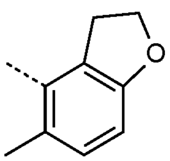, 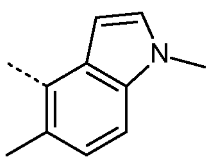, and 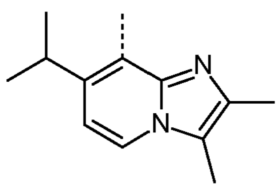.

DEFINITIONS

"Halo" shall denote fluoro, chloro and bromo; "oxo" shall denote =O, i.e. an oxygen atom doubly bound to a carbon atom; "$C_{1-3}$alkyl" shall denote a straight or branched saturated alkyl group having 1, 2, or 3 carbon atoms, respectively e.g. methyl, ethyl, 1-propyl, 2-propyl; "$C_{1-6}$alkyl" shall denote a straight or branched saturated alkyl group having 1, 2, 3 or 4 carbon atoms, respectively e.g. methyl, ethyl, 1-propyl, 2-propyl, butyl, 1-methyl-propyl, 2-methyl-1-propyl, 1,1-dimethylethyl, and the like; "$C_{1-3}$alkyloxy" shall denote an ether radical wherein $C_{1-3}$alkyl is as defined before; "mono- and polyhalo$C_{1-3}$alkyl" as used herein alone or as part of another group, refers to $C_{1-3}$alkyl as defined before, substituted with 1, 2, 3 or where possible with more halo atoms as defined before; "mono- and polyhalo$C_{1-3}$alkyloxy" as used herein alone or as part of another group, refers to $C_{1-3}$alkyloxy as defined before, substituted with 1, 2, 3 or where possible with more halo atoms as defined before; "$C_{3-6}$cycloalkyl" denotes cyclopropyl, cyclobutyl, cyclopentyl and cyclohexyl; "5- or 6-membered unsaturated heterocycle having one, two or three heteroatoms each independently selected from nitrogen and oxygen, include, but are not limited to azetidine, pyrrolidine, piperidine, piperidine, piperazine, morpholine, oxetane, tetrahydrofurane, tetrahydropyrane, dioxolane, and the like, when substituted with oxo the term includes e.g. lactams (e.g. pyrrolidinone, piperidinone); "5- or 6-membered aromatic heterocycle having one, two or three heteroatoms each independently selected from nitrogen, oxygen and sulfur" include, but are not limited to pyrrole, furane, pyrazole, imidazole, pyridine, pyrimidine, pyridazine, pyrazine, and the like.

In general, whenever the term "substituted" is used in the present invention, it is meant, unless otherwise indicated or is clear from the context, to indicate that one or more hydrogens, in particular 1 to 3 hydrogens, preferably 1 or 2 hydrogens, more preferably 1 hydrogen, on the atom or radical indicated in the expression using "substituted" are replaced with a selection of substituents from the indicated group, provided that the normal valency is not exceeded, and that the substitution results in a chemically stable compound, i.e. a compound that is sufficiently robust to survive isolation to a useful degree of purity from a reaction mixture, and formulation into a therapeutic agent.

The term "subject" as used herein, refers to an animal, preferably a mammal, most preferably a human, who is or has been the object of treatment, observation or experiment. As used herein, the term "subject" therefore encompasses patients, as well as asymptomatic or presymptomatic individuals at risk of developing a disease or condition as defined herein.

The term "therapeutically effective amount" as used herein, means that amount of active compound or pharmaceutical agent that elicits the biological or medicinal response in a tissue system, animal or human that is being sought by a researcher, veterinarian, medical doctor or other clinician, which includes alleviation of the symptoms of the disease or disorder being treated. The term "prophylactically effective amount" as used herein, means that amount of active compound or pharmaceutical agent that substantially reduces the potential for onset of the disease or disorder being prevented.

As used herein, the term "composition" is intended to encompass a product comprising the specified ingredients in the specified amounts, as well as any product which results, directly or indirectly, from combinations of the specified ingredients in the specified amounts.

Hereinbefore and hereinafter, the term "compound of Formula (I)" is meant to include the addition salts, the solvates and the stereoisomers thereof.

The terms "stereoisomers" or "stereochemically isomeric forms" hereinbefore or hereinafter are used interchangeably.

The invention includes all stereoisomers of the compound of Formula (I) either as a pure stereoisomer or as a mixture of two or more stereoisomers.

Enantiomers are stereoisomers that are non-superimposable mirror images of each other. A 1:1 mixture of a pair of enantiomers is a racemate or racemic mixture. Diastereomers (or diastereoisomers) are stereoisomers that are not enantiomers, i.e. they are not related as mirror images. If a compound contains a double bond, the substituents may be in the E or the Z configuration. If a compound contains a disubstituted cycloalkyl group, the substituents may be in the cis or trans configuration. Therefore, the invention includes enantiomers, diastereomers, racemates, E isomers, Z isomers, cis isomers, trans isomers and mixtures thereof.

The absolute configuration is specified according to the Cahn-Ingold-Prelog system. The configuration at an asymmetric atom is specified by either R or S. Resolved compounds whose absolute configuration is not known can be designated by (+) or (-) depending on the direction in which they rotate plane polarized light.

When a specific stereoisomer is identified, this means that said stereoisomer is substantially free, i.e. associated with less than 50%, preferably less than 20%, more preferably less than 10%, even more preferably less than 5%, in particular less than 2% and most preferably less than 1%, of the other isomers. Thus, when a compound of formula (I) is for instance specified as (R), this means that the compound is substantially free of the (S) isomer; when a compound of formula (I) is for instance specified as E, this means that the compound is substantially free of the Z isomer; when a compound of formula (I) is for instance specified as cis, this means that the compound is substantially free of the trans isomer.

For use in medicine, the addition salts of the compounds of this invention refer to nontoxic "pharmaceutically acceptable addition salts". Other salts may, however, be useful in the preparation of compounds according to this invention or of their pharmaceutically acceptable addition salts. Suitable pharmaceutically acceptable addition salts of the compounds include acid addition salts which may, for example, be formed by mixing a solution of the compound with a solution of a pharmaceutically acceptable acid such as hydrochloric acid, sulfuric acid, fumaric acid, maleic acid, succinic acid, acetic acid, benzoic acid, citric acid, tartaric acid, carbonic acid or phosphoric acid. Furthermore, where the compounds of the invention carry an acidic moiety, suitable pharmaceutically acceptable addition salts thereof may include alkali metal salts, e.g., sodium or potassium salts; alkaline earth metal salts, e.g., calcium or magnesium salts; and salts formed with suitable organic ligands, e.g., quaternary ammonium salts.

Representative acids which may be used in the preparation of pharmaceutically acceptable addition salts include, but are not limited to, the following: acetic acid, 2,2-dichloroactic acid, acylated amino acids, adipic acid, alginic acid, ascorbic acid, L-aspartic acid, benzenesulfonic acid, benzoic acid, 4- acetamidobenzoic acid, (+)-camphoric acid, camphorsulfonic acid, capric acid, caproic acid, caprylic acid, cinnamic acid, citric acid, cyclamic acid, ethane-1,2-disulfonic acid, ethanesulfonic acid, 2-hydroxy-ethanesulfonic acid, formic acid, fumaric acid, galactaric acid, gentisic acid, glucoheptonic acid, D-gluconic acid, D-glucoronic acid, L-glutamic acid, beta-oxo-glutaric acid, glycolic acid, hippuric acid, hydrobromic acid, hydrochloric acid, (+)-L-lactic acid, (±)-DL-lactic acid, lactobionic acid, maleic acid, (-)-L-malic acid, malonic acid, (±)-DL-mandelic acid, methanesulfonic acid, naphthalene-2-sulfonic acid, naphthalene-1,5- disulfonic acid, 1-hydroxy-2-naphthoic acid, nicotinic acid, nitric acid, oleic acid, orotic acid, oxalic acid, palmitic acid, pamoic acid, phosphoric acid, L- pyroglutamic acid, salicylic acid, 4-amino-salicylic acid, sebacic acid, stearic acid, succinic acid, sulfuric acid, tannic acid, (+)-L-tartaric acid, thiocyanic acid, p-toluenesulfonic acid, trifluoromethylsulfonic acid, and undecylenic acid. Representative bases which may be used in the preparation of pharmaceutically acceptable addition salts include, but are not limited to, the following: ammonia, L-arginine, benethamine, benzathine, calcium hydroxide, choline, dimethylethanolamine, diethanolamine, diethylamine, 2-(diethylamino)-ethanol, ethanolamine, ethylene-diamine, *N*-methyl-glucamine, hydrabamine, 1*H*-imidazole, L-lysine, magnesium hydroxide, 4-(2-hydroxyethyl)-morpholine, piperazine, potassium hydroxide, 1-(2-hydroxyethyl)-pyrrolidine, secondary amine, sodium hydroxide, triethanolamine, tromethamine and zinc hydroxide.

The names of compounds were generated according to the nomenclature rules agreed upon by the Chemical Abstracts Service (CAS) or according to the nomenclature rules agreed upon by the International Union of Pure and Applied Chemistry (IUPAC).

PREPARATION OF THE FINAL COMPOUNDS

The compounds according to the invention can generally be prepared by a succession of steps, each of which is known to the skilled person. In particular, the compounds can be prepared according to the following synthesis methods.

The compounds of Formula (I) may be synthesized in the form of racemic mixtures of enantiomers which can be separated from one another following art-known resolution procedures. The racemic compounds of Formula (I) may be converted into the corresponding diastereomeric salt forms by reaction with a suitable chiral acid. Said diastereomeric salt forms are subsequently separated, for example, by selective or fractional crystallization and the enantiomers are liberated therefrom by alkali. An alternative manner of separating the enantiomeric forms of the compounds of Formula (I) involves liquid chromatography using a chiral stationary phase. Said pure stereochemically isomeric forms may also be derived from the corresponding pure stereochemically isomeric forms of the appropriate starting materials, provided that the reaction occurs stereospecifically.

EXPERIMENTAL PROCEDURE 1

Final compounds of Formula (I) wherein $R^1$ is $-C_{1-6}alkyl-C(O)-NR^xR^y$ can be prepared by reacting an intermediate compound of Formula (II-a) with a compound of Formula (III) according to reaction scheme 1. The reaction is performed in a suitable reaction-inert solvent, such as for example $^tBuOH$, in the presence of a base, such as $Cs_2CO_3$, in the presence of a catalyst, such as $Pd(OAc)_2$ and a suitable phosphorus ligand, such as XantPhos, under thermal conditions, such as for example at 130 °C for a suitable period of time to drive the reaction to completion. In reaction scheme 1 all variables are defined as in Formula (I) and halo represents a halogen, in particular, bromo or chloro.

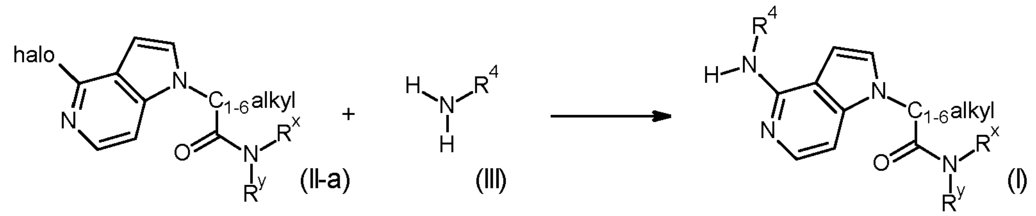
(II-a) (III) (I)

EXPERIMENTAL PROCEDURE 2

Alternatively, final compounds of Formula (I) can be prepared by reacting an intermediate compound of Formula (II-b) with a compound of Formula (IV) according to reaction scheme 2. The reaction is performed in a suitable reaction-inert solvent, such as for example DMF, in the presence of a base, such as NaH, at a suitable temperature, such as for example at 0 °C to room temperature for a suitable period of time to drive the reaction to completion. In reaction scheme 2 all variables are defined as in Formula (I) and halo represents a halogen, in particular, bromo or chloro.

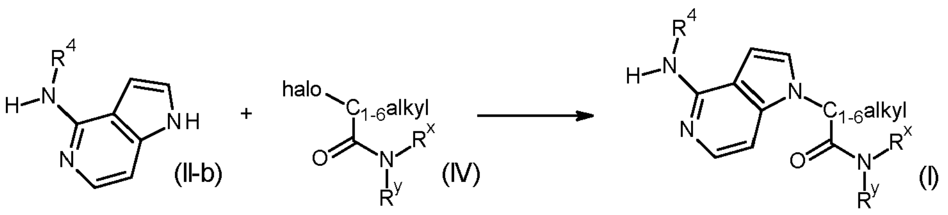
(II-b) (IV) (I)

EXPERIMENTAL PROCEDURE 3

Alternatively, final compounds of Formula (I) can be prepared by reacting an intermediate compound of Formula (II-c) with a suitable amine of Formula (V) according to reaction scheme 3. Several conditions can be utilized in order to form the amide: (a) a coupling agent such as HOBt and a carbodiimide, for example EDCI, in the presence of a suitable base such as DIPEA, in a reaction-inert solvent such as DCM and/or DMF under thermal conditions, for example by performing the reaction at room temperature; (b) a coupling reagent such as T3P® in the presence of a suitable base, such as triethylamine, in a reaction-inert solvent such as THF under suitable conditions, such as for example thermal conditions, such as from room temperature to 50 °C; (c) a coupling reagent such as HBTU in the presence of a base, such as DIPEA, in a reaction-inert solvent, such as DMF, at a suitable temperature, for example at room temperature. In reaction scheme 3 all variables are defined as in Formula (I).

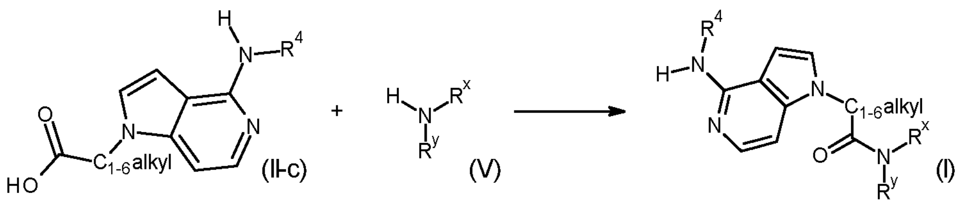
(II-c) (V) (I)

EXPERIMENTAL PROCEDURE 4

Intermediate compounds of Formula (II-a) can be prepared according to reaction scheme 4, step (i). An intermediate compound of Formula (VI) can be reacted with a compound of Formula (IV) in the presence of a suitable base, such as for example, NaH in a reaction-inert solvent, such as for example DMF, at a suitable temperature, such as 0 °C to room temperature.

Intermediate compounds of Formula (II-c) can be prepared according to reaction scheme 4, steps (ii)-(iii). Thus, a compound of Formula (VI) can be reacted with a compound of Formula (VII) in the presence of a suitable base, such as for example, NaH in a reaction-inert solvent, such as for example DMF, at a suitable temperature, such as 0 °C to room temperature. The resulting compound of Formula (VIII) can be subsequently hydrolysed under suitable acidic (e.g. TFA in DCM) or basic conditions (e.g. LiOH in THF and/or water).

In reaction scheme 4 all variables are defined as in Formula (I) and halo represents a halogen, in particular, bromo or chloro

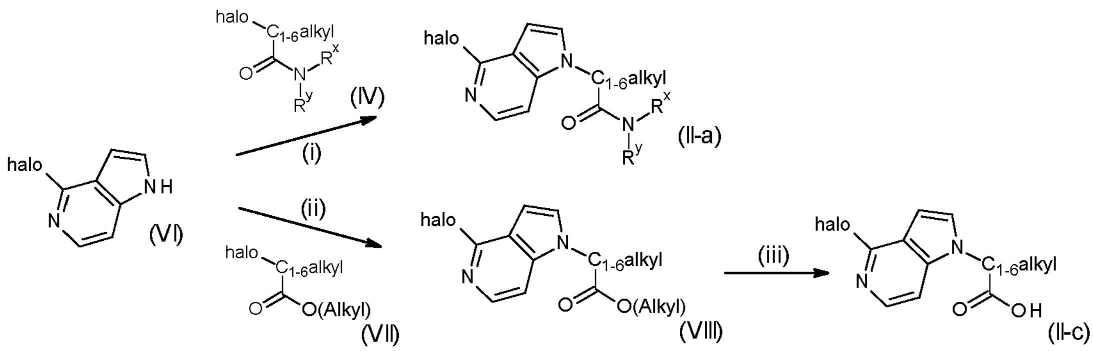


EXPERIMENTAL PROCEDURE 5

Intermediate compounds of Formula (II-b) can be prepared according to reaction scheme 5. Thus, a compound of Formula (IX), wherein PG represents a protecting group (e.g. phenylsulfonyl), can be oxidized under suitable conditions, for example by mCPBA in DCM to yield an intermediate of Formula (X). Reaction with a suitable halogenating agent (e.g. $POBr_3$ in ACN and dioxane at a suitable temperature, for example 70 °C) provides an intermediate of Formula (XI), which can then be reacted with an intermediate of Formula (III), under the conditions described in experimental procedure 1 to yield an intermediate of Formula (XII). The protecting group in (XII) can be cleaved under suitable conditions (e.g. when PG is phenylsulfonyl, by treatment with NaOH in MeOH).

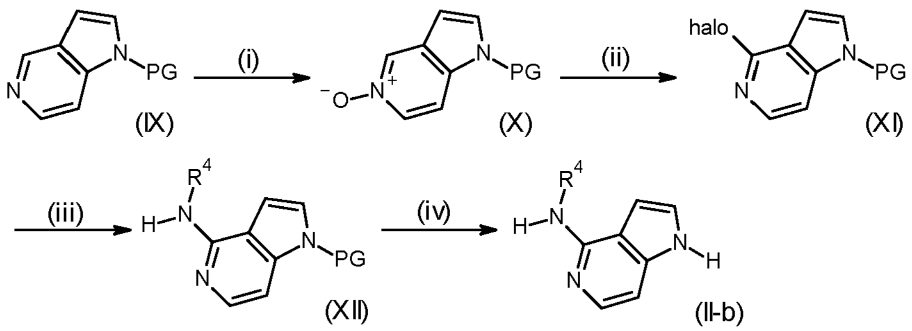


In reaction scheme 5 all variables are defined as in Formula (I) and halo represents a halogen, in particular, bromo or chloro.

Intermediate compounds of Formulae (VI) and (IX) are either commercially available or can be synthesized by reaction procedures known to the skilled person.

PHARMACOLOGY

The compounds of the present invention and the pharmaceutically acceptable compositions thereof inhibit O-GlcNAc hydrolase (OGA) and therefore may be useful in the treatment or prevention of diseases involving tau pathology, also known as tauopathies, and diseases with tau inclusions. Such diseases include, but are not limited to Alzheimer's disease, amyotrophic lateral sclerosis and parkinsonism-dementia complex, argyrophilic grain disease, chronic traumatic encephalopathy, corticobasal degeneration, diffuse neurofibrillary tangles with calcification, Down's syndrome, Familial British dementia, Familial Danish dementia, Frontotemporal dementia and parkinsonism linked to chromosome 17 (caused by MAPT mutations), Frontotemporal lobar degeneration (some cases caused by C9ORF72 mutations), Gerstmann-Sträussler-Scheinker disease, Guadeloupean parkinsonism, myotonic dystrophy, neurodegeneration with brain iron accumulation, Niemann-Pick disease, type C, non-Guamanian motor neuron disease with neurofibrillary tangles, Pick's disease, postencephalitic parkinsonism, prion protein cerebral amyloid angiopathy, progressive subcortical gliosis, progressive supranuclear palsy, SLC9A6-related mental retardation, subacute sclerosing panencephalitis, tangle-only dementia, and white matter tauopathy with globular glial inclusions.

As used herein, the term "treatment" is intended to refer to all processes, wherein there may be a slowing, interrupting, arresting or stopping of the progression of a disease or an alleviation of symptoms, but does not necessarily indicate a total elimination of all symptoms. As used herein, the term "prevention" is intended to refer to all processes, wherein there may be a slowing, interrupting, arresting or stopping of the onset of a disease.

The invention also relates to a compound according to the general Formula (I), a stereoisomeric form thereof or a pharmaceutically acceptable acid or base addition salt thereof, for use in the treatment or prevention of diseases or conditions selected from the group consisting of Alzheimer's disease, amyotrophic lateral sclerosis and parkinsonism-dementia complex, argyrophilic grain disease, chronic traumatic encephalopathy, corticobasal degeneration, diffuse neurofibrillary tangles with calcification, Down's syndrome, Familial British dementia, Familial Danish dementia, Frontotemporal dementia and parkinsonism linked to chromosome 17 (caused by MAPT mutations), Frontotemporal lobar degeneration (some cases caused by C9ORF72 mutations), Gerstmann-Sträussler-Scheinker disease, Guadeloupean parkinsonism, myotonic dystrophy, neurodegeneration with brain iron accumulation, Niemann-Pick disease, type C, non-Guamanian motor neuron disease with neurofibrillary tangles, Pick's disease, postencephalitic parkinsonism, prion protein cerebral amyloid angiopathy, progressive subcortical gliosis, progressive supranuclear palsy, SLC9A6-related mental retardation, subacute sclerosing panencephalitis, tangle-only dementia, and white matter tauopathy with globular glial inclusions.

The invention also relates to a compound according to the general Formula (I), a stereoisomeric form thereof or a pharmaceutically acceptable acid or base addition salt thereof, for use in the treatment, prevention, amelioration, control or reduction of the risk of diseases or conditions selected from the group consisting of Alzheimer's disease, amyotrophic lateral sclerosis and parkinsonism-dementia complex, argyrophilic grain disease, chronic traumatic encephalopathy, corticobasal degeneration, diffuse neurofibrillary tangles with calcification, Down's syndrome, Familial British dementia, Familial Danish dementia, Frontotemporal dementia and parkinsonism linked to chromosome 17 (caused by MAPT mutations), Frontotemporal lobar degeneration (some cases caused by C9ORF72 mutations), Gerstmann-Sträussler-Scheinker disease, Guadeloupean parkinsonism, myotonic dystrophy, neurodegeneration with brain iron accumulation, Niemann-Pick disease, type C, non-Guamanian motor neuron disease with neurofibrillary tangles, Pick's disease, postencephalitic parkinsonism, prion protein cerebral amyloid angiopathy, progressive subcortical gliosis, progressive supranuclear palsy, SLC9A6-related mental retardation, subacute sclerosing panencephalitis, tangle-only dementia, and white matter tauopathy with globular glial inclusions.

In particular, the diseases or conditions may in particular be selected from a tauopathy, more in particular a tauopathy selected from the group consisting of Alzheimer's disease, progressive supranuclear palsy, Down's syndrome, frontotemporal lobe dementia, frontotemporal dementia with Parkinsonism-17, Pick's disease, corticobasal degeneration, and agryophilic grain disease; or the diseases or conditions may in particular be neurodegenerative diseases accompanied by a tau pathology, more in particular a neurodegenerative disease selected from amyotrophic lateral sclerosis or frontotemporal lobe dementia caused by C9ORF72 mutations.

Preclinical states in Alzheimer's and tauopathy diseases:

In recent years the United States (US) National Institute for Aging and the International Working Group have proposed guidelines to better define the preclinical (asymptomatic) stages of AD (Dubois B, et al. Lancet Neurol. 2014;13:614-629;

Sperling, RA, et al. Alzheimers Dement. 2011;7:280-292). Hypothetical models postulate that Aβ accumulation and tau-aggregation begins many years before the onset of overt clinical impairment. The key risk factors for elevated amyloid accumulation, tau-aggregation and development of AD are age (ie, 65 years or older), *APOE* genotype, and family history. Approximately one third of clinically normal older individuals over 75 years of age demonstrate evidence of Aβ or tau accumulation on PET amyloid and tau imaging studies, the latter being less advanced currently. In addition, reduced Abeta-levels in CSF measurements are observed, whereas levels of non-modified as well as phosphorylated tau are elevated in CSF. Similar findings are seen in large autopsy studies and it has been shown that tau aggregates are detected in the brain as early as 20 years of age and younger. Amyloid-positive (Aβ+) clinically normal individuals consistently demonstrate evidence of an "AD-like endophenotype" on other biomarkers, including disrupted functional network activity in both functional magnetic resonance imaging (MRI) and resting state connectivity, fluorodeoxyglucose $^{18}$F (FDG) hypometabolism, cortical thinning, and accelerated rates of atrophy. Accumulating longitudinal data also strongly suggests that Aβ+ clinically normal individuals are at increased risk for cognitive decline and progression to mild cognitive impairment (MCI) and AD dementia. The Alzheimer's scientific community is of the consensus that these Aβ+ clinically normal individuals represent an early stage in the continuum of AD pathology. Thus, it has been argued that intervention with a therapeutic agent that decreases Aβ production or the aggregation of tau is likely to be more effective if started at a disease stage *before* widespread neurodegeneration has occurred. A number of pharmaceutical companies are currently testing BACE inhibition in prodromal AD

Thanks to evolving biomarker research, it is now possible to identify Alzheimer's disease at a preclinical stage before the occurrence of the first symptoms. All the different issues relating to preclinical Alzheimer's disease such as, definitions and lexicon, the limits, the natural history, the markers of progression and the ethical consequences of detecting the disease at the asymptomatic stage, are reviewed in Alzheimer's & Dementia 12 (2016) 292-323.

Two categories of individuals may be recognized in preclinical Alzheimer's disease or tauopathies. Cognitively

normal individuals with amyloid beta or tau aggregation evident on PET scans, or changes in CSF Abeta, tau and phospho-tau are defined as being in an "asymptomatic at risk state for Alzheimer's disease (AR-AD)" or in a "asymptomatic state of tauopathy". Individuals with a fully penetrant dominant autosomal mutation for familial Alzheimer's disease are said to have "presymptomatic Alzheimer's disease". Dominant autosomal mutations within the tau-protein have been described for multiple forms of tauopathies as well.

Thus, in an embodiment, the invention also relates to a compound according to the general Formula (I), a stereoisomeric form thereof or a pharmaceutically acceptable acid or base addition salt thereof, for use in control or reduction of the risk of preclinical Alzheimer's disease, prodromal Alzheimer's disease, or tau-related neurodegeneration as observed in different forms of tauopathies.

As already mentioned hereinabove, the term "treatment" does not necessarily indicate a total elimination of all symptoms, but may also refer to symptomatic treatment in any of the disorders mentioned above. In view of the utility of the compound of Formula (I), there is provided a compound for use in a method of treating subjects such as warm-blooded animals, including humans, suffering from or a method of preventing subjects such as warm-blooded animals, including humans, suffering from any one of the diseases mentioned hereinbefore.

Said methods comprise the administration, i.e. the systemic or topical administration, preferably oral administration, of a prophylactically or a therapeutically effective amount of a compound of Formula (I), a stereoisomeric form thereof, a pharmaceutically acceptable addition salt or solvate thereof, to a subject such as a warm-blooded animal, including a human.

Therefore, the invention also relates to a compound for use in a method for the prevention and/or treatment of any of the diseases mentioned hereinbefore comprising administering a prophylactically or a therapeutically effective amount of a compound according to the invention to a subject in need thereof.

A method of treatment may also include administering the active ingredient on a regimen of between one and four intakes per day. In these methods of treatment the compounds according to the invention are preferably formulated prior to administration. As described herein below, suitable pharmaceutical formulations are prepared by known procedures using well known and readily available ingredients. The compounds of the present invention, that can be suitable to treat or prevent any of the disorders mentioned above or the symptoms thereof, may be administered alone or in combination with one or more additional therapeutic agents. Combination therapy includes administration of a single pharmaceutical dosage formulation which contains a compound of Formula (I) and one or more additional therapeutic agents, as well as administration of the compound of Formula (I) and each additional therapeutic agent in its own separate pharmaceutical dosage formulation. For example, a compound of Formula (I) and a therapeutic agent may be administered to the patient together in a single oral dosage composition such as a tablet or capsule, or each agent may be administered in separate oral dosage formulations.

A skilled person will be familiar with alternative nomenclatures, nosologies, and classification systems for the diseases or conditions referred to herein. For example, the fifth edition of the Diagnostic & Statistical Manual of Mental Disorders (DSM-5™) of the American Psychiatric Association utilizes terms such as neurocognitive disorders (NCDs) (both major and mild), in particular, neurocognitive disorders due to Alzheimer's disease. Such terms may be used as an alternative nomenclature for some of the diseases or conditions referred to herein by the skilled person.

PHARMACEUTICAL COMPOSITIONS

The present invention also provides compositions for preventing or treating diseases in which inhibition of O-GlcNAc hydrolase (OGA) is beneficial, such as Alzheimer's disease, progressive supranuclear palsy, Down's syndrome, frontotemporal lobe dementia, frontotemporal dementia with Parkinsonism-17, Pick's disease, corticobasal degeneration, agryophilic grain disease, amyotrophic lateral sclerosis or frontotemporal lobe dementia caused by C9ORF72 mutations, said compositions comprising a therapeutically effective amount of a compound according to formula (I) and a pharmaceutically acceptable carrier or diluent.

While it is possible for the active ingredient to be administered alone, it is preferable to present it as a pharmaceutical composition. Accordingly, the present invention further provides a pharmaceutical composition comprising a compound according to the present invention, together with a pharmaceutically acceptable carrier or diluent. The carrier or diluent must be "acceptable" in the sense of being compatible with the other ingredients of the composition and not deleterious to the recipients thereof.

The pharmaceutical compositions of this invention may be prepared by any methods well known in the art of pharmacy. A therapeutically effective amount of the particular compound, in base form or addition salt form, as the active ingredient is combined in intimate admixture with a pharmaceutically acceptable carrier, which may take a wide variety of forms depending on the form of preparation desired for administration. These pharmaceutical compositions are desirably in unitary dosage form suitable, preferably, for systemic administration such as oral, percutaneous or parenteral administration; or topical administration such as via inhalation, a nose spray, eye drops or via a cream, gel, shampoo or the like. For example, in preparing the compositions in oral dosage form, any of the usual pharmaceutical media may

be employed, such as, for example, water, glycols, oils, alcohols and the like in the case of oral liquid preparations such as suspensions, syrups, elixirs and solutions; or solid carriers such as starches, sugars, kaolin, lubricants, binders, disintegrating agents and the like in the case of powders, pills, capsules and tablets. Because of their ease in administration, tablets and capsules represent the most advantageous oral dosage unit form, in which case solid pharmaceutical carriers are obviously employed. For parenteral compositions, the carrier will usually comprise sterile water, at least in large part, though other ingredients, for example, to aid solubility, may be included. Injectable solutions, for example, may be prepared in which the carrier comprises saline solution, glucose solution or a mixture of saline and glucose solution. Injectable suspensions may also be prepared in which case appropriate liquid carriers, suspending agents and the like may be employed. In the compositions suitable for percutaneous administration, the carrier optionally comprises a penetration enhancing agent and/or a suitable wettable agent, optionally combined with suitable additives of any nature in minor proportions, which additives do not cause any significant deleterious effects on the skin. Said additives may facilitate the administration to the skin and/or may be helpful for preparing the desired compositions. These compositions may be administered in various ways, e.g., as a transdermal patch, as a spot-on or as an ointment.

It is especially advantageous to formulate the aforementioned pharmaceutical compositions in dosage unit form for ease of administration and uniformity of dosage. Dosage unit form as used in the specification and claims herein refers to physically discrete units suitable as unitary dosages, each unit containing a predetermined quantity of active ingredient calculated to produce the desired therapeutic effect in association with the required pharmaceutical carrier. Examples of such dosage unit forms are tablets (including scored or coated tablets), capsules, pills, powder packets, wafers, injectable solutions or suspensions, teaspoonfuls, tablespoonfuls and the like, and segregated multiples thereof.

The exact dosage and frequency of administration depends on the particular compound of Formula (I) used, the particular condition being treated, the severity of the condition being treated, the age, weight, sex, extent of disorder and general physical condition of the particular patient as well as other medication the individual may be taking, as is well known to those skilled in the art. Furthermore, it is evident that said effective daily amount may be lowered or increased depending on the response of the treated subject and/or depending on the evaluation of the physician prescribing the compounds of the instant invention.

Depending on the mode of administration, the pharmaceutical composition will comprise from 0.05 to 99% by weight, preferably from 0.1 to 70% by weight, more preferably from 0.1 to 50% by weight of the active ingredient, and, from 1 to 99.95% by weight, preferably from 30 to 99.9% by weight, more preferably from 50 to 99.9% by weight of a pharmaceutically acceptable carrier, all percentages being based on the total weight of the composition.

The present compounds can be used for systemic administration such as oral, percutaneous or parenteral administration; or topical administration such as via inhalation, a nose spray, eye drops or via a cream, gel, shampoo or the like. The compounds are preferably orally administered. The exact dosage and frequency of administration depends on the particular compound according to Formula (I) used, the particular condition being treated, the severity of the condition being treated, the age, weight, sex, extent of disorder and general physical condition of the particular patient as well as other medication the individual may be taking, as is well known to those skilled in the art. Furthermore, it is evident that said effective daily amount may be lowered or increased depending on the response of the treated subject and/or depending on the evaluation of the physician prescribing the compounds of the instant invention. The amount of a compound of Formula (I) that can be combined with a carrier material to produce a single dosage form will vary depending upon the disease treated, the mammalian species, and the particular mode of administration. However, as a general guide, suitable unit doses for the compounds of the present invention can, for example, preferably contain between 0.1 mg to about 1000 mg of the active compound. A preferred unit dose is between 1 mg to about 500 mg. A more preferred unit dose is between 1 mg to about 300 mg. Even more preferred unit dose is between 1 mg to about 100 mg. Such unit doses can be administered more than once a day, for example, 2, 3, 4, 5 or 6 times a day, but preferably 1 or 2 times per day, so that the total dosage for a 70 kg adult is in the range of 0.001 to about 15 mg per kg weight of subject per administration. A preferred dosage is 0.01 to about 1.5 mg per kg weight of subject per administration, and such therapy can extend for a number of weeks or months, and in some cases, years. It will be understood, however, that the specific dose level for any particular patient will depend on a variety of factors including the activity of the specific compound employed; the age, body weight, general health, sex and diet of the individual being treated; the time and route of administration; the rate of excretion; other drugs that have previously been administered; and the severity of the particular disease undergoing therapy, as is well understood by those of skill in the area.

A typical dosage can be one 1 mg to about 100 mg tablet or 1 mg to about 300 mg taken once a day, or, multiple times per day, or one time-release capsule or tablet taken once a day and containing a proportionally higher content of active ingredient. The time-release effect can be obtained by capsule materials that dissolve at different pH values, by capsules that release slowly by osmotic pressure, or by any other known means of controlled release.

It can be necessary to use dosages outside these ranges in some cases as will be apparent to those skilled in the art. Further, it is noted that the clinician or treating physician will know how and when to start, interrupt, adjust, or terminate therapy in conjunction with individual patient response.

The invention also provides a kit comprising a compound according to the invention, prescribing information

also known as "leaflet", a blister package or bottle, and a container. Furthermore, the invention provides a kit comprising a pharmaceutical composition according to the invention, prescribing information also known as "leaflet", a blister package or bottle, and a container. The prescribing information preferably includes advice or instructions to a patient regarding the administration of the compound or the pharmaceutical composition according to the invention. In particular, the prescribing information includes advice or instruction to a patient regarding the administration of said compound or pharmaceutical composition according to the invention, on how the compound or the pharmaceutical composition according to the invention is to be used, for the prevention and/or treatment of a tauopathy in a subject in need thereof. Thus, in an embodiment, the invention provides a kit of parts comprising a compound of Formula (I) or a stereoisomeric for thereof, or a pharmaceutically acceptable salt or a solvate thereof, or a pharmaceutical composition comprising said compound, and instructions for preventing or treating a tauopathy. The kit referred to herein can be, in particular, a pharmaceutical package suitable for commercial sale.

For the compositions, methods and kits provided above, one of skill in the art will understand that preferred compounds for use in each are those compounds that are noted as preferred above. Still further preferred compounds for the compositions, methods and kits are those compounds provided in the non-limiting Examples below.

EXPERIMENTAL PART

Hereinafter, the term "m.p." means melting point, "min" means minutes, "AcOH" means acetic acid, "aq." means aqueous, "DIBAL" means diisobutylaluminium hydride, "r.m." means reaction mixture, "r.t." or "RT" means room temperature, "rac" or "RS" means racemic, "sat." means saturated, "SFC" means supercritical fluid chromatography, "SFC-MS" means supercritical fluid chromatography/mass spectrometry, "LC-MS" means liquid chromatography/mass spectrometry, "HOBt" means 1-hydroxybenzotriazole, "HPLC" means high-performance liquid chromatography, "NP" means normal phase, "RP" means reversed phase, "Rt" means retention time (in minutes), "[M+H]+" means the protonated mass of the free base of the compound, "wt" means weight, "THF" means tetrahydrofuran, "EtOAc" means ethyl acetate, "DCE" means dichloroethane, "DCM" means dichloromethane, "DME" means 1,2-dimethoxyethane, "DMF" means dimethylformamide, "DIPEA" means N,N-diisopropylethylamine, "EDC" means ethylcarbodiimide, "$Et_2O$" means diethyl ether, "MeOH" means methanol, "MeCN" means acetonitrile, "MW" means microwave, "org." means organic, "sol." means solution, "Boc" means tert-butoxycarbonyl, "TLC" means thin layer chromatography, "Pd/C" means palladium on carbon, "EtOH" means ethanol, "DIPE" means diisopropyl ether, "T3P" means propylphosphonic anhydride solution, "$Pd(OAc)_2$" means palladium(II) acetate, "$Pd_2dba_3$" tris(dibenzylideneacetone)dipalladium(0), "$PdCl_2(dppf)$" means 1,1'-[bis(diphenylphosphino)ferrocene]dichloropalladium(II), "$Pd(PPh_3)_4$" means tetrakis(triphenylphosphine)palladium(0), "XantPhos" means 4,5-bis(diphenylphosphino)-9,9-dimethylxanthene, "HBTU" means *N*,*N*,*N'*,*N'*-tetramethyl-*O*-(1*H*-benzotriazol-1-yl)uranium hexafluorophosphate, "*i*-PrOH" means isopropyl alcohol.

Microwave assisted reactions were performed in a single-mode reactor: Initiator™ Sixty EXP microwave reactor (Biotage AB), or in a multimode reactor: MicroSYNTH Labstation (Milestone, Inc.).

Thin layer chromatography (TLC) was carried out on silica gel 60 F254 plates (Merck) using reagent grade solvents. Open column chromatography was performed on silica gel, particle size 60 Å, mesh = 230-400 (Merck) using standard techniques. Automated flash column chromatography was performed using ready-to-connect cartridges, on irregular silica gel, particle size 15-40 μm (normal phase disposable flash columns) on different flash systems: either a SPOT or LAFLASH systems from Armen Instrument, or PuriFlash® 430evo systems from Interchim, or 971-FP systems from Agilent, or Isolera 1SV systems from Biotage.

PREPARATION OF INTERMEDIATES

PREPARATION OF INTERMEDIATE 1

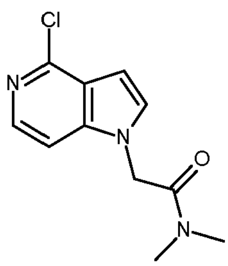

I-1

To a solution of 4-chloro-1H-pyrrolo[3,2-c]pyridine ([60290-21-3], 4.74 g, 31.07 mmol) in DMF (72.16 mL) at 0 °C was added NaH (60% dispersion in mineral oil, 1.74 g, 43.49 mmol) and the reaction mixture was warmed to rt and stirred for 30 min. Then the reaction mixture was cooled to 0 °C and 2-chloro-N,N-dimethylacetamide ([2675-89-0], 3.83 mL, 37.28 mmol) was added and the reaction mixture was warmed to rt for 2 h. $NaHCO_3$ sat. sol. was added and the organic layer was extracted with EtOAc, then washed with water and brine, then dried over $MgSO_4$ and solvent was removed. The residue was purified by flash column chromatography (Heptane/EtOAc from 80/20 to 0/100) to obtain 1-1 (6.34 g, yield 68%) as a white solid.

The following intermediates were prepared in an analogous manner from either 4-bromo-1H-pyrrolo[3,2-c]pyridine ([1000342-68-6]) or 4-chloro-1H-pyrrolo[3,2-c]pyridine ([60290-21-3]) and the indicated reagent.

| REAGENT | INTERMEDIATE |
|---|---|
| 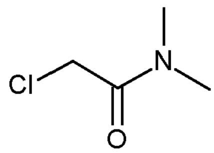 [2675-89-0] | 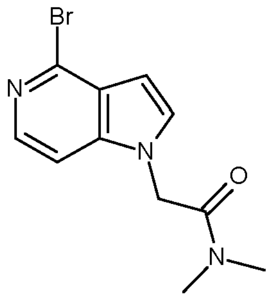 I-2 |
| 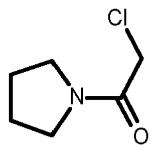 [20266-00-6] | 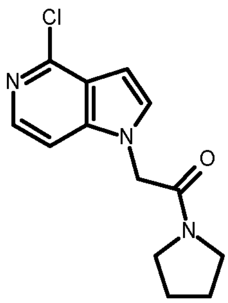 I-3 |
| 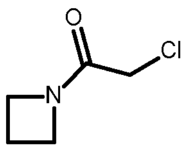 [63177-41-3] | 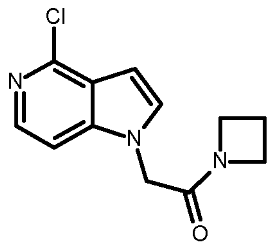 I-4 |
| 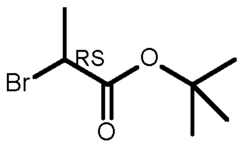 [5292-43-3] | 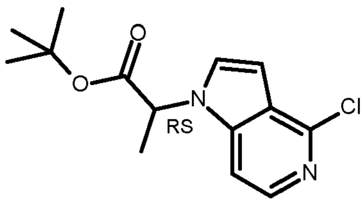 I-5 |
| 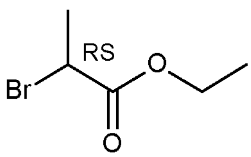 [535-11-5] | 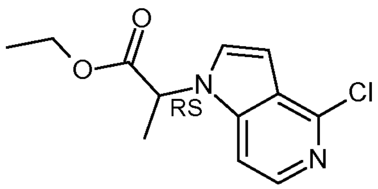 I-6 |

(continued)

| REAGENT | INTERMEDIATE |
|---|---|
| 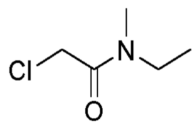 [2746-07-8] | 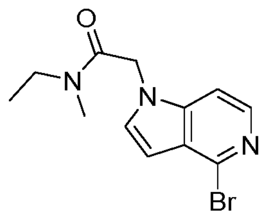 **I-45** |
| 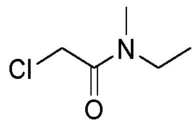 | 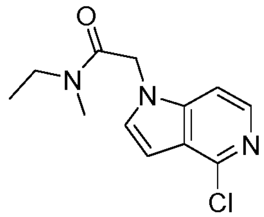 **I-46** |
| 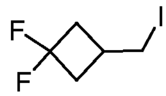 [1434142-22-9] | 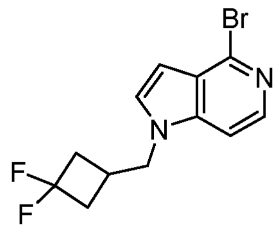 **I-47** |
| 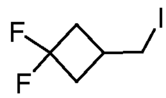 [1434142-22-9] | 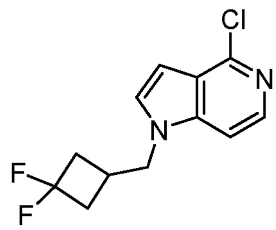 **I-48** |

PREPARATION OF INTERMEDIATE 7

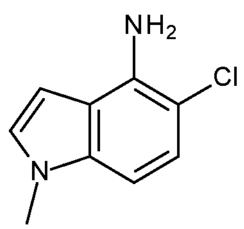

To a mixture of 1-methyl-1H-indol-4-amine ([85696-95-3], 481 mg, 3.29 mmol) in dry DCM (9.62 mL) was added N-chlorosuccinimide (461.32 mg, 3.45 mmol) portionwise at 0 °C and the reaction mixture was stirred at 0 °C for 30 min and then at rt for 30 min. The solvent was then evaporated, and the compound was purified by flash column chroamotgaphy

(silica gel; eluent: DCM/heptane (from 30/70 to 80/20). The desired fractions were concentrated, yielding 1-7 (240 mg, 40%).

The following intermediates were prepared in an analogous manner to that described for **I-7** from the indicated starting material.

| STARTING MATERIAL | INTERMEDIATE |
|---|---|
| 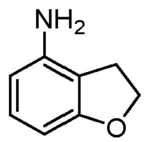 [61090-37-7] | 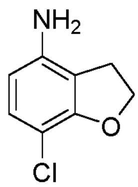 **I-49** |
| 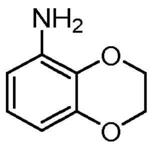 [16081-45-1] | 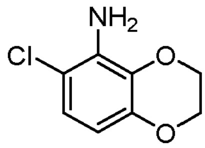 **I-50a** 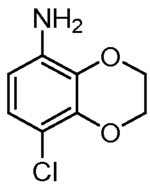 **I-50b** |
| 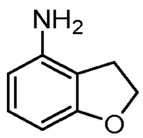 [61090-37-7] | 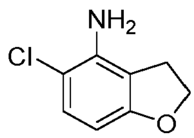 **I-51** |
| 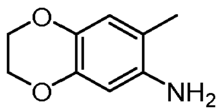 [59820-84-7] | 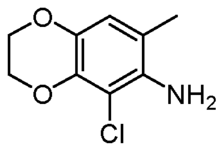 **I-52** |

PREPARATION OF INTERMEDIATE **I-53**

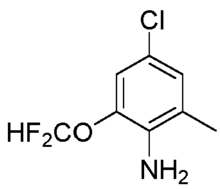

**I-53**

N-Chlorosuccinimide (400 mg, 3.00 mmol) was added to a solution of 2-(difluoromethoxy)-6-methylaniline [139909-66-3] (520 mg, 2.85 mmol) in DMF (9.5 mL). The reaction mixture was stirred at 66 °C for 1 h and poured out in water. The aqueous phase was extracted with EtOAc. The combined organic extracts were washed with brine, dried ($MgSO_4$), filtered and evaporated under reduced pressure. The crude mixture was purified by flash column chromatography (silica, heptane/EtOAc, gradient from 0 to 35 %) to afford **I-53** (476 mg, 80%).

The following intermediates were prepared in an analogous manner to that described for **I-53** from the indicated starting material.

| STARTING MATERIAL | INTERMEDIATE |
|---|---|
| 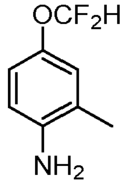 [37211-57-9] | 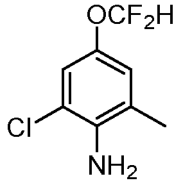 I-54 |
| 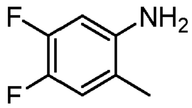 [875664-57-6] | 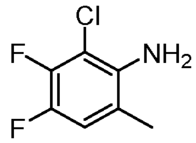 I-55 |
| 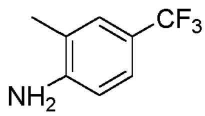 [67169-22-6] | 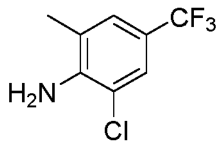 I-56 |

PREPARATION OF INTERMEDIATE 8

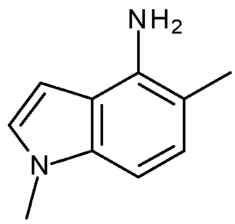

N.B. Use dry glassware and perform reaction under nitrogen atmosphere.

A mixture of I-7 (221 mg, 1.22 mmol), methylzinc chloride ([5158-46-3], 1.22 mL, 2 M, 2.45 mmol) and bis(tri-tert-butylphosphine)palladium(0) ([53199-31-8], 93.79 mg, 0.18 mmol) in dry THF (10 mL) was stirred at rt for 2 h, then stirred at 60 °C for 48 h. The reaction was then quenched with $NH_4Cl$ sat. sol. and evaporated to an aqueous layer which was extracted with DCM, dried over $MgSO_4$ and concentrated. The resulting residue was purified by flash column chromatography (silica gel; eluent: DCM/MeOH from 100/0 to 90/10). The desired fractions were concentrated, yielding 1-8 (42 mg, 21%) as a white solid.

PREPARATION OF INTERMEDIATE 9

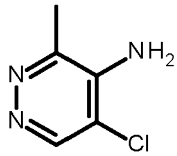

A mixture of 3,5-dichloropyridazin-4-amine ([53180-76-0], 100 mg, 0.61 mmol), trimethylboroxine ([823-96-1], 30.62 mg, 0.24 mmol) and $K_2CO_3$ sat. sol. (12.5 mL) in DME (2.5 mL) were degassed with nitrogen. $Pd(PPh_3)_4$ (42.28

mg, 0.037 mmol) was added and the rm was degassed again. Then the reaction mixture was stirred and heated under nitrogen atmosphere for 4 h at 160 °C in a pressure tube. The solvent was evaporated, the residue was taken up in water/DCM and the organic phase was separated, dried over $MgSO_4$ and evaporated again. The residue was purified by flash column chromatography (silica gel; eluent: DCM/MeOH (100/0 to 92/8). The desired fractions were evaporated, yielding 1-9 (33 mg, yield 37.69%) as a white solid.

PREPARATION OF INTERMEDIATE 10

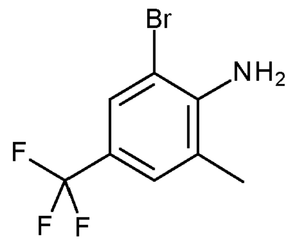

To a suspension of 2-methyl-4-(trifluoromethyl)aniline ([67169-22-6], 5 g, 28.55 mmol), KBr (1.02 g, 8.55 mmol), and hexaammonium molybdate ([12027-67-7], 83.06 mg, 0.071 mmol) in HOAc (25.72 mL, 449.66 mmol) at rt (in Easymax™) was added sodium perborate tetrahydrate ([7632-04-4], 1.21 g, 7.84 mmol). Additional KBr (1.02 g, 8.55 mmol), hexaammonium molybdate (83.06 mg, 0.071 mmol) and sodium perborate tetrahydrate (1.21 g, 7.84 mmol) were added portionwise every 10 min (3x), and after 1 h, the reaction was treated with a final portion of KBr (1.02 g, 8.55 mmol), hexaammonium molybdate (83.06 mg, 0.071 mmol) and sodium perborate tetrahydrate (1.21 g, 7.84 mmol). After 1 h, the mixture was poured into water, neutralized with solid sodium bicarbonate, extracted with diethyl ether, washed with water, then brine, dried over magnesium sulfate, and concentrated to give 1-10 (7.1 g, 98%) as a brown oil.

PREPARATION OF INTERMEDIATE **I-57**

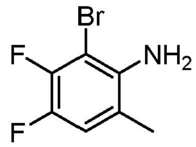

**I-57**

N-Bromosuccinimide (3.26 g, 18.3 mmol) was dissolved in DMF (10 mL) and added dropwise to a solution of 4,5-difluoro-2-methylaniline [875664-57-6] (2.50 g, 17.5 mmol) in anhydrous DMF (21.3 mL) at 0 °C. The reaction mixture was slowly warmed to room temperature and stirred for 15 min. The reaction mixture was poured out in water, extracted with $Et_2O$, dried ($MgSO_4$), filtered and concentrated under reduced pressure. The residue was purified by flash column chromatography (silica, heptane/EtOAc, gradient from 100:0 to 70:30) to afford **I-57** (1.8 g, 46%) as a brown solid.

The following intermediates were prepared in an analogous manner to that described for **I-57** from the indicated starting materials.

| STARTING MATERIAL | INTERMEDIATE |
|---|---|
| 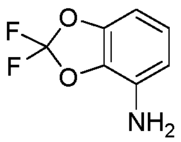 [106876-54-4] | 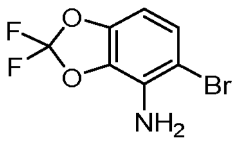 **I-58** |

(continued)

| STARTING MATERIAL | INTERMEDIATE |
|---|---|
| 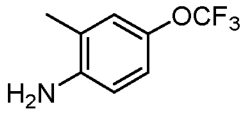 [86256-59-9] | 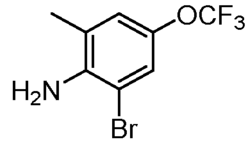 **I-59** |

PREPARATION OF INTERMEDIATE **I-60**

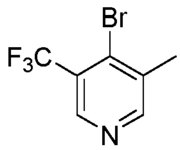

**I-60**

Zinc sulfinate (1.0 g, 3.00 mmol) was added to a solution of 3-bromo-4-methylpyridine (300 mg, 1.74 mmol) in a mixture of $CHCl_3$ (9.8 mL) and $H_2O$ (3 mL) at 0 °C and the reaction mixture was stirred vigorously. *tert*-Butyl hydroperoxide (70% purity, 0.72 mL, 5.23 mmol) was added dropwise and the reaction mixture was stirred at 50 °C for 48 h. Additional quantity of zinc sulfinate (1.00 g, 3.00 mmol) was added and the reaction mixture was stirred at 50 °C for another 24 h. The mixture was cooled to room temperature, and a saturated solution of $NaHCO_3$/EDTA was added until pH=7. The solution was diluted with DCM and washed with EDTA disodium salt/$NaHCO_3$ solution. The aqueous layer was extracted with DCM and the combined organic layers were washed with brine, dried ($MgSO_4$), filtered and concentrated under reduced pressure. The crude mixture was purified by flash column chromatography (silica, DCM) to afford **I-60** (165 mg, 34%, 88% purity) as a white solid.

PREPARATION OF INTERMEDIATE 11

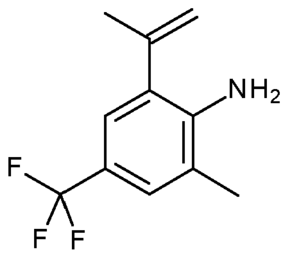

A mixture of I-10 (4 g, 15.74 mmol), isoprene boronic acid pinacolester ([126726-62-3], 2910.43 mg, 17.32 mmol) and $Pd(PPh_3)_4$ (1091.67 mg, 0.94 mmol) in $K_2CO_3$ sat. sol. (0.5 mL) and DME (64.55 mL) was stirred and heated under nitrogen atmosphere for 90 min at 120 °C in a pressure tube. The solvent was then evaporated, the residue was taken up in water/DCM and the organic phase was separated, dried over $MgSO_4$ and evaporated again. The residue was purified by column chromatography (silica gel; eluent: heptane/EtOAc, gradient from 100/0 to 50/50). The pure fractions were evaporated, yielding 1-11 (2.19 g, 65%) as a brown oil.

The following intermediates were prepared in an analogous manner to that described for **I-11** from the indicated starting materials and reagents.

| STARTING MATERIAL | REAGENT | INTERMEDIATE |
|---|---|---|
| 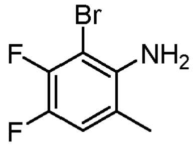 I-57 | 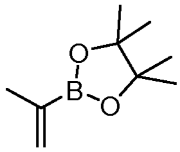 [126726-62-3] | 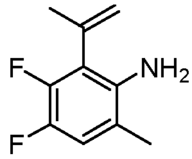 I-61 |
| 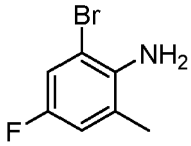 [202865-77-8] | 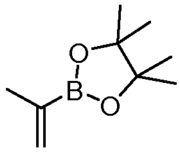 [126726-62-3] | 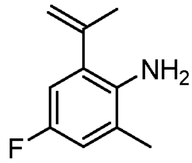 I-62 |

PREPARATION OF INTERMEDIATE 12

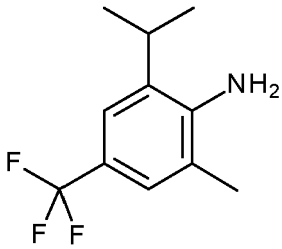

A mixture of I-11 (2.19 g, 10.18 mmol), Pd/C (10%, 1082.9 mg, 1.02 mmol) in MeOH (266.37 mL) was hydrogenated with hydrogen at rt for 2 h. The catalyst was filtered and the filtrate was evaporated, yielding 1-12 (2.2 g, yield 99.52%) as a brown oil.

The following intermediates were prepared in an analogous manner to that described for **I-12** from the indicated starting materials and reagents.

| STARTING MATERIAL | INTERMEDIATE |
|---|---|
| 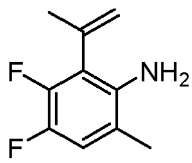 I-61 | 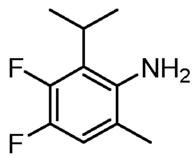 I-63 |
| 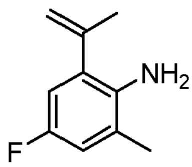 I-62 | 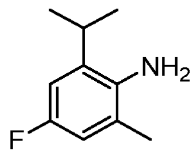 I-64 |

PREPARATION OF INTERMEDIATE 13

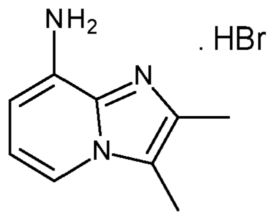

A solution of 2,3-pyridinediamine (112 g, 1.03 mmol) and 3-bromo-2-butanone (190 g, 1.26 mmol) in EtOH (1.85 L) was refluxed under $N_2$ for 22 h. The reaction mixture was then cooled to rt, the solid was filtered and washed with EtOH (200 mL ca), recrystallized from $Et_2O$ and dried in vacuo to yield 1-13 (92.3 g, 37%).

PREPARATION OF INTERMEDIATE 14

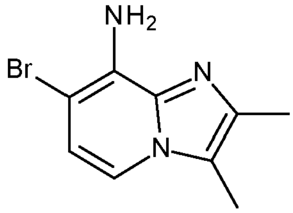

To a solution of I-13 (36.3 g, 0.15 mmol) and NaOAc anhydrous (5.56 g, 0.07 mmol) in AcOH (100 mL) at 50 °C was added $Br_2$ (24.21 g, 0.15 mmol) in AcOH (120 mL) over 1.5 h and the mixture was stirred overnight at 50 °C. The mixture was then cooled to rt and $Et_2O$ (1.5 L) were added, the resulting solid was filtered off and washed with $Et_2O$. The solid was added to NaOH (3N, 1.3 L) and stirred at rt for 20 min, then the aqueous layer was extracted with DCM (3 x 500 mL). The combined organic layers were drived over MgSO4, concentrated and purified by column chromatography (silica; eluent DCM/MeOH(NH3) 100 to 98/2). The resulting solid was triturated with DCM to yield 1-14 (20 g, 56%)

PREPARATION OF INTERMEDIATE 15

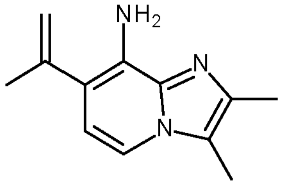

To a solution of I-14 (1000 mg, 4.165 mmol) in dioxane (10 mL) and $NaHCO_3$ aq. soln. (10 mL) was added 4,4,5,5-tetramethyl-2-(1-methylethenyl)-1,3,2-dioxaborolane ([126726-62-3], 839.86 mg, 4.998 mmol) and $Pd(PPh_3)_4$ (481.288 mg, 0.416 mmol), and the reaction was then heated using a MW at 150°C for 10 min. The reaction mixture was diluted using EtOAc and washed with brine, the organic fraction was dried over $MgSO_4$ and concentrated in vacuo. The residue was purified by flash column chromatography (silica, DCM/($NH_3$ in MeOH), gradient from 100/0 to 97/3) to afford 1-15 (750 mg, yield 89.47%).

The following intermediates were prepared in an analogous manner to that described for 1-15 from the indicated starting materials and reagents.

| STARTING MATERIAL | REAGENT | INTERMEDIATE |
|---|---|---|
| 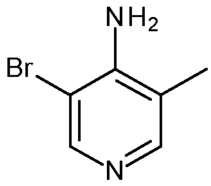 [97944-43-9] (1000 mg, 5.346 mmol) | 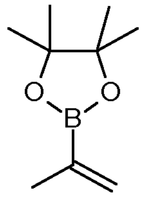 [126726-62-3] | 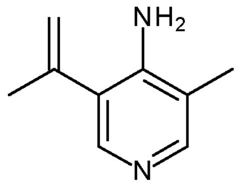 1-16 (650 mg, 82%) |
| 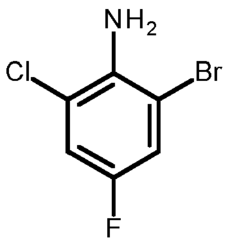 [201849-14-1] (350 mg, 1.559 mmol) | 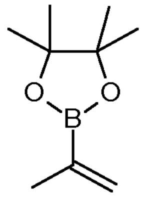 [126726-62-3] | 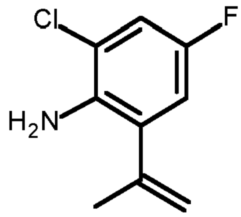 1-17 (135.7mg, 47%) |
| 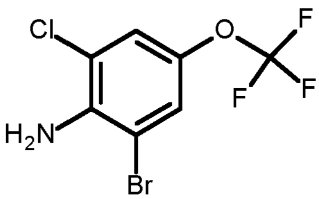 [1228108-74-4] (485mg, 167 mmol) | 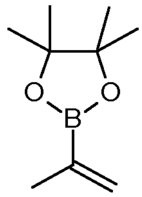 [126726-62-3] | 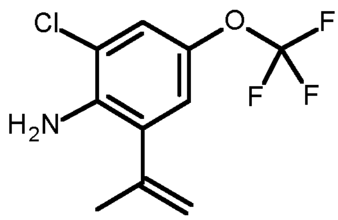 I-18 (62.7mg, 14.9%) |
| 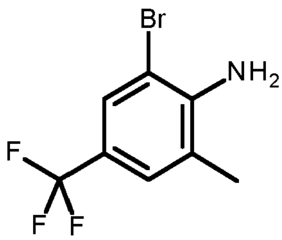 [1100212-65-4] | 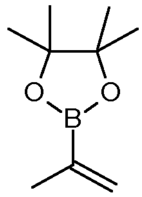 [126726-62-3] | 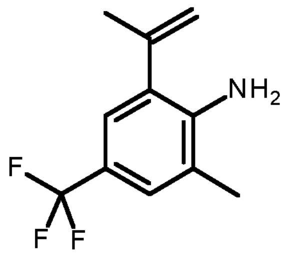 1-19 (2.19g, 64%) |
| 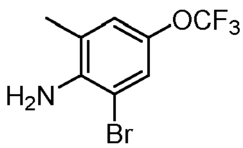 I-59 | 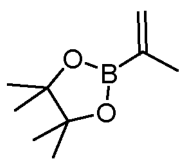 [126726-62-3] | 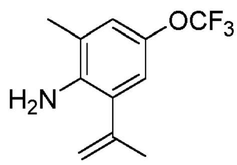 I-65 |

PREPARATION OF INTERMEDIATE 20

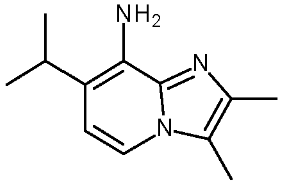

To a solution of I-15 (750 mg, 3.726 mmol) in EtOH (20 mL) was added Pd/C (10%, 3965.545 mg, 3.73 mmol), the reaction mixture was stirred under an atmosphere of hydrogen for 2 h. The reaction mixture was filtered through diatomaceous earth and concentrated in vacuo. The crude mixture was purified by column chromatography (silica, DCM/($NH_3$ in MeOH), gradient from 100/0 to 97/3) to afford 1-20 (680 mg, 90%).

The following intermediates were prepared in an analogous manner to that described for 1-20 from the indicated starting materials and reagents.

| STARTING MATERIAL | INTERMEDIATE |
|---|---|
| 1-16 | 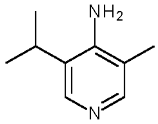 1-21 (900 mg, 89%) |
| 1-17 | 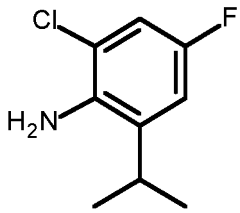 1-22 (77.2 mg, 56%) |
| 1-18 | 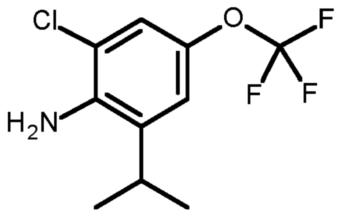 1-23 (52.5 mg, 83%) |
| 1-19 | 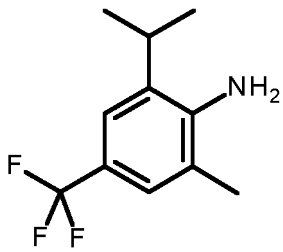 1-24 (2.2 g, 99%) |
| 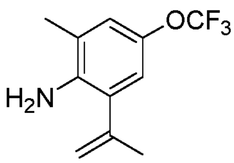 I-65 | 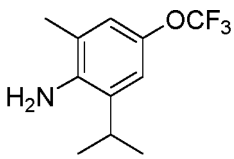 I-66 |

PREPARATION OF INTERMEDIATE **I-67**

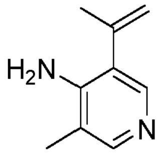

**I-67**

A mixture of 3-bromo-5-methylpyridine-4-amine (5.00 g, 27 mmol), isopropenylboronic acid pinacol ester (6.70 g, 40 mmol), $Pd(PPh_3)_4$ (3.20 g, 2.70 mmol), 1,4-dioxane (50 mL), and $NaHCO_3$ (sat. aq., 50 mL) was stirred under reflux for 16 h. Then the suspension was cooled and diluted with water and DCM until clear phase separation. The aqueous phase was extracted with DCM. The combined organic extracts were dried ($MgSO_4$), filtered and concentrated under reduced pressure. The crude mixture was purified by flash column chromatography (silica, mobile phase gradient: 0-3% 7N $NH_3$/MeOH in DCM). The residue was combined with another fraction (1.5 g) and dissolved in *i*-PrOH (20 mL). The mixture was treated with HCl (6M in *i*-PrOH, 9 mL, 54 mmol) and stirred over the weekend. The mixture was ice-cooled and the product was collected by filtration to afford **I-67** (4.5 g, 76%) as a white solid.

The following intermediate was prepared in an analogous manner to that described for **I-67** from the indicated starting material and reagent.

| STARTING MATERIAL | REAGENT | INTERMEDIATE |
|---|---|---|
| 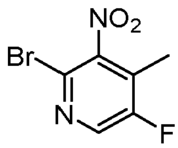 [97944-43-9] | 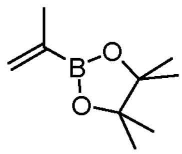 [126726-62-3] | 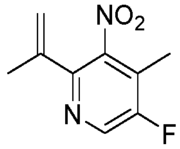 **I-68** |

PREPARATION OF INTERMEDIATE **I-69**

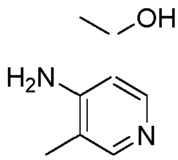

**I-69**

**I-67** (1.5 g, 8.12 mmol) was cooled to 10° C and $H_2SO_4$ (3.42 mL, 50% in water) was added dropwise with stirring over 10 min. The reaction mixture was stirred at 0 °C over the week end. The reaction mixture was added to an ice-cold solution of NaOH (100 mL) and $K_2CO_3$ was added. The aqueous phase was extracted with $CHCl_3$. The solvent was evaporated under reduced pressure and the residue was stirred with $Et_2O$. The resulting solid was filtered off and dried at ... °C to afford **I-69** (449 mg, 33%).

PREPARATION OF INTERMEDIATE **I-70**

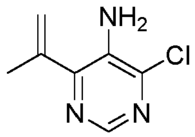

**I-70**

A sealed tube was charged with 5-amino-4,6-dichloropyrimidine [5413-85-4] (1.50 g, 9.15 mmol), isopropenylboronic acid pinacol ester (1.72 mL, 9.15 mmol), $Pd(PPh_3)_4$ (1.09 g, 9.24 mmol), 1,4-dioxane (22 mL), and $NaHCO_3$ (sat., aq., 22 mL). The reaction mixture was stirred under reflux for 16 h, cooled to room temperature and diluted with water and DCM until clear phase separation. The aqueous phase was extracted with DCM. The combined organic extracts were dried ($MgSO_4$), filtered and concentrated under reduced pressure to afford **I-70** which was used as such in the next step.

PREPARATION OF INTERMEDIATE **I-71**

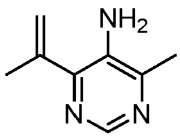

**I-71**

To a suspension of **I-70** in 1,4-dioxane (16.3 mL) was added trimethylboroxine (3.7 mL, 26.5 mmol). $Pd(dppf)Cl_2$ (324 mg, 442 μmol) and $K_2CO_3$ (3.67 g, 26.5 mmol) were added and the reaction mixture was purged with $N_2$. The reaction mixture was stirred at 90 °C overnight. The reaction mixture was diluted with EtOAc, filtered and evaporated under reduced pressure. The crude mixture was purified via Prep HPLC (stationary phase: RP Vydac Denali C18 - 10μm, 200g, 5cm I.D., mobile phase: 0.25% $NH_4HCO_3$ solution in water, MeOH) to afford **I-71** (612 mg, 49% over 2 steps).

The following intermediates were prepared in an analogous manner to that described for **I-71** from the indicated starting materials.

| STARTING MATERIAL | INTERMEDIATE |
|---|---|
| 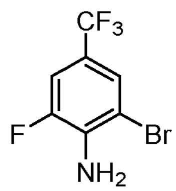 [1034325-63-7] | 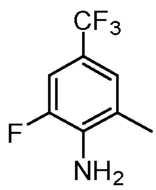 **I-72** |
| 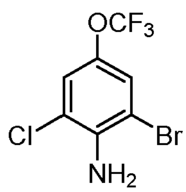 [1228108-74-4] | 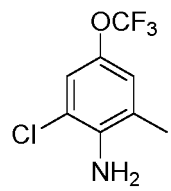 **I-73** |

PREPARATION OF INTERMEDIATE **I-74**

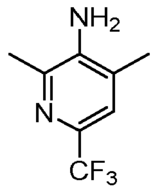

**I-74**

2,4-Dibromo-6-(trifluoromethyl)pyridine-3-amine [1214365-67-9] (900 mg, 2.81 mmol) was dissolved in a mixture of 1,4-dioxane (7.2mL) and water(0.9mL). Trimethylboroxine (1.13 mL, 8.07 mmol), Pd(ddpf)$Cl_2$•DCM (206 mg, 0.25 mmol) and $K_2CO_3$ (1.17 g, 8.47 mmol) were added and the reaction mixture was heated at 140 °C for 1 h in a microwave. The mixture was combined with another fraction (0.31 mmol) and diluted with water and EtOAc. The aqueous layer was extracted. The combined organic extracts were washed with brine, dried ($MgSO_4$), filtered and evaporated under reduced pressure. The crude mixture was purified by flash column chromatography (silica, DCM) to afford **I-74** (424 mg, 72%)

The following intermediate was prepared in an analogous manner to that described for **I-74** from the indicated starting material.

| STARTING MATERIAL | INTERMEDIATE |
|---|---|
| 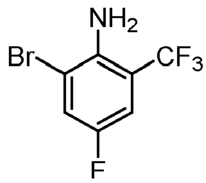 [875664-27-0] | 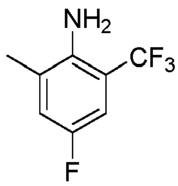 **I-75** |

PREPARATION OF INTERMEDIATE **I-76**

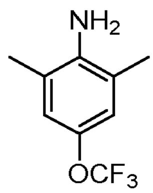

**I-76**

A mixture of 2,6-dibromo-4-(trifluoromethoxy)aniline [88149-49-9] (1.00 g, 2.99 mmol), trimethylboroxine (0.93 mL, 6.55 mmol), $Pd(PPh_3)_4$ (207 mg, 0.18 mmol) and $K_2CO_3$ (1.24 g, 8.96 mmol) in 1,4-dioxane (1.56 mL) was stirred at 130 °C for 2 h. The reaction mixture was diluted with water and DCM. The layers were separated and the organic phase was dried ($MgSO_4$), filtered and evaporated under reduced pressure. The crude mixture was purified by flash column chromatography (silica, heptane/EtOAc, gradient from 100:0 to 70:30) to afford **I-76** (220 mg, 36%).

PREPARATION OF INTERMEDIATE **I-77**

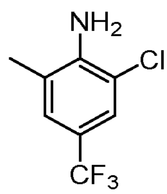

**I-77**

A sealed tube was charged with 2-bromo-6-chloro-4-(trifluoromethyl)aniline [109919-26-8] (1.00 g, 3.64 mmol) , trimehtylboroxine (0.61 mL, 4.37 mmol), $Pd(PPh_3)_4$ (0.43 g, 368 μmol), 1,4-dioxane (8.6 mL), $K_2CO_3$ (1.00 g, 7.29 mmol) and water (1.32 mL). The reaction mixture was stirred at 90 °C overnight, and at 120 °C for another 2 h. The mixture was cooled to room temperature and diluted with water and DCM until clear phase separation. The aqueous phase was extracted with DCM. The combined organic extracts were dried ($MgSO_4$), filtered and concentrated under reduced pressure. The crude mixture was purified by flash column chromatography (silica, heptane/EtOAc, gradient from 100:0 to 90:10) to afford **I-77** (376 mg, 49%).

PREPARATION OF INTERMEDIATE **I-78**

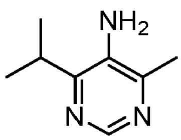

**I-78**

To a solution of **I-71** (612 mg, 4.10 mmol) in MeOH (25 mL) was added Pd/C (5%, 175 mg, 0.08 mmol) and the reaction mixture was stirred at room temperature for 1 h under $H_2$ atmosphere. The reaction mixture was filtered and the filtrate was evaporated under reduced pressure to afford **I-78** (634 mg, 85%).

PREPARATION OF INTERMEDIATE **I-79**

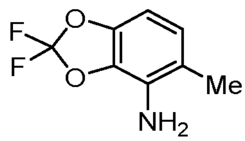

**I-79**

$Pd(dppf)Cl_2 \cdot CH_2Cl_2$ (85.7 mg, 0.11 mmol) was added to a stirred mixture of methylboronic acid [13061-96-6] (151 mg, 2.52 mmol), **I-58** (529 mg, 2.10 mmol) and $Na_2CO_3$ (667 mg, 6.30 mmol) in 1,4-dioxane (12 mL) and water (3 mL) in a sealed tube and under $N_2$ atmosphere. The reaction mixture was stirred at 90 °C for 16 h and diluted with water. The mixture was extracted with EtOAc. The organic layer was dried ($MgSO_4$), filtered and evaporated under reduced pressure. The crude mixture was purified by flash column chromatography (silica, heptane/EtOAc, gradient from 100:0 to 90:10) to afford **I-79** as a colorless oil.

The following intermediate was prepared in an analogous manner to that described for **I-79** from the indicated starting material and reagent.

| STARTING MATERIAL | REAGENT | INTERMEDIATE |
|---|---|---|
| 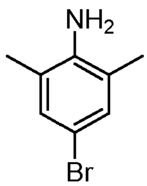 [24596-19-8] | 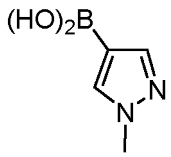 [847818-55-7] | 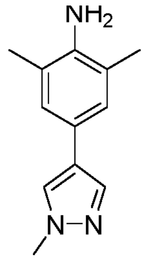 **I-80** |

PREPARATION OF INTERMEDIATE **I-81**

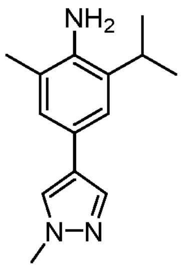

**I-81**

A mixture of 4-bromo-2-isopropyl-6-methylaniline [773887-07-3] (871 mg, 3.82 mmol), 1-methyl-1H-pyrazole-4-boronic acid [847818-55-7] (744 mg, 4.58 mmol) and $Na_2CO_3$ (1.21 g, 11.4 mmol) in 1,4-dioxane (16.3 mL) and water (68.9 μL) was purged with $N_2$ for 5 min. $PdCl_2$(dppf) (156 mg, 0.19 mmol) was added and the reaction mixture was stirred at 100 °C for 6 h. The mixture was diluted with water and EtOAc.

The aqueous phase was extracted. The combined organic extracts were dried ($MgSO_4$), filtered and concentrated under reduced pressure. The crude mixture was purified by flash column chromatography (silica, heptane/EtOAc, gradient from 100:0 to 50:50) to afford **I-81** (855 mg, 97%).

PREPARATION OF INTERMEDIATE **I-82**

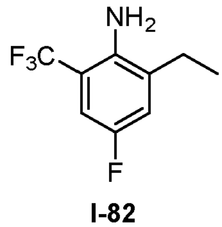

**I-82**

To a suspension of 2-bromo-4-fluoro-6-(trifluoromethyl)aniline [875664-27-0] (1.00 g, 3.88 mmol) in 1,4-dioxane (10 mL) were added ethylboronic acid [4433-63-0] (573 mg, 7.75 mmol), Pd(dppf)$Cl_2$ (142 mg, 0.19 mmol) and $K_2CO_3$ (1.61 g, 11.6 mmol). The reaction mixture was stirred at 120 °C for 3 h under $N_2$ atmosphere in a sealed tube. The reaction mixture was diluted with EtOAc, filtered and evaporated under reduced pressure. The crude mixture was purified by flash column chromatography (silica, heptane/EtOAc, gradient from 100:0 to 70:30) to afford **I-82** (0.51 g, 63%).

PREPARATION OF INTERMEDIATE **I-83**

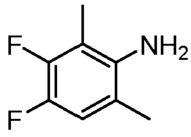

**I-83**

A mixture of **I-57** (650 mg, 2.93 mmol) in anhydrous THF (14.6 mL) was degassed for 10 min with $N_2$. Pd(*t*-$Bu_3P)_2$ (43.9 mg, 85.9 μmol) was added followed by methylzinc chloride (2M in THF, 2.20 mL, 4.40 mmol) with a syringe while the temperature of the reaction mixture was maintained to room temperature. The reaction mixture was stirred for 1 h and diluted with water. The mixture was filtered through dicalite and the filtrate was evaporated under reduced pressure (water remained). Water (20 mL) was added and the residue was extracted with DCM. The organic phase was dried ($MgSO_4$), filtered and volatiles were evaporated under reduced pressure. The crude mixture was purified by flash column chromatography (silica, heptane/EtOAc, gradient from 100:0 to 70:30) to afford **I-83** (430 mg, 93%).

SYNTHESIS OF INTERMEDIATE **I-84**

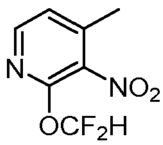

**I-84**

NaH (60% dispersion in mineral oil, 649 mg, 16.2 mmol) was added to a slurry of 2-hydroxy-4-methyl-3-nitropyridine [21901-18-8] (1.00 g, 6.49 mmol) in MeCN (70 mL) at 0 ° C and under $N_2$ atmosphere. The reaction mixture was stirred for 45 min at room temperature and 2,2-difluoro-(fluorosulfonyl)acetic acid [1717-59-5] (0.89 mL, 8.37 mmol) was added dropwise. The reaction mixture was stirred at 20 °C overnight. $NH_4Cl$ (sat., aq.) was added and the mixture was extracted with EtOAc (twice). The combined organic extracts were washed with brine, dried ($MgSO_4$), filtered and concentrated to dryness. The crude mixture was purified by flash column chromatography (silica, heptane/EtOAc, gradient from 100:0 to 50:50) to afford **I-84** (670 mg, 51%).

The following intermediates were prepared in an analogous manner to that described for **I-84** from the indicated starting material.

| STARTING MATERIAL | INTERMEDIATE |
|---|---|
| 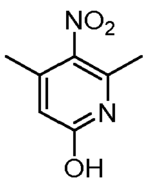 [22934-24-3] | 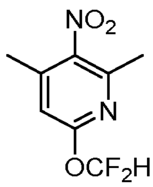 **I-85a** 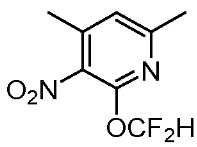 **I-85b** |

PREPARATION OF INTERMEDIATE **I-86**

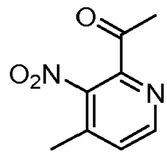

**I-86**

To a solution of 2-bromo-4-methyl-3-nitropyridine [23056-45-3] (4.00 g, 18.4 mmol) in toluene (200 mL) were added tributyl(1-ethoxyvinyl)tin (10.2 mL, 30.2 mmol) and $Pd(PPh_3)_4$ (2.34 g, 2.03 mmol). The reaction mixture was stirred for 16 h at 100 °C. Additional quantity of tributyl(1-ethoxyvinyl)tin (3.5 mL) was added and the reaction mixture was stirred for another 4 h. HCl (37% in $H_2O$, 38.5 mL, 461 mmol) was added at 0 °C and the mixture was stirred for 1 h at room temperature. The reaction mixture was quenched with KF (aq., 100 mL). The mixture was extracted with EtOAc (3 x 50 mL). The combined organic extracts were washed with brine, dried ($Na_2SO_4$), filtered and evaporated under reduced pressure. The crude mixture was purified by flash column chromatography (silica, heptane/EtOAc, gradient from 100:0 to 70:30) to afford **I-86** (2.64 g, 72%)

PREPARATION OF INTERMEDIATE **I-87**

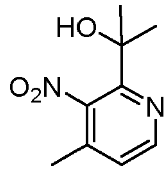

**I-87**

To a solution of **I-86** (1.45 g, 8.05 mmol) in THF (19.2 mL) at 0 °C was added dropwise methylmagnesium bromide (1.4 M solution, 7.67 mL, 10.7 mmol). The reaction mixture was stirred at room temperature for 3 h. Additional quantity of methylmagnesium bromide (0.5 eq) was added and the reaction mixture was stirred for another hour. The reaction was quenched with $NH_4Cl$ (sat., aq.), and extracted with EtOAc (3 times). The combined organic extracts were dried ($Na_2SO_4$), filtered and concentrated under reduced pressure. The crude mixture was purified by flash column chromatography (silica, DCM/MeOH, gradient from 100:0 to 99:1) to afford **I-87** (977 mg, 41%).

PREPARATION OF INTERMEDIATE **I-88**

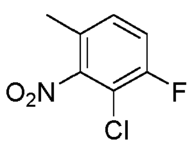

**I-88**

To a solution of 4-fluoro-2-nitrotoluene [446-10-6] (1.00 g, 6.45 mmol) in THF (10 mL) at -78 °C was added N-chlorosuccinimide (2.58 g, 19.3 mmol) and sodium bis(trimethylsilyl)amide (1M solution, 12.9 mL, 12.9 mmol) while maintaining the internal temperature of the reaction mixture below -75 °C. The reaction mixture was stirred at -78 °C for 30 min and partitioned between HCl (5%) and EtOAc. The layers were separated and the organic phase was dried ($MgSO_4$), filtered and the solvent was evaporated under reduced pressure. The crude mixture was purified by flash column chromatography (silica, heptane/EtOAc) to afford **I-88** (3.62 g, 59%).

PREPARATION OF INTERMEDIATE **I-89**

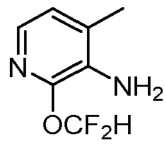

**I-89**

**I-84** (0.81 g, 3.97 mmol) was dissolved in EtOH (22 mL), THF (7.4 mL) and water (7.4 mL). Iron (1.77 g, 31.7 mmol) and $NH_4Cl$ (2.55 g, 47.6 mmol) were added. The reaction mixture was stirred in a sealed tube at 60 °C for 2 h. The reaction mixture was diluted in EtOH and filtered through Celite®. The Celite® pad was washed with EtOH, and the filtrate was concentrated under reduced pressure to ~ 2 mL. The solution was diluted with DCM, washed with $NaHCO_3$ (sat., aq.), dried ($MgSO_4$), filtered and evaporated under reduced pressure to afford **I-89** (685 mg, 79% yield, 80% purity).

The following intermediates were prepared in an analogous manner to that described for **I-89** from the indicated starting materials.

| STARTING MATERIAL | INTERMEDIATE |
|---|---|
| 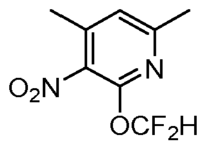 **I-85b** | 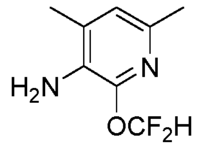 **I-90** |
| 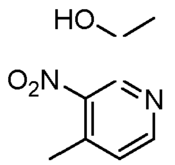 **I-87** | 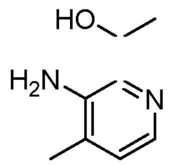 **I-91** |
| 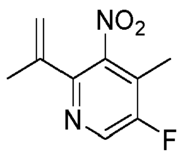 **I-68** | 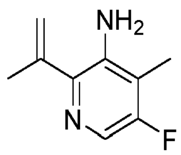 **I-92** |
| 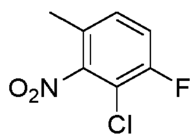 **I-88** | 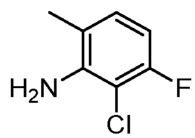 **I-93** |

PREPARATION OF INTERMEDIATE **I-94**

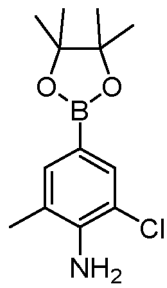

**I-94**

To a solution of 4-bromo-2-chloro-6-methylaniline [30273-42-8] (650 mg, 2.95 mmol) in DMF (20 mL) were added bis(pinacolato)diboron (1.05 g, 4.13 mmol), KOAc (0.87 g, 8.84 mmol) and $PdCl_2$(dppf) (216 mg, 0.295 mmol). The reaction mixture was stirred at 100 °C for 1 h. The reaction mixture was poured out in water and the aqueous phase was extracted with EtOAc (twice). The combined organic layers were passed through a Celite® plug. The filtrate was washed with brine, dried ($MgSO_4$), filtered and concentrated under reduced pressure to afford **I-94** (780 mg) which was used as such in the next step.

PREPARATION OF INTERMEDIATE **I-95**

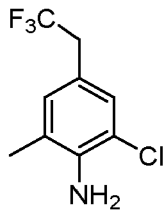

**I-95**

XantPhos (123 mg, 0.21 mmol) and $Pd_2dba_3$ (66.7 mg, 72.9 μmol) were added to a mixture of **I-94** and $CF_3CH_2I$ (0.58 mL, 5.83 mmol) in 1,4-dioxane (32.8 mL) under $N_2$ atmosphere. Water (1.45 mL) and $CS_2CO_3$ (3.80 g, 11.7 mmol) were successively added and the reaction mixture was stirred at 80 °C overnight. The reaction mixture was cooled to room temperature, diluted with 1,4-dioxane and filtered through a pad of Celite®. The filtrate was washed with water and brine, dried ($MgSO_4$), filtered and evaporated under reduced pressure. The crude mixture was purified by flash column chromatography (silica, heptane/EtOac, gradient from 100:0 to 0:100) to afford **I-95** (147 mg, 15% over 2 steps).

PREPARATION OF INTERMEDIATE **I-96**

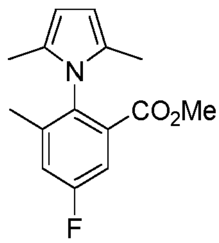

**I-96**

A mixture of methyl 2-amino-5-fluoro-3-methylbenzoate [952479-98-0] (3.21 g, 17.5 mmol) and 2,5-hexanedione [110-13-4] (4.11 mL, 35.0 mmol) in acetone (30 mL) was stirred under reflux for 2 h. The reaction mixture was cooled to room temperature and poured into cold water. The mixture was diluted with DCM. The layers were separated and the

aqueous phase was extracted with DCM. The combined organic layers were dried ($MgSO_4$), filtered and concentrated under reduced pressure. The crude mixture was purified by flash column chromatography (silica, heptane/EtOAc, gradient from 100:0 to 90:10) to afford **I-96** (1.77 g, 39%).

PREPARATION OF INTERMEDIATE **I-97**

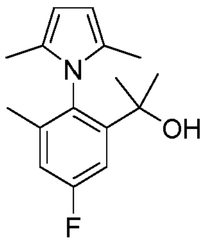

**I-97**

To a solution of **I-96** (1.77 g, 6.77 mmol) in THF (24.4 mL) at 0° C was added dropwise methylmagnesium bromide (1.4 M solution, 29.0 mL, 40.6 mmol). The reaction mixture was stirred at room temperature for 4 h. The reaction was quenched with $NH_4Cl$ (sat., aq.), and extracted with EtOAc (3 times). The combined organic layers were dried ($Na_2SO_4$), filtered and concentrated under reduced pressure. The crude mixture was purified by flash column chromatography (silica, heptane/EtOAc, gradient from 100:0 to 90:10) to afford **I-97.**

PREPARATION OF INTERMEDIATE **I-98**

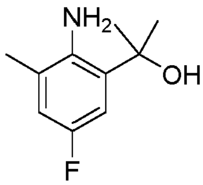

**I-98**

A solution of **I-97** in EtOH (61.7 mL) and water (33 mL) was treated with hydroxylamine hydrochloride (15.6 g, 225 mmol) and KOH (7.104 g, 126.6 mmol) and heated at 100 °C overnight. The mixture was cooled to room temperature and the residue was extracted with EtOAc (twice). The combined organic phases were washed with brine, dried ($Na_2SO_4$), filtered and concentrated under reduced pressure. The crude mixture was purified by flash column chromatography (silica, heptane/EtOAc, gradient from 100:0 to 50:50) to afford **I-98** (220 mg, 17% over 2 steps).

PREPARATION OF INTERMEDIATE **I-99**

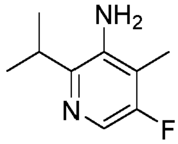

**I-99**

To a solution of **I-92** (233 mg, 1.40 mmol) in THF (17 mL) was added platinum (5.46 mg, 0.03 mmol) and the reaction mixture was stirred at room temperature for 1 h under $H_2$ atmosphere. The reaction mixture was filtered and the filtrate was concentrated under reduced pressure. The residue was combined with another fraction (1.40 mmol) and

purified by flash column chromatography (silica, DCM/MeOH, gradient from 100:0 to 90:10) to afford **I-99** (224 mg, 48%).

PREPARATION OF INTERMEDIATE **I-100**

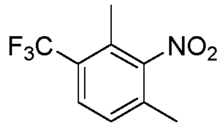

**I-100**

A mixture of 1-iodo-2,4-dimethyl-3-nitrobenzene [56404-21-8] (1.50 g, 5.41 mmol), methyl 2,2-difluoro-2-(fluorosulfonyl)acetate [680-15-9] (6.24 g, 32.5 mmol), CuI (1.24 g, 6.50 mmol) and DIPEA (0.70 g, 5.41 mmol) in anhydrous DMF (13.9 mL) was stirred at 80 °C for 2 h. The mixture was poured out in water and the aqueous phase was extracted with $Et_2O$. The combined organic extracts were washed with brine, dried ($MgSO_4$), filtered and concentrated under reduced pressure. The crude mixture was purified by flash column chromatography (silica, pentane/$Et_2O$, gradient from 100:0 to 90:10) to afford **I-100** (840 mg, 71%) as a yellow oil.

PREPARATION OF INTERMEDIATE **I-101**

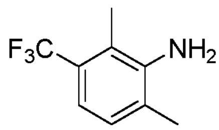

**I-101**

To a solution of **I-100** (1.12 g, 5.11 mmol) in MeOH (49.1 mL) was added Pd/C (10%, 5.44 mg, 5.1 µmol). The reaction mixture was stirred at room temperature for 18 h under $H_2$ atmosphere. The catalyst was filtered off and the filtrate was evaporated under reduced pressure. The residue was purified by flash column chromatography (silica, heptane/EtOAc, gradient from 100:0 to 80:20) to afford **I-101** (810 mg, 84%) as a colourless oil.

PREPARATION OF INTERMEDIATE **I-102**

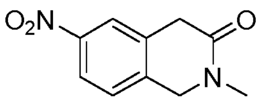

**I-102**

Phosphorus pentoxide (2.35 g, 16.5 mmol) was added to methanesulfonic acid (19.5 mL, 301 mmol) under $N_2$ atmosphere and the mixture was stirred at room temperature for 5 h. *N*-Methyl-2-(3-nitrophenyl)acetamide [19281-10-8] (2.60 g, 13.4 mmol) and paraformaldehyde (508 mg, 16.1 mmol) were added and the reaction mixture was stirred at 80 °C for 48 h. The reaction mixture was cooled to 0 °C and diluted with water. The residue was dissolved in EtOAc and the pH was adjusted to 8 with NaOH (5M, aq.). The aqueous phase was extracted with EtOAc. The combined organic extracts were dried ($MgSO_4$), filtered and concentrated under reduced pressure. The crude mixture was purified by flash column chromatography (silica, heptane/EtOAc, gradient from 100:0 to 0:100) to afford **I-102** (302 mg, 11%) as a white solid.

PREPARATION OF INTERMEDIATE **I-103**

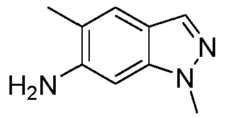

**I-103**

Pd/C (10% purity, 71.7 mg, 67.3 μmol) was added to a solution of 1,5-dimethyl-6-nitro-1H-indazol [78416-45-2] (515 mg, 2.69 mmol) in EtOH (10 mL). The mixture was purged with $H_2$ and the reaction mixture was stirred at 50 °C under $H_2$ atmosphere for 16 h. The mixture was filtered over Celite® and washed with EtOAc. The filtrate was concentrated under reduced pressure. The crude mixture was purified by flash column chromatography (silica, heptane/EtOAc, gradient from 100: 0 to 50:50) to afford **I-103** (315 mg, 72%) as an orange solid.

The following intermediate was prepared in an analogous manner to that described for **I-103** from the indicated starting material.

| STARTING MATERIAL | INTERMEDIATE |
|---|---|
| 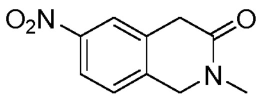 **I-102** | 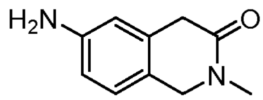 **I-104** |

PREPARATION OF INTERMEDIATE **I-105**

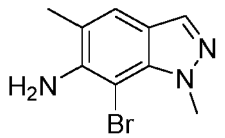

**I-105**

**I-103** (310 mg, 1.92 mmol) was dissolved in DCM (15 mL). A solution of bromine (0.10 mL, 2.02 mmol) in DCM (4 mL) was added dropwise. The reaction mixture was stirred at room temperature for 3 h and diluted with DCM. The mixture was washed with water, dried ($MgSO_4$), filtered and volatiles were removed under reduced pressure. The crude mixture was purified by flash column chromatography (silica, heptane/EtOAc, gradient from 100:0 to 80:20) to afford 1-105 (362 mg, 78%) as a light orange solid.

The following intermediates were prepared in an analogous manner to that described for **I-105** from the indicated starting materials.

| STARTING MATERIAL | INTERMEDIATE |
|---|---|
| 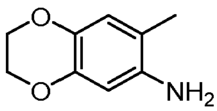 [59820-84-7] | 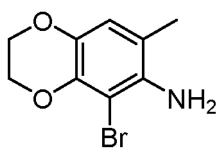 **I-106** |

(continued)

| STARTING MATERIAL | INTERMEDIATE |
|---|---|
| 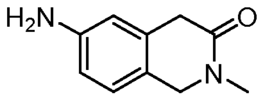 I-104 | 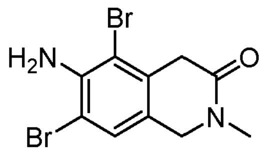 I-107 |

PREPARATION OF INTERMEDIATE **I-108**

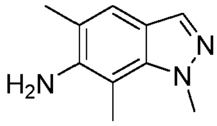

**I-108**

**I-105** (362 mg, 1.51 mmol) and methylboronic acid [13061-96-6] (230 mg, 3.77 mmol) were added to a stirred solution of $Na_2CO_3$ (479 mg, 4.52 mmo) in 1,4-dioxane (4 mL) and $H_2O$ (1 mL). Pd(dppf)$Cl_2$•DCM (61.6 mg, 75.4 μmol) was added and the reaction mixture was stirred at 105 °C for 16 h. The reaction mixture was diluted with water and EtOAc. The organic layer was separated, dried ($MgSO_4$), filtered and concentrated under reduced pressure. The crude mixture was purified by flash column chromatography (silica, heptane/EtOAc, gradient from 100:0 to 80:20) to afford **I-108** (162 mg, 61%) as a light yellow solid.

The following intermediates were prepared in an analogous manner to that described for intermediate **I-108** from the indicated starting materials.

| STARTING MATERIAL | INTERMEDIATE |
|---|---|
| 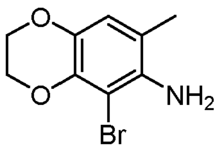 I-106 | 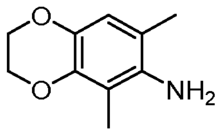 I-109 |
| 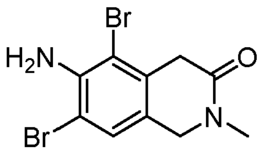 I-107 | 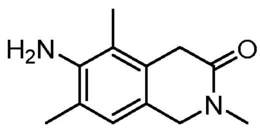 I-110 |

PREPARATION OF INTERMEDIATE 25

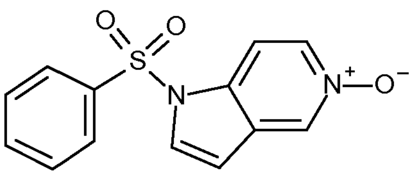

An EasyMax 400 mL reactor was charged with a solution of 1-(phenylsulfonyl)-1H-pyrrolo[3,2-c]pyridine ([109113-39-5], 12 g, 46.458 mmol) in DCM (315 mL). solution was added mCPBA (12.026 g, 69.686 mmol). The reaction mixture was stirred at rt for 60 min, and quenched with aqueous sodium sulfite solution. The aqueous layer was separated and the organic layer was washed with aqueous sodium bicarbonate solution and brine. The organic layer was isolated, dried ($MgSO_4$) and concentrated in vacuo to give a cream solid. This solid was crystallized in ACN to afford 1-25 (8.3 g, 65%). The filtrate was concentrated to afford a second batch of I-25 (3.5 g), which was purified by column chromatography (silica gel; eluent: DCM/7N $NH_3$ in MeOH 100/0 to 95/5) to afford 1.8 g of the compound, which was crystallized in ACN to afford an additional batch of I-25 (1.39 g, 11%).

PREPARATION OF INTERMEDIATE 26

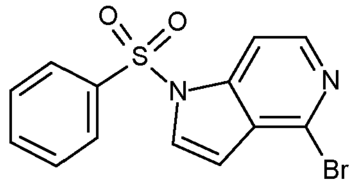

In an EasyMax reactor, 1-25 (9.7 g, 35.363 mmol) was dissolved in dry ACN (138 mL) and dry dioxane (138 mL) under $N_2$ atm. To this solution was added portionwise phosphorus oxybromide (32.394 g, 112.995 mmol).. The resulting mixture was stirred at 70°C overnight. The reaction mixture was concentrated, and DCM and water were added to the mixture. The organic layer was washed with brine, dried ($MgSO_4$), filtered and concentrated in vacuo to afford a crude (36 g) that was purified by column chromatography (silica gel; eluent: heptane/EtOAc 100/0 to 0/100) to afford 1-26 (8.6 g, 72%) as a white solid.

PREPARATION OF INTERMEDIATE 27

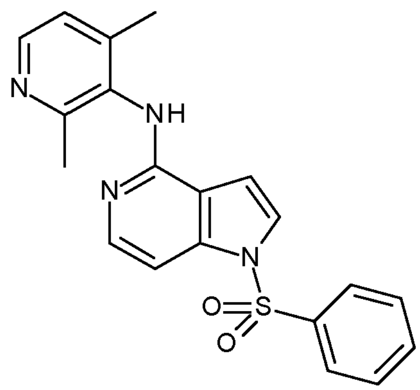

A mixture of I-26 (1 g, 2.966 mmol), 3-amino-2,4-dimethylpyridine (398.5 mg, 3.262 mmol) and $Cs_2CO_3$ (2.126 g, 6.524 mmol) in tBuOH (12 mL) was degassed with nitrogen. $Pd(OAc)_2$ (66.6 mg, 0.297 mmol) and Xantphos (171.6 mg, 0.297 mmol) were added and the mixture was heated 1 h at 120 °C. The solvent was removed in vacuo and the crude was diluted with water and extracted with DCM. The organic layers were dried ($MgSO_4$), filtered and concentrated to afford a crude (1.3 g) that was purified by column chromatography (silica gel; eluent: DCM/7N $NH_3$ in MeOH 100/0 to 95/5). The residue (1 g) was further purified by Prep HPLC (stationary phase: XBridge Prep C18 3.5μm, 4.6×100mm; mobile phase: 0.2% $NH_4HCO_3$ solution in water, MeOH) to afford 1-27 (560 mg, yield 50%).

PREPARATION OF INTERMEDIATE 28

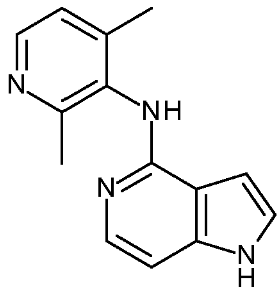

1-27 (1.2 g, 3.171 mmol) and NaOH (1M in $H_2O$, 9.5 mL, 9.512 mmol) were stirred in MeOH (19 mL). The reaction mixture was stirred at 40 °C. After 1 h, the reaction was quenched with addition of some drops of 1M HCl. The reaction mixture was evaporated in vacuo and water was added to the residue. The suspended solid was collected, EtOAc was added and the aqueous layer was extracted with EtOAc (3x). The organic layers were dried ($MgSO_4$), filtered and concentrated to afford a crude (1.2 g) that was purified by column chromatography (silica gel; eluent: DCM/7N $NH_3$ in MeOH 100/0 to 95/5) to afford 1-28 (245 mg, 32).

PREPARATION OF INTERMEDIATE 29

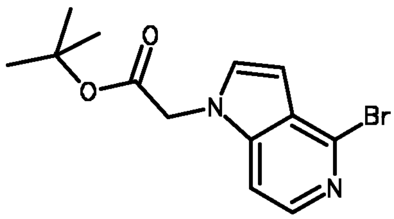

Sodium hydride (0.071 g, 1.78 mmol) was added to a stirred solution of 4-bromo-1H-pyrrolo[3,2-c]pyridine ([1000342-68-6], 350 mg, 1.776 mmol) in DMF (11 mL) over 1 min. The mixture was stirred at rt for 1 h under nitrogen. tert-Butyl bromoacetate (0.262 mL, 1.776 mmol) was added dropwise and the mixture was stirred at rt for 12 h. The mixture was diluted in water and EtOAc. The organic layer was washed with water (×2) and brine, then separated, dried ($MgSO_4$), filtered and the solvents were evaporated in vacuo. The crude product was purified by flash column chromatography (silica; EtOAc in heptane 0/100 to 30/70). The desired fractions were collected and concentrated in vacuo to yield 1-29 (415 mg, 75%) as a white solid.

The following intermediate was prepared in an analogous manner to that described for 1-29 from the indicated starting material and reagent.

| STARTING MATERIAL | REAGENT | INTERMEDIATE |
|---|---|---|
| 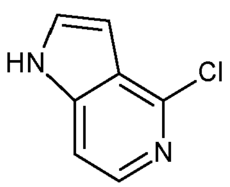 [60290-21-3] | 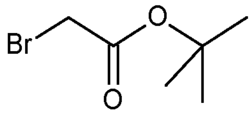 [5292-43-3] | 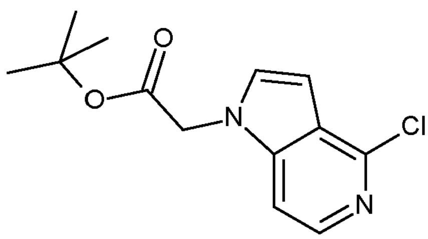 I-30 |

(continued)

| STARTING MATERIAL | REAGENT | INTERMEDIATE |
|---|---|---|
| 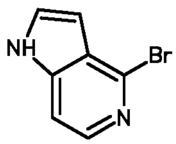 [1000342-68-6] (250 mg, 1.269 mmol) | 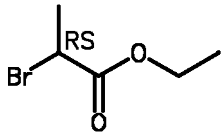 [535-11-5] | 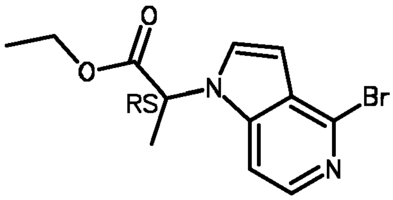 I-31 (305 mg, 81%) |
| 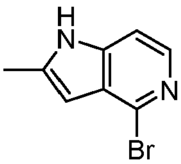 [1190313-58-6] | 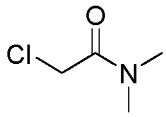 [2675-89-0] | 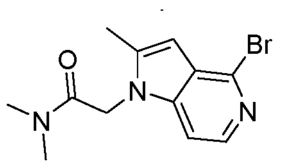 **I-111** |
| 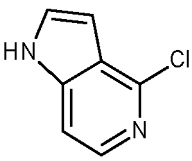 [60290-21-3] | 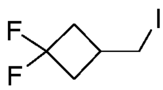 [1434142-22-9] | 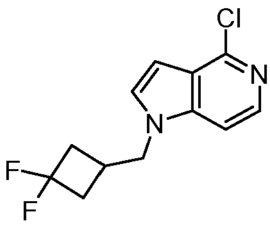 **I-112** |
| 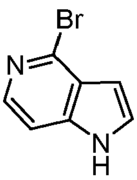 [1000342-68-6] | 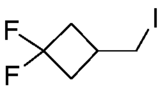 [1434142-22-9] | 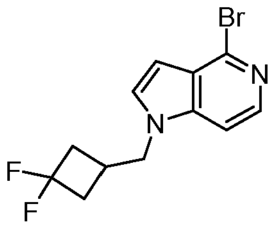 **I-121** |
| 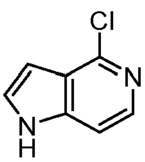 [60290-21-3] | 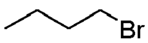 [109-65-9] | 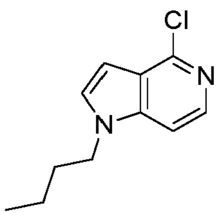 **I-113** |

PREPARATION OF INTERMEDIATES 32A AND 32B

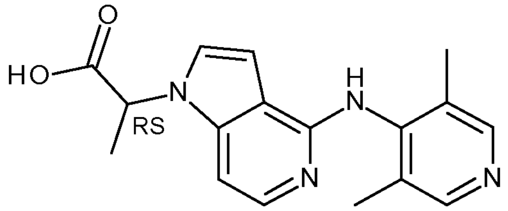

I-32a

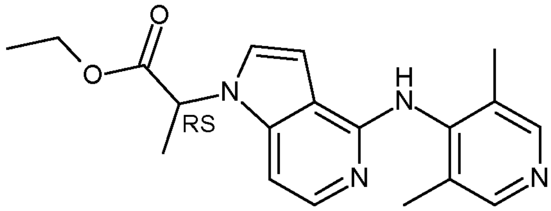

I-32b

$Pd_2dba_3$ (25.64 mg, 0.028 mmol), XantPhos (40.50 mg, 0.07 mmol) and $Cs_2CO_3$ (456.15 mg, 1.4 mmol) were added to a stirred solution of 3,5-dimethylpyridine-4-amine (111.17 mg, 0.91 mmol) and 1-31 (208.00 mg, 0.7 mmol) in DMF (7.5 mL). The reaction mixture was stirred at 105 °C for 24 h, then it was cooled to rt, filtered through diatomaceous earth and washed with EtOAc. The filtrate was partitioned between $NaHCO_3$ and EtOAc. The aqueous phase was extracted with EtOAc twice. The combined organic phases were washed with brine, dried ($MgSO_4$), filtered and the solvents were evaporated in vacuo. The crude product was purified by reverse phase from 95% [25mM $NH_4HCO_3$] - 5% [MeCN: MeOH 1:1] to 0% [25mM $NH_4HCO_3$]-100% [MeCN: MeOH 1:1]. The desired fractions were collected and concentrated in vacuo to yield I-32a (41 mg, 19%) and I-32b (17 mg, 7%) as a light yellow solids.

The following intermediate was prepared in an analogous manner to that described for I-32a/b from the indicated starting material and reagent.

| STARTING MATERIAL | REAGENT | INTERMEDIATE |
|---|---|---|
| I-29 | 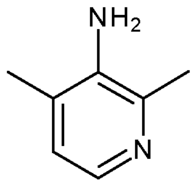 [1073-21-8] | 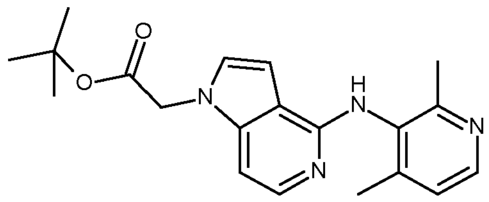 I-33 |
| I-6 | 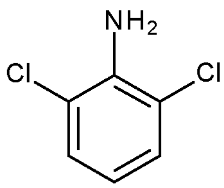 [608-31-1] | 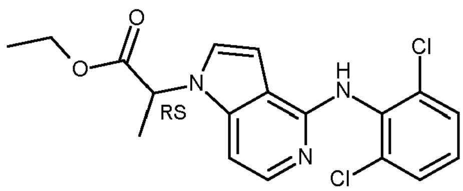 I-34 |
| I-31 | 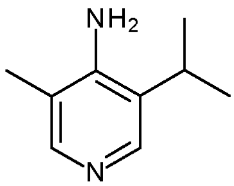 VILL acastello_77_1 | 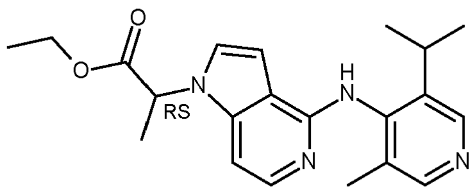 I-35 |
| I-31 | 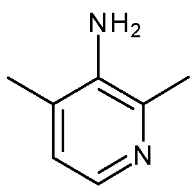 [1073-21-8] | 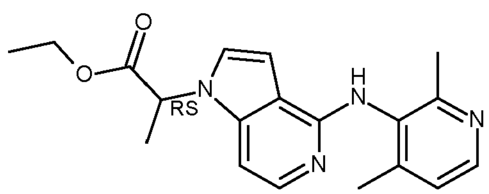 I-36 |

(continued)

| STARTING MATERIAL | REAGENT | INTERMEDIATE |
|---|---|---|
| I-29 | 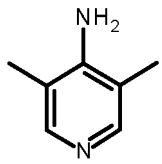 [43078-60-0] | 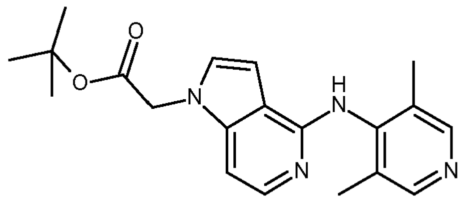 I-37 |
| I-30 | 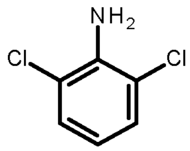 [608-31-1] | 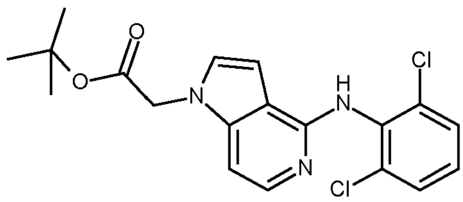 I-38 |

INTERMEDIATE 39

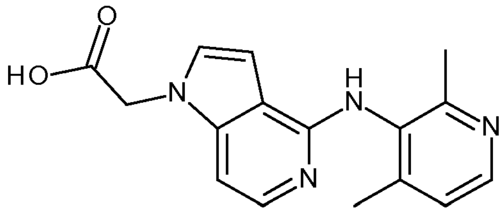

TFA (0.408 mL, 5.334 mmol) was added to a stirred solution of I-33 (47 mg, 0.133 mmol) in DCM (1.533 mL). The mixture was stirred at rt for 16 h. The mixture was concentrated in vacuo and dried under high vacuo to yield 1-39 (38.52 mg) as a brown oil that was used in the subsequent step without further purification.

The following intermediate was prepared in an analogous manner to that described for 1-39 from the indicated starting material.

| STARTING MATERIAL | INTERMEDIATE |
|---|---|
| 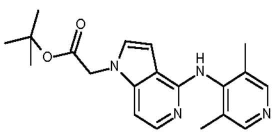 I-37 | 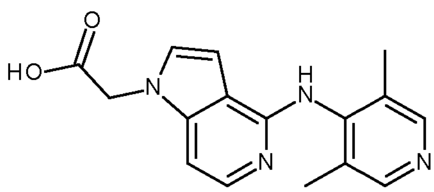 I-40 |
| 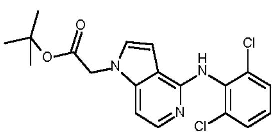 I-38 | 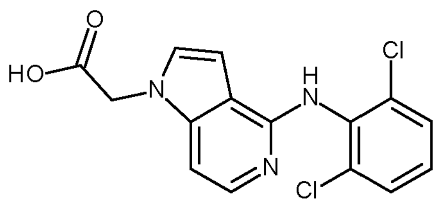 I-41 |

(continued)

| STARTING MATERIAL | INTERMEDIATE |
|---|---|
| 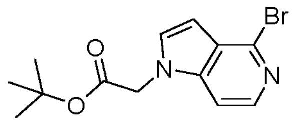 **I-29** | 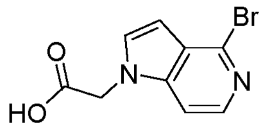 **I-114** |

PREPARATION OF INTERMEDIATE 42

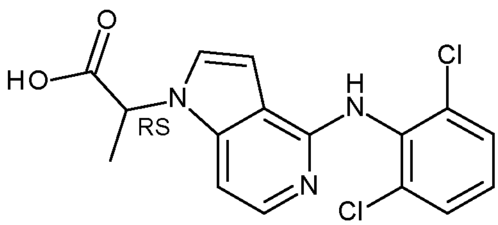

LiOH.$H_2O$ (23.43 mg, 0.558 mmol) was added to a solution of I-34 (200 mg, 0.465 mmol) in THF (3.80 mL) and $H_2O$ (0.95 mL). The reaction mixture was stirred for 1 h at rt. The mixture was concentrated in vacuo and dried under high vacuo to yield 1-42 (159.2 mg, 98%) as a white solid which was used in the subsequent reaction step without further purification.

The following intermediate was prepared in an analogous manner to that described for I-42 from the indicated starting material.

| STARTING MATERIAL | INTERMEDIATE |
|---|---|
| 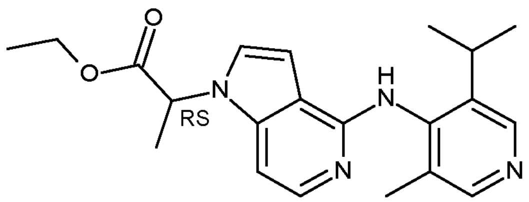 I-35 | 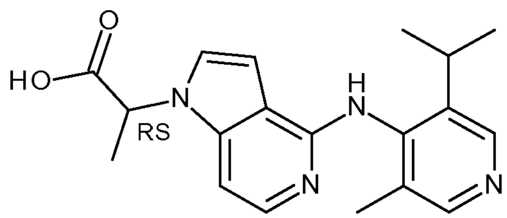 I-43 |
| 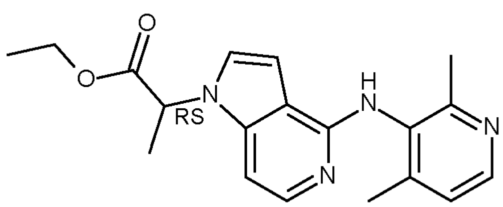 I-36 | 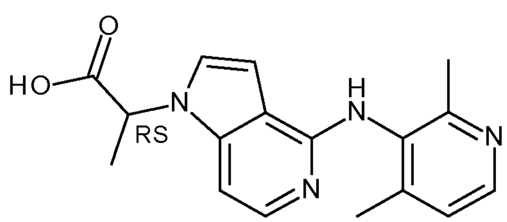 I-44 |

PREPARATION OF INTERMEDIATE **I-115**

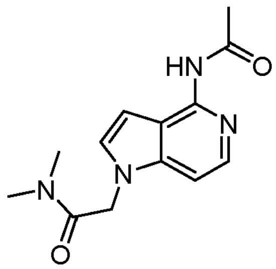

**I-115**

Acetamide (120 mg, 2.03 mmol) and intermediate **I-1** (438 mg, 1.84 mmol) were added to a stirred mixture of $Pd(OAC)_2$ (16.5 mg, 73.7 μmol), XantPhos (95.9 mg, 0.17 mmol) and $Cs_2CO_3$ (1.20 g, 3.69 mmol) in anhydrous 1,4-dioxane (10 mL) under $N_2$ atmosphere. The reaction mixture was stirred at 90 °C for 18 h. The residue was dissolved in EtOAc and water. The organic layer was washed with water, dried ($MgSO_4$), filtered and concentrated under reduced pressure. The crude mixture was purified by flash column chromatography (silica, heptane/EtOAc, gradient from 100:0 to 0:100) to afford **I-115** (166 mg, 35%) as a yellow solid.

The following intermediate was prepared in an analogous manner to that described for **I-115** from the indicated starting material.

| STARTING MATERIAL | INTERMEDIATE |
|---|---|
| 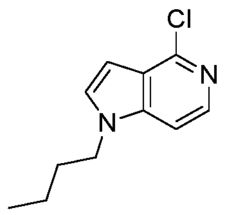 **I-113** | 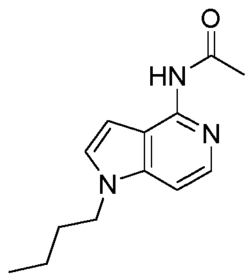 **I-116** |

PREPARATION OF INTERMEDIATE **I-117**

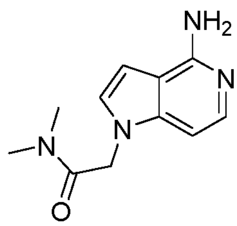

**I-117**

HCl (1.25M in MeOH, 2.55 mL) was added to a solution of **I-115** (166 mg, 0.64 mmol) in MeOH (2 mL) and the reaction mixture was stirred for 72 h at 80 °C. The solvents were evaporated under reduced pressure. $NaHCO_3$ was added and the aqueous phase was extracted with EtOAc. The combined organic layers were dried ($MgSO_4$), filtered and evaporated under reduced pressure to afford **I-117**.

The following intermediate was prepared in an analogous manner to that described for **I-117** from the indicated starting material.

| STARTING MATERIAL | INTERMEDIATE |
| --- | --- |
| 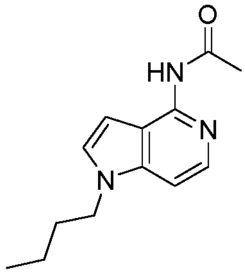 I-116 | 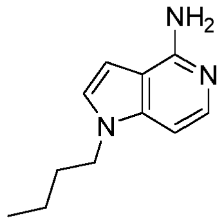 I-118 |

PREPARATION OF INTERMEDIATE **I-119**

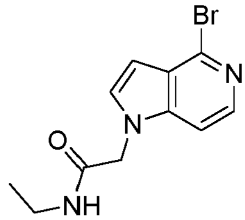

**I-119**

A mixture of **I-114** as a TFA salt (2.00 g, 5.42 mmol), ethylamine hydrochloride (442 mg, 5.42 mmol), HBTU (2.06 g, 5.42 mmol) and DIPEA (5.60 mL, 32.5 mmol) in DCM (80 mL) was stirred at room temperature for 2 h. NaOH (IN, aq., 5 mL) was added and the mixture was stirred for 5 min. The organic layer was separated, dried ($MgSO_4$), filtered and evaporated under reduced pressure. The crude mixture was purified by flash column chromatography (silica, DCM/MeOH, gradient from 100:0 to 98:2) to afford **I-119** (0.74 g, 48%).

The following intermediate was prepared in an analogous manner to that described for **I-119** from the indicated starting material and reagent.

| STARTING MATERIAL | REAGENT | INTERMEDIATE |
| --- | --- | --- |
| 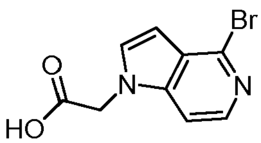 I-114 | $MeNH_2$•HCl | 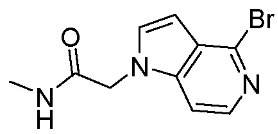 I-120 |

PREPARATION OF FINAL COMPOUNDS

PREPARATION OF CO. NO. 1

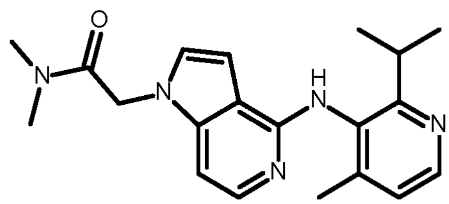

A mixture of I-1 (2.2 g, 9.26 mmol), 4-methyl-2-(1-methylethyl)-3-pyridinamine ([1698293-93-4], 1.53 g, 10.18 mmol) and $Cs_2CO_3$ (6.64 g, 20.37 mmol) in tBuOH (40 mL, 426.32 mmol) was degassed with nitrogen. $Pd(OAc)_2$ (207.87 mg, 0.93 mmol) and Xantphos (535.75 mg, 0.93 mmol) were added and the reaction mixture was heated at 130°C for 90 min. The solvent was evaporated and the residue was taken in water/DCM + 5 g $NaHCO_3$, then filtered. The organic layer was separated and the aqueous phase was extracted twice with DCM. The combined organic layers were dried over $MgSO_4$ and evaporated, then the crude was purified by column chromatography (normal phase silica; eluent: ($MeOH/NH_3$ in MeOH, 7N) in DCM, from 0/100 to 95/5.

The pure fractions were recrystallized from hot ACN, yielding Co. No. 1 (1098 mg, yield 33.74%) as a white solid.

The following compounds were prepared in an analogous manner to the synthesis of Co. No. 1 by reaction of the corresponding intermediates and the indicated reagents.

| INTERMEDIATE | ANILINE | COMPOUND |
|---|---|---|
| I-2 | 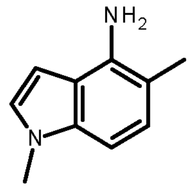 I-8 | 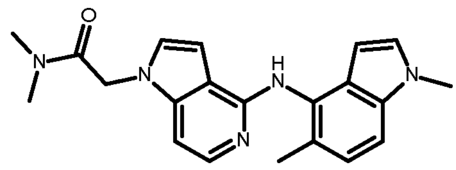 Co. No. 2 |
| I-2 | 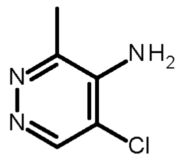 I-9 | 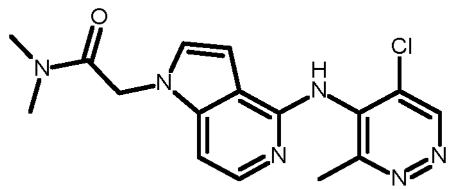 Co. No. 3 |
| I-2 | 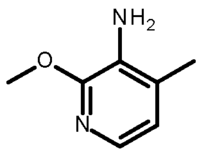 [76005-99-7] | 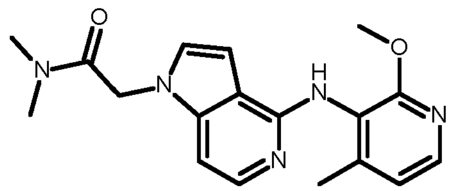 Co. No. 4 |
| I-1 | 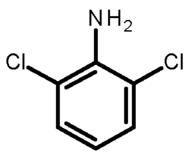 [608-31-1] | 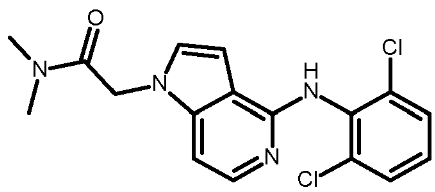 Co. No. 5 |
| I-1 | 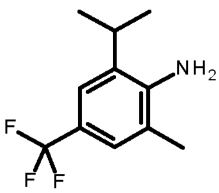 I-12 | 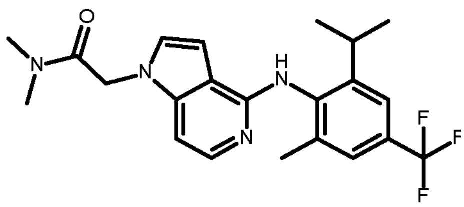 Co. No. 6 |

(continued)

| INTERMEDIATE | ANILINE | COMPOUND |
|---|---|---|
| I-1 | 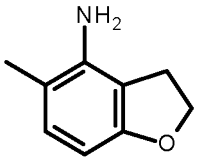 [1369347-10-3] | 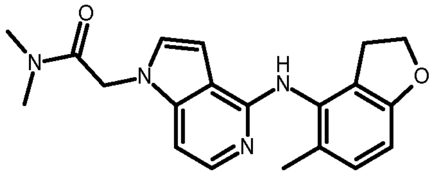 Co. No. 7 |
| I-2 | 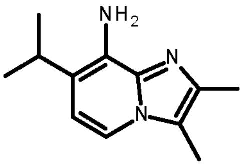 I-20 | 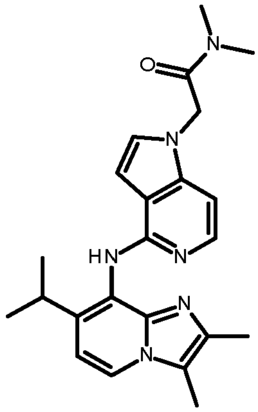 Co. No. 8 |
| I-2 | 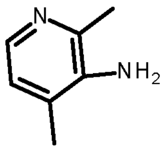 [1073-21-8] | 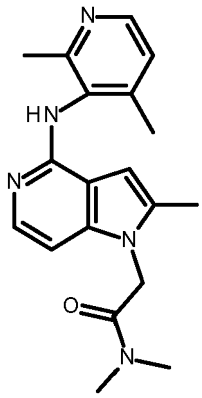 Co. No. 9 |
| I-4 | 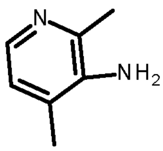 [1073-21-8] | 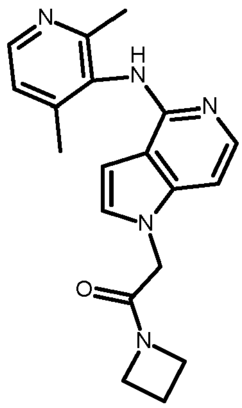 Co. No. 10 |

(continued)

| INTERMEDIATE | ANILINE | COMPOUND |
|---|---|---|
| I-4 | 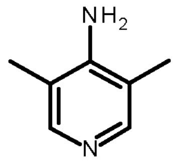 [43078-60-0] | 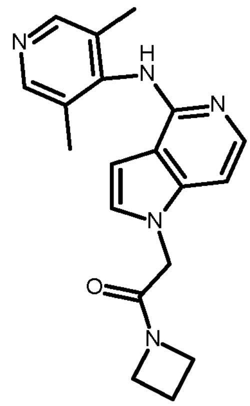 Co. No. 11 |
| I-4 | 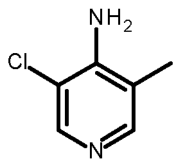 [97944-42-8] | 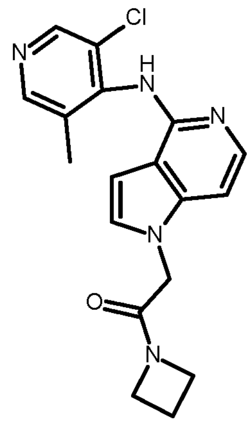 Co. No. 12 |
| I-3 | 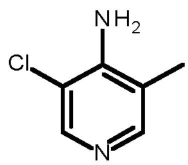 [97944-42-8] | 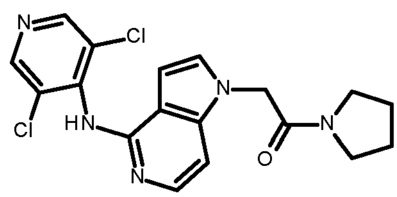 Co. No. 13 |
| I-1 | 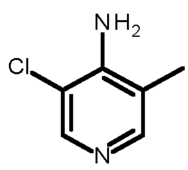 [97944-42-8] | 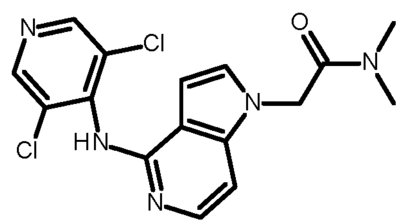 Co. No. 14 |

(continued)

| INTERMEDIATE | ANILINE | COMPOUND |
|---|---|---|
| I-1 | 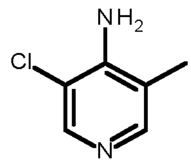 [97944-42-8] | 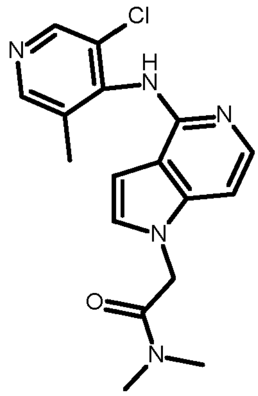 Co. No. 15 |
| I-1 | 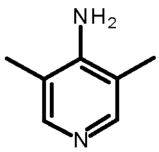 [43078-60-0] | 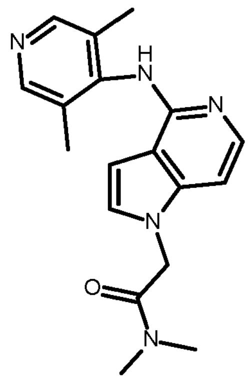 Co. No. 16 |
| I-1 | 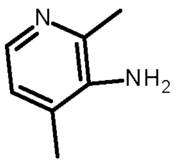 [1073-21-8] | 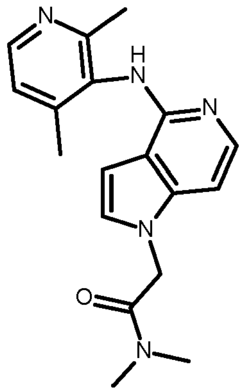 Co. No. 17 |

(continued)

| INTERMEDIATE | ANILINE | COMPOUND |
|---|---|---|
| I-1 | 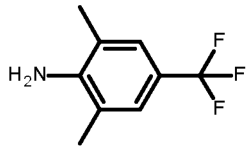 [144991-53-7] | 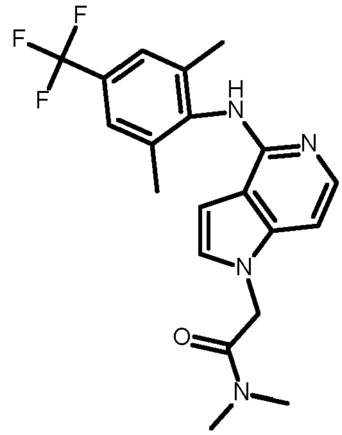 Co. No. 18 |
| I-1 | 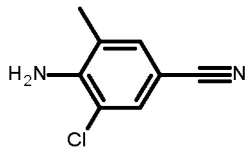 [158296-69-6] | 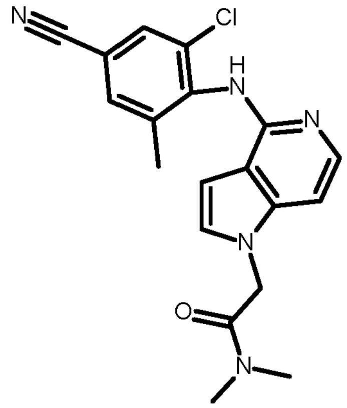 Co. No. 19 |
| I-2 | 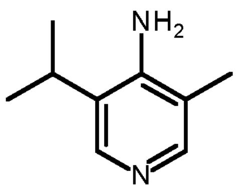 I-21 | 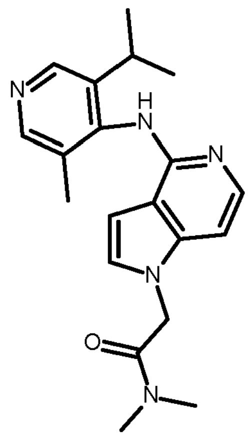 Co. No. 20 |

(continued)

| INTERMEDIATE | ANILINE | COMPOUND |
|---|---|---|
| I-1 | 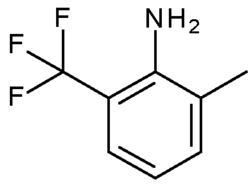 [88301-98-8] | 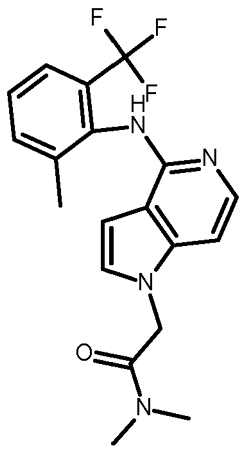 Co. No. 21 |
| I-1 | 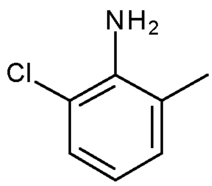 [87-63-8] | 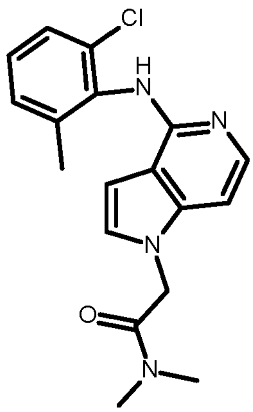 Co. No. 22 |
| I-1 | 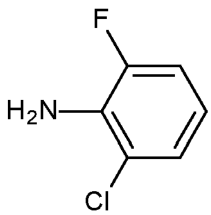 [363-51-9] | 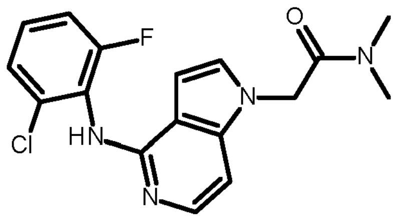 Co. No. 23 |
| I-1 | 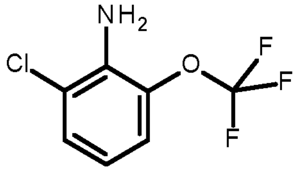 [1261673-70-4] | 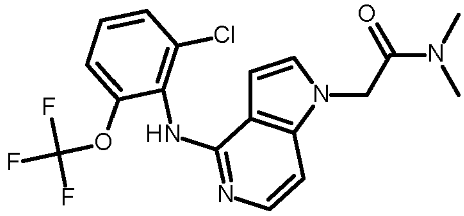 Co. No. 24 |

(continued)

| INTERMEDIATE | ANILINE | COMPOUND |
|---|---|---|
| I-1 | 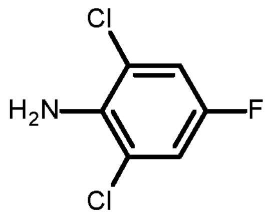 [344-19-4] | 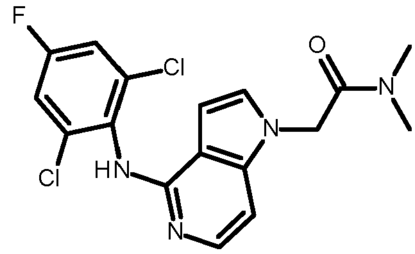 Co. No. 25 |
| I-1 | 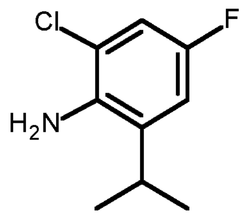 I-22 | 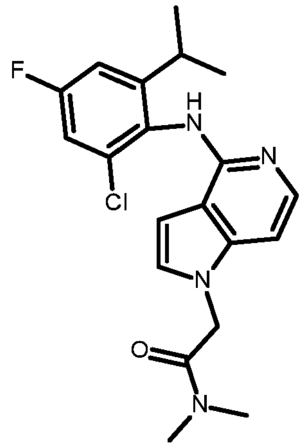 Co. No. 26 |
| I-1 | 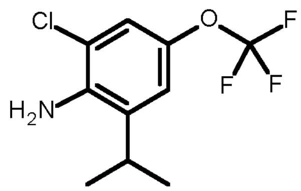 I-23 | 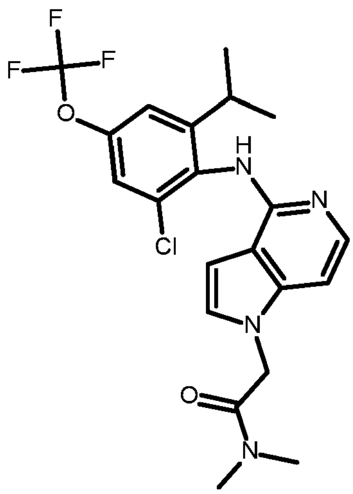 Co. No. 27 |

(continued)

| INTERMEDIATE | ANILINE | COMPOUND |
|---|---|---|
| I-1 | [36556-56-6] | 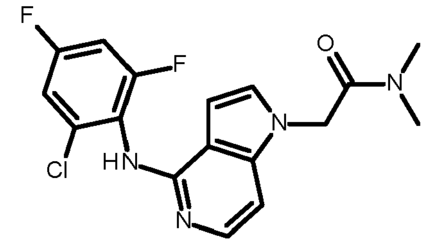 Co. No. 28 |
| I-1 | 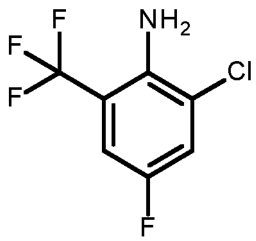 [1235439-54-9] | 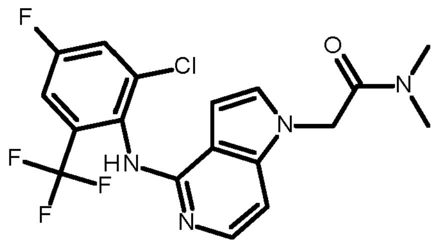 Co. No. 29 |
| **I-2** | 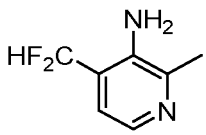 [1804438-91-2] | 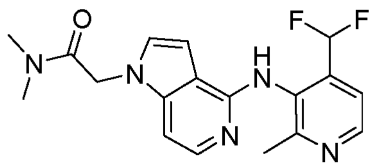 **Co. No. 38** |
| **I-1** | 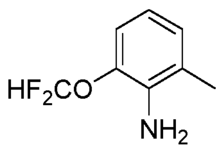 [139909-66-3] | 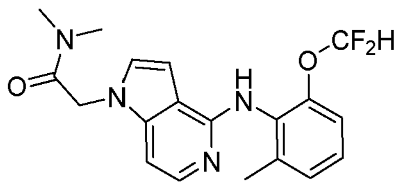 **Co. No. 39** |
| **I-1** | 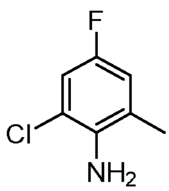 [332903-47-6] | 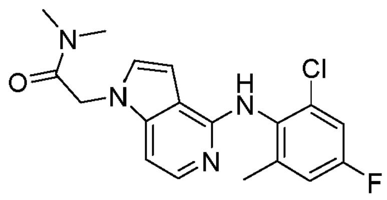 **Co. No. 40** |
| **I-1** | 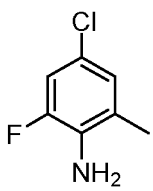 [939989-99-8] | 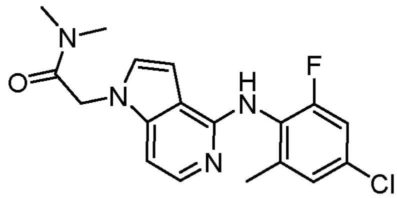 **Co. No. 41** |

(continued)

| INTERMEDIATE | ANILINE | COMPOUND |
|---|---|---|
| **I-1** | 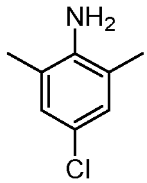 [24596-18-7] | 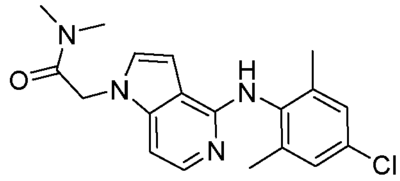 **Co. No. 42** |
| **I-1** | 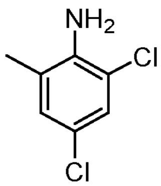 [30273-00-8] | 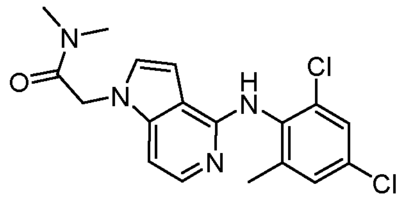 **Co. No. 43** |
| **I-2** | 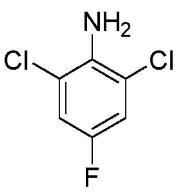 [344-19-4] | 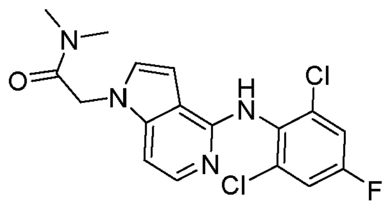 **Co. No. 44** |
| **I-111** | 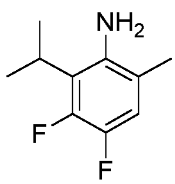 **I-63** | 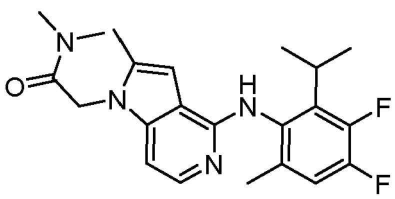 **Co. No. 45** |
| **I-1** | 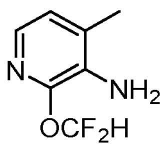 **I-89** | 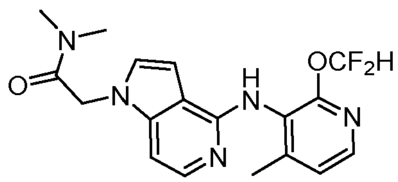 **Co. No. 46** |
| **I-45** | 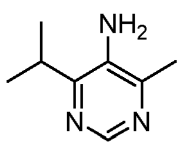 **I-78** | 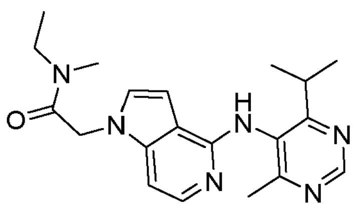 **Co. No. 47** |

(continued)

| INTERMEDIATE | ANILINE | COMPOUND |
|---|---|---|
| I-1 | 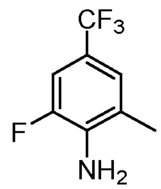 I-72 | 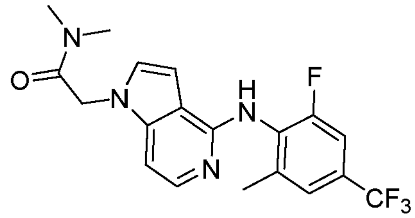 Co. No. 48 |
| I-1 | 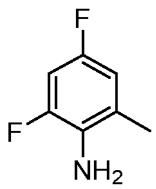 [1464825-76-0] | 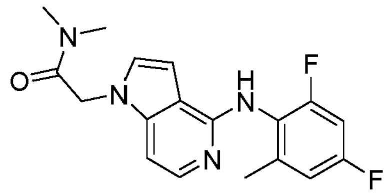 Co. No. 49 |
| I-1 | 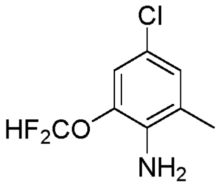 I-53 | 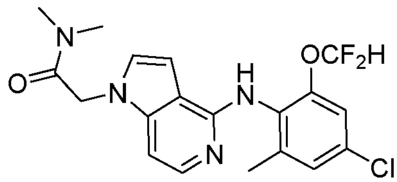 Co. No. 50 |
| I-1 | 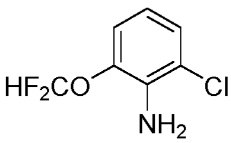 [139909-66-3] | 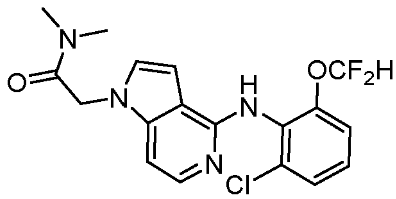 Co. No. 51 |
| I-1 | 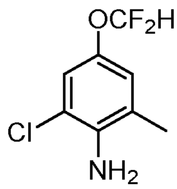 I-54 | 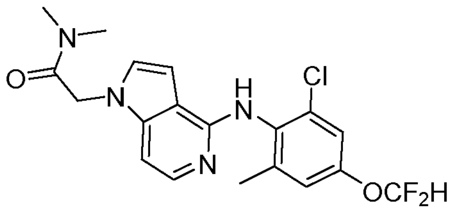 Co. No. 52 |
| I-1 | 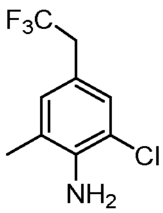 I-95 | 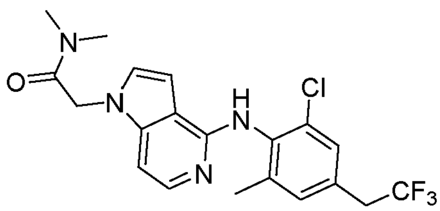 Co. No. 53 |

(continued)

| INTERMEDIATE | ANILINE | COMPOUND |
|---|---|---|
| **I-1** | 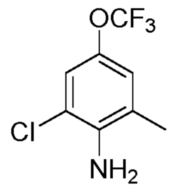 **I-73** | 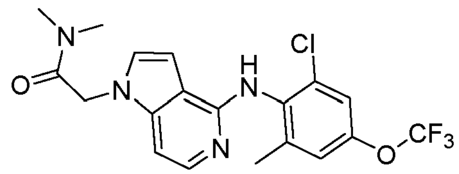 **Co. No. 54** |
| **I-45** | 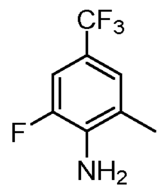 **I-72** | 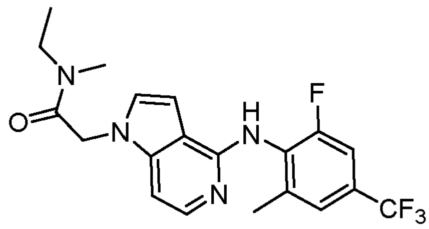 **Co. No. 55** |
| **I-45** | 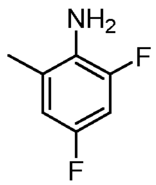 [1464825-76-0] | 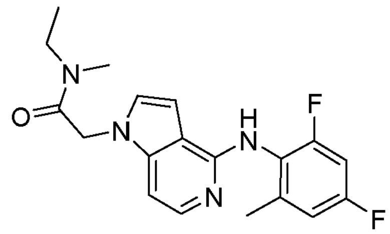 **Co. No. 56** |
| **I-45** | 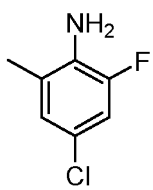 [939989-99-8] | 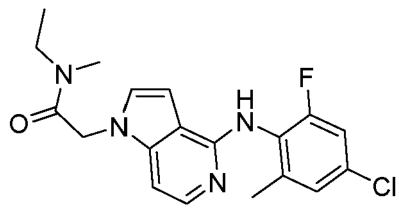 **Co. No. 57** |
| **I-2** | 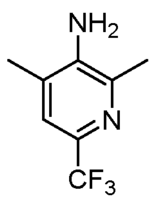 **I-74** | 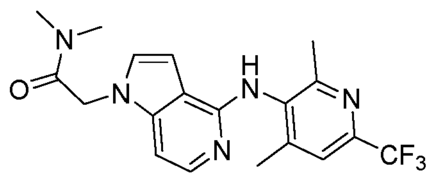 **Co. No. 58** |
| 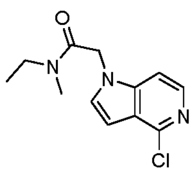 | 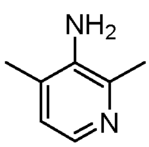 [1073-21-8] | 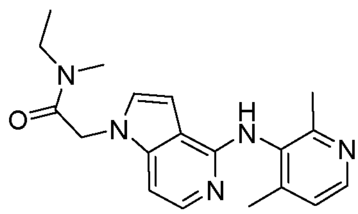 **Co. No. 59** |

(continued)

| INTERMEDIATE | ANILINE | COMPOUND |
|---|---|---|
| **I-45** | 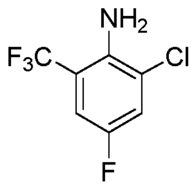 [1235439-54-9] | 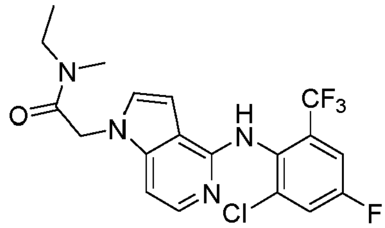 **Co. No. 60** |
| **I-45** | 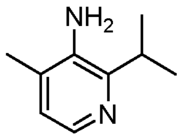 [1698293-93-4] | 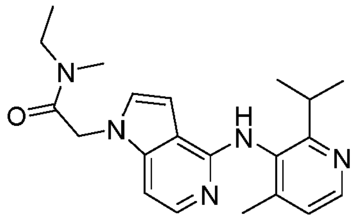 **Co. No. 61** |
| **I-45** | 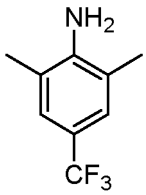 [144991-53-7] | 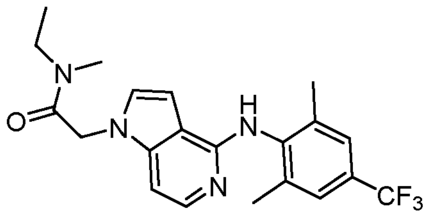 **Co. No. 62** |
| **I-45** | 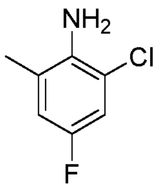 [332903-47-6] | 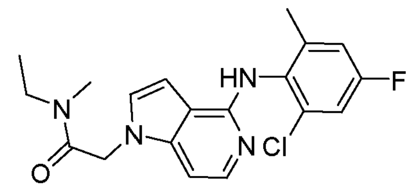 **Co. No. 63** |
| **I-2** | 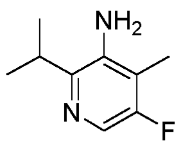 **I-99** | 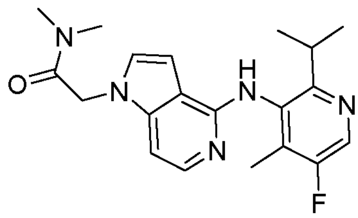 **Co. No. 64** |
| **I-45** | 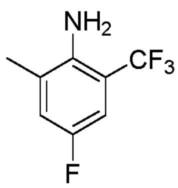 **I-75** | 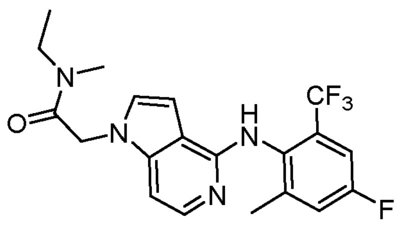 **Co. No. 65** |

(continued)

| INTERMEDIATE | ANILINE | COMPOUND |
|---|---|---|
| **I-2** | 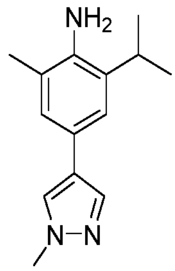 **I-81** | 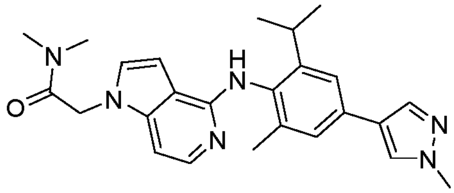 **Co. No. 66** |
| **I-45** | 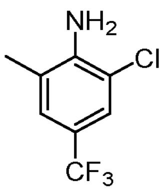 **I-77** | 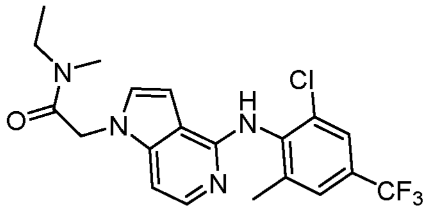 **Co. No. 67** |
| **I-2** | 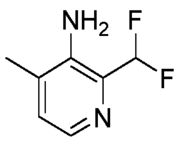 | 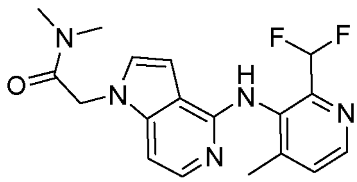 **Co. No. 68** |
| **I-1** | 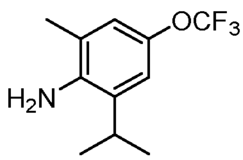 **I-66** | 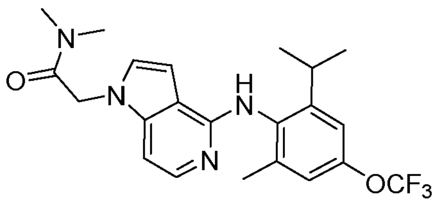 **Co. No. 69** |
| **I-2** | 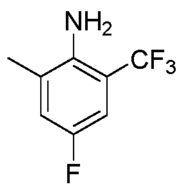 **I-75** | 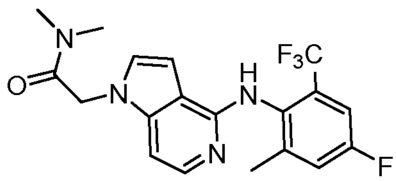 **Co. No. 70** |
| **I-2** | 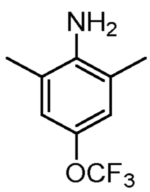 **I-76** | 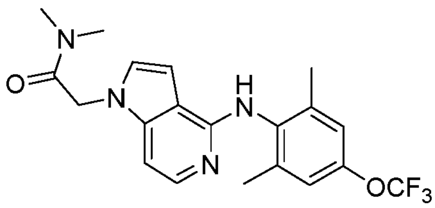 **Co. No. 71** |

(continued)

| INTERMEDIATE | ANILINE | COMPOUND |
|---|---|---|
| **I-2** | 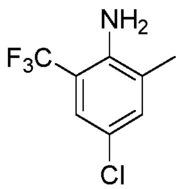 [1240528-24-8] | 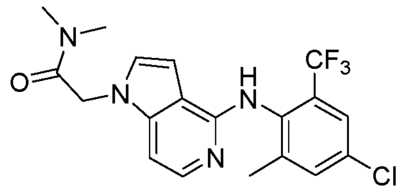 **Co. No. 72** |
| **I-2** | 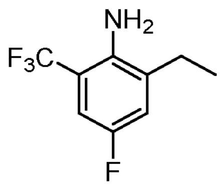 **I-82** | 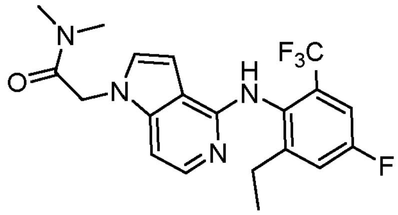 **Co. No. 73** |
| **I-2** | 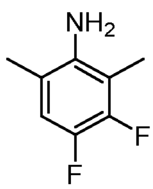 **I-83** | 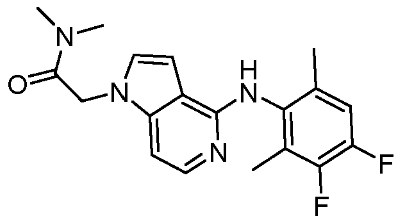 **Co. No. 74** |
| **I-1** | 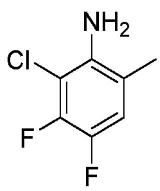 **I-55** | 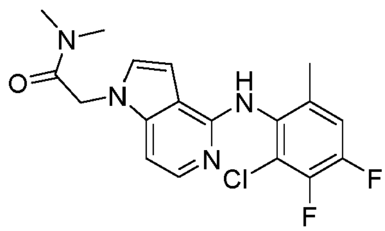 **Co. No. 75** |
| **I-1** | 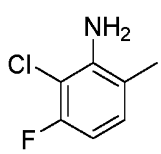 **I-93** | 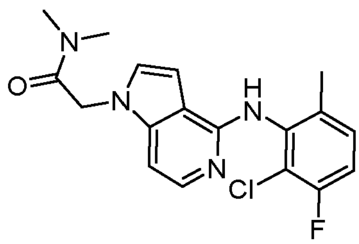 **Co. No. 76** |
| **I-1** | 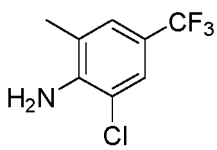 **I-56** | 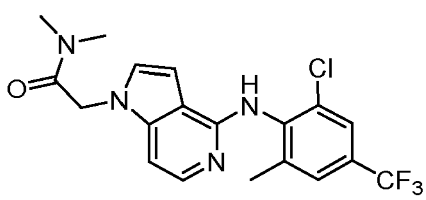 **Co. No. 77** |

(continued)

| INTERMEDIATE | ANILINE | COMPOUND |
|---|---|---|
| **I-119** | 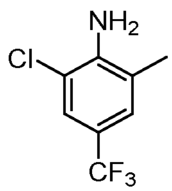 **I-56** | 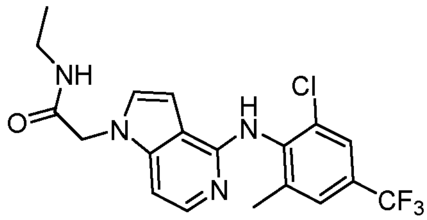 **Co. No. 78** |
| **I-120** | 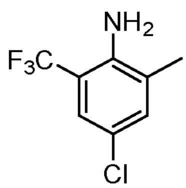 [1240528-24-8] | 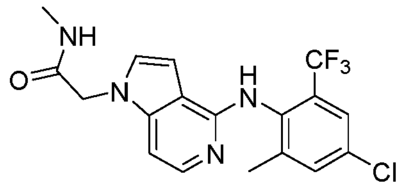 **Co. No. 79** |
| **I-45** | 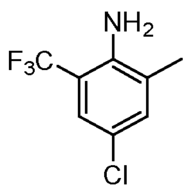 [1240528-24-8] | 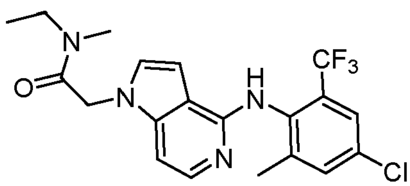 **Co. No. 80** |
| **I-120** | 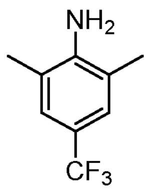 [14491-53-7] | 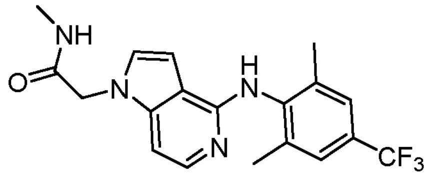 **Co. No. 81** |
| **I-120** | 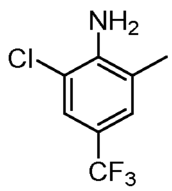 **I-56** | 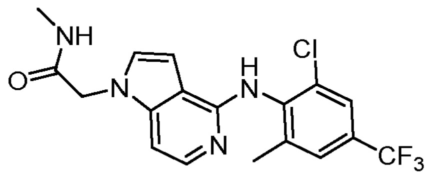 **Co. No. 82** |
| **I-2** | 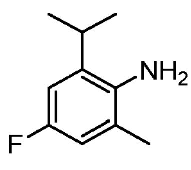 **I-64** | 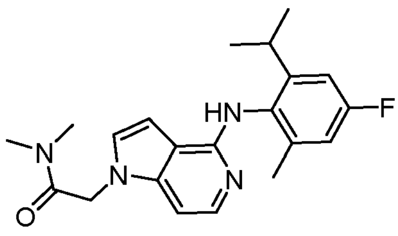 **Co. No. 83** |

(continued)

| INTERMEDIATE | ANILINE | COMPOUND |
|---|---|---|
| **I-1** | 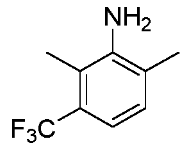 **I-101** | 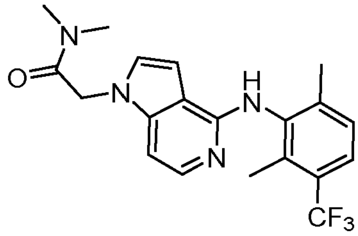 **Co. No. 84** |

**PREPARATION OF COMPOUND NO. CO. 85**

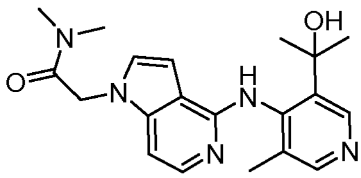

**Co. No. 85**

A mixture of **I-2** (200 mg, 0.71 mmol), **I-69** (100 mg, 0.60 mmol) and $Cs_2CO_3$ (396 mg, 1.21 mmol) in anhydrous DMF (1.43 mL) was degassed with $N_2$. Xantphos (46.6 mg, 80.6 μmol) and $Pd(OAc)_2$ (36.6 mg, 0.163 mmol) were added and the reaction mixture was stirred at 120 °C for 4 h. The reaction mixture was cooled down and diluted with DCM and $H_2O$. The aqueous phase was extracted with DCM, dried ($MgSO_4$), filtered and co evaporated with MIK. The crude mixture was purified via Prep HPLC (stationary phase: RP Vydac Denali C18 - 10μm, 200g, 5cm I.D., mobile phase: 0.25% $NH_4HCO_3$ solution in water, MeCN) to afford compound **85** (37 mg, 17%).

The following compounds were prepared in an analogous manner to that described for compound **85** from the indicated intermediates and reagents.

| INTERMEDIATE | REAGENT | PRODUCT |
|---|---|---|
| **I-2** | 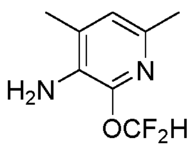 **I-90** | 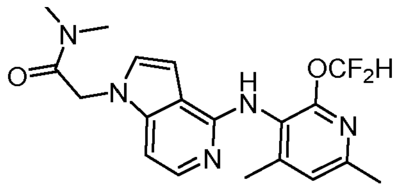 **Co. No. 86** |
| **I-2** | 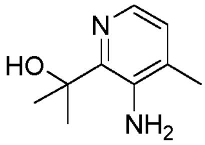 **I-78** | 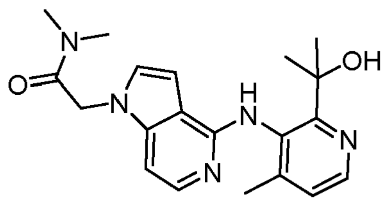 **Co. No. 87** |

(continued)

| INTERMEDIATE | REAGENT | PRODUCT |
|---|---|---|
| **I-2** | 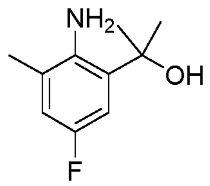 **I-98** | 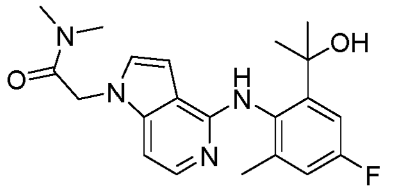 **Co. No. 88** |
| **I-2** | 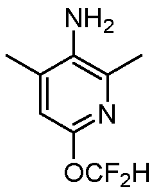 [1224432-57-8] | 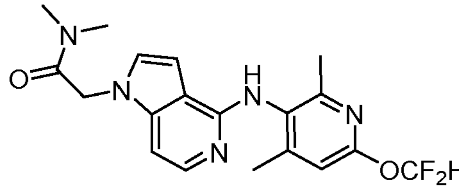 **Co. No. 89** |

PREPARATION OF COMPOUND 30

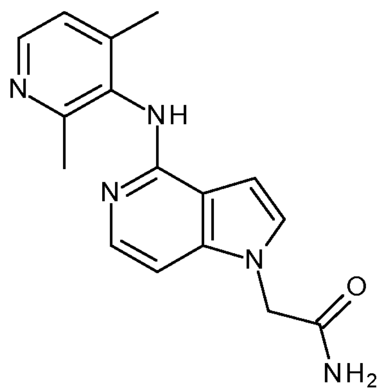

1-28 (50 mg, 0.21 mmol) was dissolved in DMF (2 mL). NaH (60% dispersion in mineral oil, 9.2 mg, 0.231 mmol) was added at 0°C and stirred then at rt. When gas evolution stopped, 2-bromoacetamide (35 mg, 0.252 mmol) was added at 0°C. Then the reaction mixture was stirred at rt for 16 h and quenched with water and EtOAc was added. The aqueous layer was extracted three times with EtOAc. The combined organic layers were washed with brine, dried ($MgSO_4$), filtered and concentrated to afford a crude (65 mg) that was purified by column chromatography (silica gel; eluent: DCM/7NNH$_3$ MeOH 100/0 to 95/5) to afford Co. No. 30 (6.4 mg, 10%).

The following compound was prepared in an analogous manner to that described for compound 30 from the indicated starting material and reagents.

| Intermediate | Reagent | Compound |
|---|---|---|
| 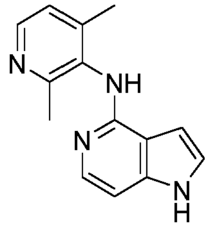 I-28 | 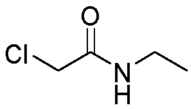 [105-35-1] | 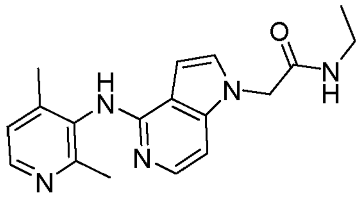 Co. No. 90 |

PREPARATION OF COMPOUND 31

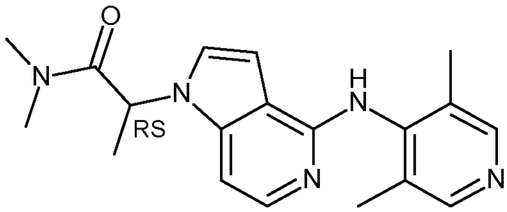

Dimethylamine hydrochloride (12.93 mg, 0.16 mmol) was added to a stirred solution of I-32a (41 mg, 0.13 mmol), HOBt hydrate (21.42 mg, 0.16 mmol), DIPEA (0.069 mL, 0.40 mmol), and EDC.HCl (30.39 mg, 0.16 mmol) in DCM (2.04 mL) and DMF (0.5 mL) at rt and the reaction mixture was stirred at rt for 16 h. Saturated $NaHCO_3$ solution and EtOAc were added, and the aqueous phase was extracted with EtOAc twice. The combined organic layers were washed with brine, dried ($MgSO_4$), filtered and the solvents were evaporated in vacuo. The crude product was purified by flash column chromatography (silica; MeOH in DCM 0/100 to 10/90). The desired fractions were collected and concentrated to yield Co. No. 31 (13 mg, 29%) as a white solid.

The following compounds were prepared in an analogous manner to the synthesis of Co. No. 31 by reaction of the corresponding intermediates and the indicated reagents.

| Starting material | Reagent | Compound |
|---|---|---|
| I-39 | $NH_2Me$ | 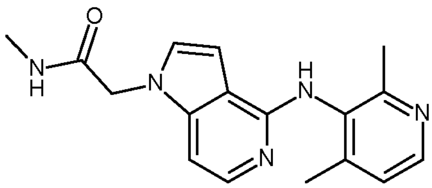 Co. No. 32 |

(continued)

| Starting material | Reagent | Compound |
|---|---|---|
| I-43 | $NHMe_2$ | 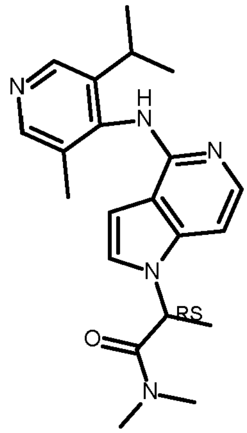 Co. No. 33 |

PREPARATION OF COMPOUND 34

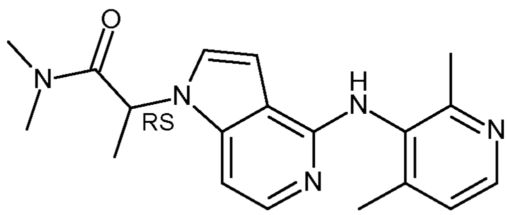

Dimethylamine solution (2M in THF, 0.15 mL, 0.3 mmol) was added to a stirred solution of I-44 (31.04 mg, 0.1 mmol), T3P® ([68957-94-8], 0.149 mL, 0.25 mmol) and $Et_3N$ (0.035 mL, 0.25 mmol) in THF (1 mL) at rt. The reaction mixture was stirred at 50 °C for 3 h. The mixture was diluted with sat $NH_4Cl$ and extracted with EtOAc.

The aqueous layer was extracted with EtOAc (×2). The combined organic layers were dried ($MgSO_4$), filtered and the solvents were evaporated in vacuo. The crude product was purified by flash column chromatography (silica; MeOH in $CH_2Cl_2$ 0/100 to 10/90). The desired fractions were collected and concentrated in vacuo to yield Co. No. 34 (10 mg, 30%) as a white solid.

PREPARATION OF COMPOUND 35

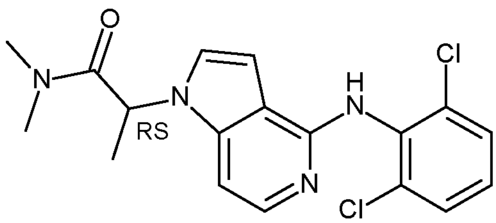

HBTU (88.74 mg, 0.23 mmol) was added to a stirred solution of I-42 (63.04 mg, 0.18 mmol) and DIPEA (0.094 mL, 0.54 mmol) in DMF (5.57 mL) at rt. The reaction mixture was stirred at rt for 30 min. Then, dimethylamine solution (2M in THF, 0.099 mL, 0.198 mmol) was added and the mixture was stirred at rt for 16 h. The solvent was evaporated

in vacuo and the crude product was purified by reverse phase from 72% [25mM $NH_4HCO_3$] - 28% [MeCN: MeOH 1:1] to 36% [25mM $NH_4HCO_3$] - 64% [MeCN: MeOH 1:1]. The desired fractions were collected and concentrated in vacuo and repurified by flash column chromatography (silica; MeOH in $CH_2Cl_2$ 0/20 to 1/20). The desired fractions were collected and concentrated in vacuo to yield Co. No. 35 (13 mg, 19%) as a white solid.

The following compounds were prepared in an analogous manner to the synthesis of Co. No. 35 by reaction of the corresponding intermediates and the indicated reagents.

| INTERMEDIATE | REAGENT | COMPOUND |
|---|---|---|
| I-40 | $MeNH_2$ | 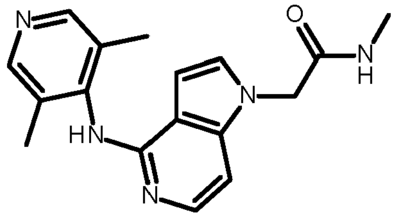 Co. No. 36 |
| I-41 | $MeNH_2$ | 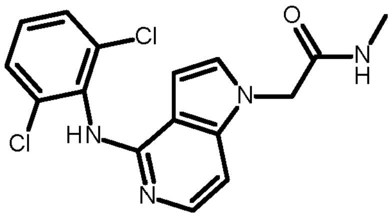 Co. No. 37 |

Compound 32 was also prepared according to this procedure starting from 1-39 and $NH_2Me$ ()

PREPARATION OF COMPOUND **91**

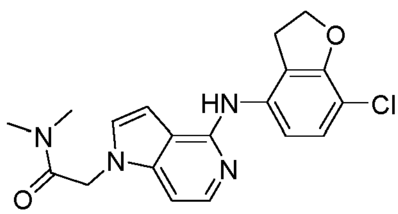

**91**

$Pd_2dba_3$ (8.25 mg, 9.01 μmol), XantPhos (13.0 mg, 22.5 μmol) and $Cs_2CO_3$ (110 mg, 0.34 mmol) were dissolved in DMF (2.25 mL) in a sealed tube while $N_2$ was bubbling. **I-49** (42.0 mg, 0.25 mmol) and **I-1** (53.5 mg. 0.23 mmol) were added and the reaction mixture was stirred at room temperature for 10 min. Then, the reaction mixture was heated at 110 °C for 20 h. The reaction mixture was cooled to room temperature, filtered through Celite® and washed with EtOAc. The solvent was removed under reduced pressure and the crude mixture was purified by reverse phase column chromatography (mobile phase: HCOOH (0.1%)/(MeCN:MeOH, 1:1), gradient from 95:5 to 63:37) to afford compound 91 (42 mg, 50%) as a white solid.

The following compounds were prepared in an analogous manner to that described for Co. No. 91 from the indicated intermediates and reagents.

| INTERMEDIATE | REAGENT | COMPOUND |
|---|---|---|
| I-1 | 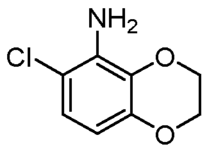 I-50a | 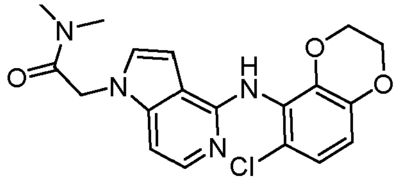 Co. No. 92 |
| I-2 | 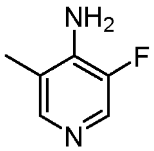 [13958-85-5] | 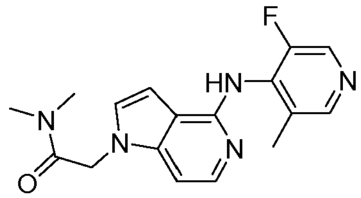 Co. No. 93 |
| I-1 | 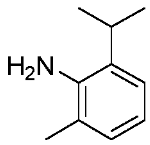 [5266-85-3] | 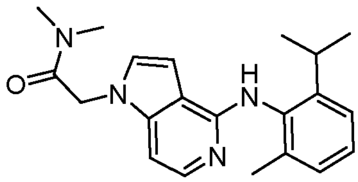 Co. No. 94 |
| I-1 | 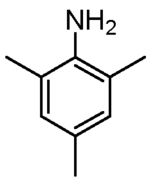 [88-05-1] | 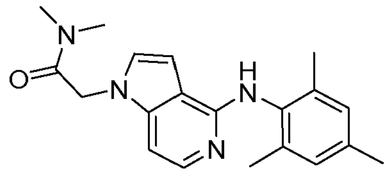 Co. No. 95 |
| I-1 | 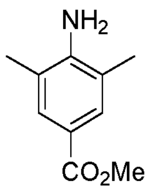 [3095-48-5] | 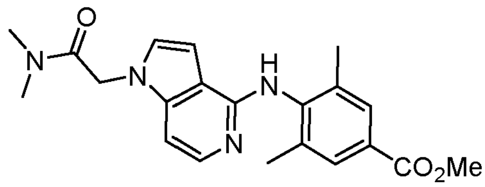 Co. No. 96 |
| I-2 | 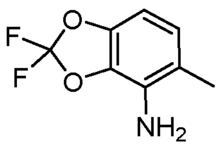 I-79 | 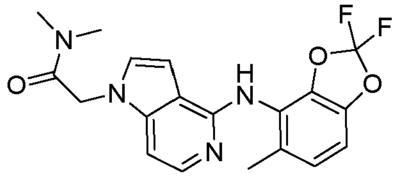 Co. No. 97 |
| I-117 | 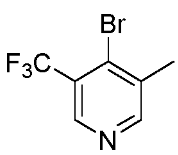 I-60 | 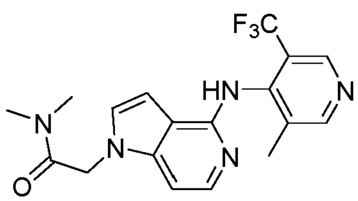 Co. No. 98 |

(continued)

| INTERMEDIATE | REAGENT | COMPOUND |
|---|---|---|
| **I-1** | 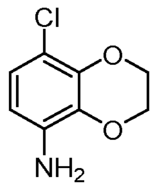 **I-50b** | 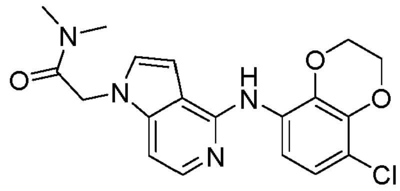 **Co. No. 99** |
| **I-1** | 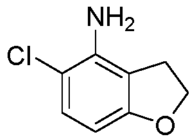 **I-51** | 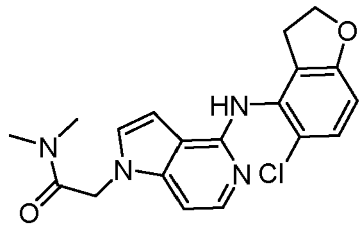 **Co. No. 100** |
| **I-1** | 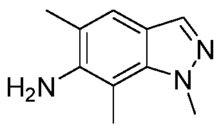 **I-108** | 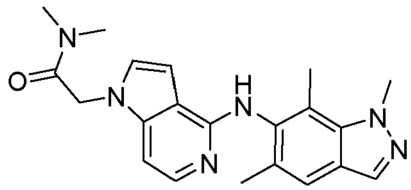 **Co. No. 101** |
| **I-112** | 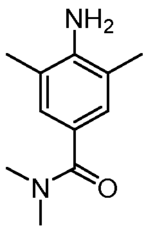 [1234661-58-5] | 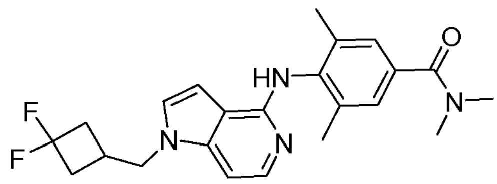 HCl salt **Co. No. 102** |
| **I-1** | 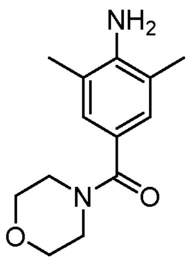 [1458654-04-0] | 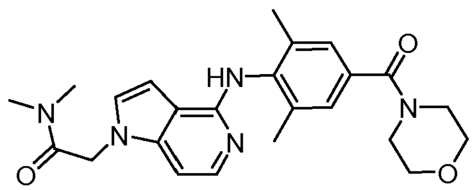 HCl salt **Co. No. 103** |
| **I-1** | 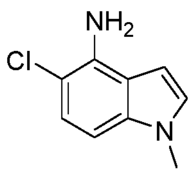 **I-7** | 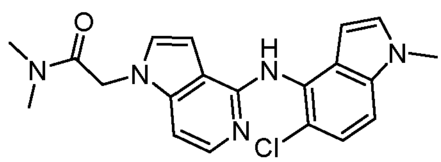 **Co. No. 104** |

(continued)

| INTERMEDIATE | REAGENT | COMPOUND |
|---|---|---|
| **I-1** | 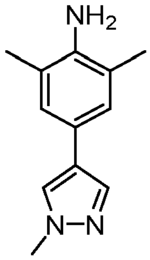 **I-80** | 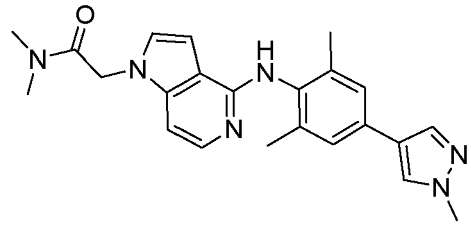 HCl salt **Co. No. 105** |
| **I-2** | 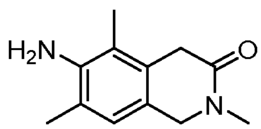 **I-110** | 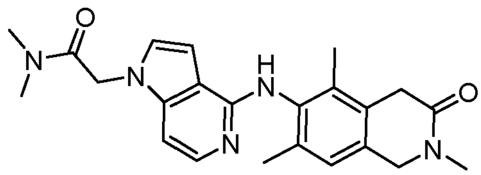 **Co. No. 106** |
| **I-1** | 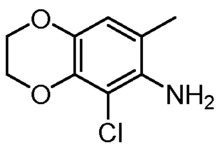 **I-52** | 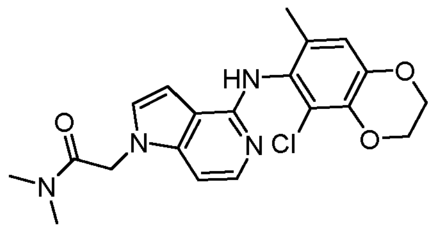 HCl salt **Co. No. 107** |
| **I-121** | 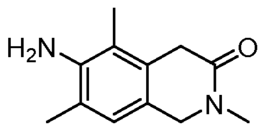 **I-110** | 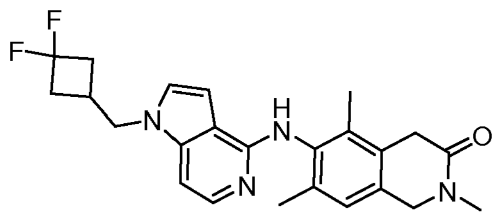 **Co. No. 108** |
| **I-112** | 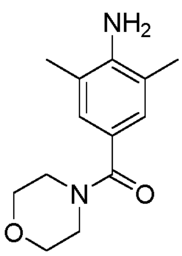 [1458654-04-0] | 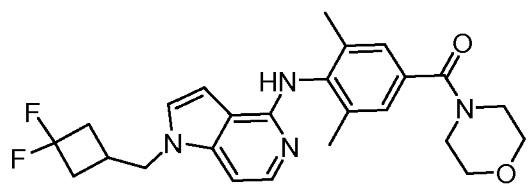 **Co. No. 109** |

PREPARATION OF COMPOUND **110**

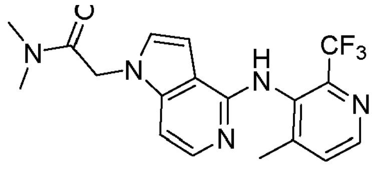

**110**

$Pd_2dba_3$ (27.7 mg, 30.2 μmol) was added to a mixture of XantPhos (35.0 mg, 60.5 μmol) and $Cs_2CO_3$ (296 mg, 0.91 mmol) in DMF (10 mL) and 1,4-dioxane (10 mL) while $N_2$ was bubbling. The mixture was stirred at 40 °C for 5 min and 3-bromo-4-methyl-2-(trifluoromethyl)pyridine [1448776-80-4] (165 mg, 0.61 mmol) was added. The mixture was stirred for 10 min and **I-117** (132 mg, 0.61 mmol) was added. The reaction mixture was stirred at 95 °C for 16 h. The reaction mixture was diluted with water and the aqueous phase was extracted with EtOAc (3 times). The combined organic extracts were dried ($MgSO_4$), filtered on Celite® and the filtrate was evaporated under reduced pressure. The crude mixture was purified by reverse phase chromatography (mobile phase: $NH_4CO_3$ (25 mM in $H_2O$)/(MeCN/MeOH, 1:1), gradient from 90:10 to 54:46). The residue was tritured with $Et_2O$ to afford compound 110 (23.3 mg, 10%) as a white solid.

The following compounds were prepared in an analogous manner to that described for compound **110** from the indicated starting materials and reagents.

| STARTING MATERIAL | REAGENT | COMPOUND |
|---|---|---|
| **I-118** | 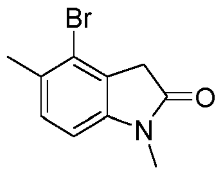 [1571136-59-8] | 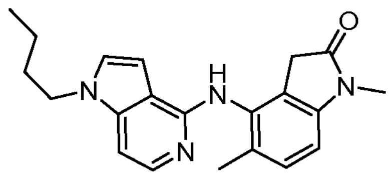 **Co. No. 111** |
| **I-1** | 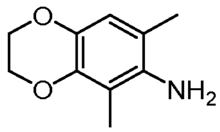 **I-109** | 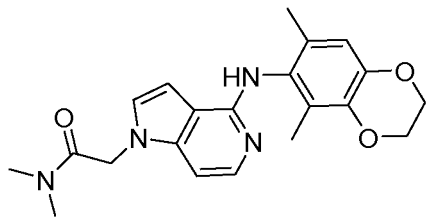 HCl salt **Co. No. 112** |

PREPARATION OF COMPOUND **113**

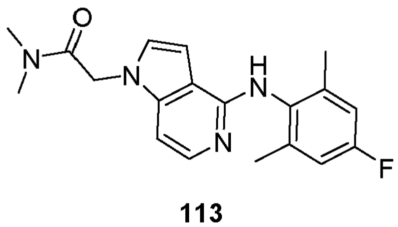

**113**

$Pd(OAc)_2$ (3.78 mg, 16.8 μmol), XantPhos (21.9 mg, 37.9 μmol) and $Cs_2CO_3$ (274 mg, 0.84 mmol) were added to a stirred solution of 4-fluoro-2,6-dimethylaniline [392-70-1] (64.4 mg, 0.46 mmol) and **I-1** (100 mg, 0.42 mmol) in 1,4-dioxane (1.5 mL). The reaction mixture was stirred at 90 °C for 12 h in a sealed tube, cooled to room temperature and filtered through Celite®. The residue was washed with EtOAc. The filtrate was evaporated under reduced pressure. The

crude mixture was purified by reverse phase chromatography (mobile phase: [$NH_4OAc$ (65 mM in $H_2O$)/MeCN (90:10)]/[MeCN/MeOH (1:1)], gradient from 90:10 to 54:46 to afford compound 113 (56 mg, 39%) as a white solid.

PREPARATION OF COMPOUND **114**

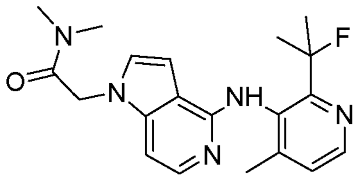

**114**

To a mixture of compound **85** (203 mg, 0.46 mmol) in DCM (2 mL) was added DAST (0.18 mL, 1.38 mmol) at 0 °C. The reaction mixture was stirred at room temperature for 1.5 h. The mixture was diluted with $NaHCO_3$ and extracted with DCM. The combined organic extracts were washed with water, dried ($MgSO_4$), filtered and concentrated under reduced pressure. The crude mixture was purified by flash column chromatography (silica, mobile phase: DCM/(MeOH/$NH_3$), gradient from 100:0 to 98:2). A second purification was performed via Prep SFC (stationary phase: Chiralpak Daicel IC 20 × 250 mm, mobile phase: $CO_2$, EtOH + 0.4% *i*-$PrNH_2$) to afford compound **114** (18 mg, 11%).

ANALYTICAL PART

MELTING POINTS

Values are either peak values or melt ranges, and are obtained with experimental uncertainties that are commonly associated with this analytical method.

DSC823e or DSC1 STAR (indicated as DSC) & Mettler Toledo MP50:
For a number of compounds, melting points were determined with a DSC823e or a DSC1 STAR (Mettler-Toledo). Melting points were measured with a temperature gradient of 10°C/minute. Maximum temperature was 300°C.

For a number of compounds, melting points were determined with a MP50 (Mettler-Toledo). Melting points were measured with a temperature gradient of 10°C/minute.

LCMS

GENERAL PROCEDURE

The High Performance Liquid Chromatography (HPLC) measurement was performed using a LC pump, a diode-array (DAD) or a UV detector and a column as specified in the respective methods. If necessary, additional detectors were included (see table of methods below).

Flow from the column was brought to the Mass Spectrometer (MS) which was configured with an atmospheric pressure ion source. It is within the knowledge of the skilled person to set the tune parameters (e.g. scanning range, dwell time...) in order to obtain ions allowing the identification of the compound's nominal monoisotopic molecular weight (MW). Data acquisition was performed with appropriate software. Compounds are described by their experimental retention times ($R_t$) and ions. If not specified differently in the table of data, the reported molecular ion corresponds to the $[M+H]^+$ (protonated molecule) and/or $[M-H]^-$ (deprotonated molecule). In case the compound was not directly ionizable the type of adduct is specified (i.e. $[M+NH_4]^+$, $[M+HCOO]^-$, etc....). For molecules with multiple isotopic patterns (Br, Cl), the reported value is the one obtained for the lowest isotope mass. All results were obtained with experimental uncertainties that are commonly associated with the method used. Hereinafter, "SQD" means Single Quadrupole Detector, "MSD" Mass Selective Detector, "RT" room temperature, "BEH" bridged ethylsiloxane/silica hybrid, "DAD" Diode Array Detector, "HSS" High Strength silica.

LCMS Method codes (Flow expressed in mL/min; column temperature (T) in °C; Run time in minutes)

TABLE 1. LC-MS Methods (Flow expressed in mL/min; column temperature (T) in °C; Run time in min).

| Method code | Instrument | Column | Mobile phase | Gradient | Flow-Col T | Run time (min) |
|---|---|---|---|---|---|---|
| 1 | Waters: Acquity® UPLC® -DAD and SQD | Waters : BEH (1.8μm, 2.1* 100mm) | A: 10mM $CH_3COONH_4$ in 95% $H_2O$ + 5% $CH_3CN$ B: $CH_3CN$ | From 100% A to 5% A in 2.10min, to 0% A in 0.90min, to 5% A in 0.5min | 0.6<br>-------<br>55 | 3.5 |
| 2 | Waters: Acquity® UPLC® -DAD, SQD and ELSD | Waters : HSS T3 (1.8μm, 2.1* 100mm) | A: 10mM $CH_3COONH_4$ in 95% $H_2O$ + 5% $CH_3CN$ B: $CH_3CN$ | From 100% A to 5% A in 2.10min, to 0% A in 0.90min, to 5% A in 0.5min | 0.6<br>-------<br>55 | 3.5 |
| 3 | Waters: Acquity® UPLC® - DAD and SQD | Waters : HSS T3 (1.8μm, 2.1* 100mm) | A: 10mM CH3COONH4 in 95% H2O + 5% CH3CN B: CH3CN | From 100% A to 5% A in 2.10min, to 0% A in 0.90min, to 5% A in 0.5min | 0.7<br>-------<br>55 | 3.5 |
| 4 | Waters: Acquity® UPLC® - DAD and SQD | Waters : BEH C18 (1.7μm, 2.1*50mm) | A: 10mM CH3COONH4 in 95% H2O + 5% CH3CN B: CH3CN | From 95% A to 5% A in 1.3min, held for 0.7 min | 0.8<br>-------<br>55 | 2 |
| 5 | Agilent: 1100-DAD and MSD | YMC: Pack ODS-AQ (3μm, 4.6x50mm) | A: HCOOH 0.1% in water, B: CH3CN | 95% A to 5% A in 4.8min, held for 1min, back to 95% A in 0.2min. | 2.6 | 6 |
| 6 | Waters: Acquity® UPLC® - DAD and SQD | Waters : BEH C18 (1.7μm, 2.1*50mm) | A: 10mM CH3COONH4 in 95% H2O + 5% CH3CN B: CH3CN | From 95% A to 5% A in 1.3 min, held for 0.2 min, to 95% A in 0.2 min held for 0.1 min | 0.7<br>-------<br>70 | 1.8 |
| 7 | Waters: Acquity® UPLC®-DAD, SQD | Waters : BEH (1.8μm, 2.1*100mm ) | A: 0.1% $NH_4HCO_3$ in $H_2O$ B: $CH_3CN$ | From 100% A to 5% A in 2.10min, to 0% A in 0.90min, to 5% A in 0.5min | 0.6<br>-------<br>55 | 3.5 |

TABLE 2. Analytical data - melting point (M.p.) and LCMS: $[M+H]^+$ means the protonated mass of the free base of the compound, $[M-H]^-$ means the deprotonated mass of the free base of the compound or the type of adduct specified $[M+CH_3COO]^-$). $R_t$ means retention time (in min). For some compounds exact mass was determined

| Co. No. | Mp (°C) | Rt (min) | UV Area % | $[M+H]^+$ | $[M-H]^-$ | LCMS Method |
|---|---|---|---|---|---|---|
| 1 | 232.30 | 1.48 | 100.00 | 352 | 350 | 1 |
| 2 | 251.73 | 1.25 | 100.00 | 362 | | 3 |
| 3 | | 1.04 | 100 | 345 | 343 | 1 |
| 4 | 221.78 | 1.13 | 100.00 | 340 | 338 | 1 |
| 5 | 215.70 | 0.74 | 100.00 | 363 | 361 | 4 |
| 6 | 228.29 | 1.82 | 98.10 | 419 | 417 | 1 |
| 7 | | 1.55 | 100.00 | 351 | 349 | 1 |
| 8 | | 1.34 | 100.00 | 405 | | 1 |
| 9 | | 1.11 | 100.00 | 338 | 336 | 1 |

(continued)

| Co. No. | Mp (°C) | Rt (min) | UV Area % | $[M+H]^+$ | $[M-H]^-$ | LCMS Method |
|---|---|---|---|---|---|---|
| 10 | | 1.09 | 100.00 | 336 | 334 | 1 |
| 11 | | | | | | |
| 12 | 211.88 | 1.27 | 100.00 | 356 | 354 | 1 |
| 13 | | 1.46 | 100.00 | 390 | 388 | |
| 14 | | 0.64 | 100.00 | 364 | 362 | 4 |
| 15 | | 1.28 | 100.00 | 344 | 342 | 2 |
| 16 | | 1.18 | 100.00 | 324 | 322 | 1 |
| 17 | | 1.09 | 100.00 | 324 | | 2 |
| 18 | 214.46 | 1.87 | 100.00 | 391 | 389 | 1 |
| 19 | | 0.92 | 90.81 | 368 | 366 | 4 |
| 20 | | 1.26 | 100.00 | 352 | 350 | 1 |
| 21 | 187.16 | 0.92 | 100.00 | 377 | 375 | 4 |
| 22 | 211.10 | 0.85 | 100.00 | 343 | 341 | 4 |
| 23 | 202.75 | 0.87 | 100.00 | 347 | | 4 |
| 24 | 200.76 | 0.98 | 100.00 | 413 | 411 | 4 |
| 25 | | 0.94 | 100.00 | 381 | 379 | 4 |
| 26 | | 0.88 | 100.00 | 389 | 387 | 4 |
| 27 | 69.34 | 1.06 | 100.00 | 455 | 453 | 4 |
| 28 | | 1.45 | 100.00 | 365 | 363 | 1 |
| 29 | | 0.95 | 86.29 | 415 | 413 | 4 |
| 30 | | 1.01 | 0.59 | 296 | 294 | 1 |
| 31 | 239.9[(b)] | 0.73 | 100.00 | 338 | 336 | 6 |
| 32 | | 0.35 | 99.00 | 310 | | 5 |
| 33 | 196.4[(b)] | 1.27 | 98.00 | 366 | | 5 |
| 34 | 188.2[(b)] | 0.71 | 99.00 | 338 | | 5 |
| 35 | 208.2[(b)] | 1.78 | 99.00 | 377 | | 5 |
| 36 | 178.1[(b)] | 0.47 | 99.00 | 311 | | 5 |
| 37 | | 1.64 | 0.99 | 350 | | 5 |
| 38 | | 1.31 | 99.22 | 360 | 358 | 1 |
| 39 | | 1.61 | 100 | 375 | 373 | 1 |
| 40 | 191.6[(b)] | 1.57 | 100 | 361 | | 1 |
| 41 | | 1.61 | 100 | 361 | | 1 |
| 42 | 209.9[(b)] | 0.78 | 98.54 | 357 | 355 | 4 |
| 43 | 210.4[(b)] | 0.85 | 100 | 377 | 375 | 4 |
| 44 | | 1.75 | 100 | 395 | 393 | 1 |
| 45 | | 1.91 | 96.95 | 401 | | 1 |
| 46 | | 1.48 | 100 | 376 | | 1 |
| 47 | | 0.65 | 100 | 367 | 365 | 4 |

(continued)

| Co. No. | Mp (°C) | Rt (min) | UV Area % | $[M+H]^+$ | $[M-H]^-$ | LCMS Method |
|---|---|---|---|---|---|---|
| 48 | 209.8[(b)] | 1.8 | 100 | 395 | | 1 |
| 49 | | 1.47 | 100 | 345 | | 1 |
| 50 | | 1.74 | 100 | 409 | 407 | 1 |
| 51 | | 1.67 | 100 | 395 | 393 | 1 |
| 52 | | 1.67 | 100 | 409 | | 1 |
| 53 | | 1.73 | 96.51 | 425 | 423 | 1 |
| 54 | 182.9[(b)] | 1.83 | 100 | 427 | | 1 |
| 55 | | 0.98 | 100 | 409 | 407 | 4 |
| 56 | | 1.61 | 99.66 | 359 | 357 | 1 |
| 57 | | 1.68 | 100 | 375 | 373 | 1 |
| 58 | | 1.63 | 98.84 | 392 | 390 | 1 |
| 59 | | 1.18 | 100 | 338 | 336 | 1 |
| 60 | | 1.75 | 100 | 429 | 427 | 1 |
| 61 | | 1.43 | 99.03 | 366 | 364 | 1 |
| 62 | | 2 | 98.71 | 405 | 403 | 7 |
| 63 | | 0.81 | 96.44 | 375 | | 4 |
| 64 | | 0.75 | 100 | 370 | 368 | 4 |
| 65 | | 1.78 | 100 | 409 | 407 | 1 |
| 66 | | 1.52 | 100 | 431 | 429 | 1 |
| 67 | | 1.94 | 98.81 | 425 | 423 | 1 |
| 68 | | 1.28 | 100 | 360 | | 1 |
| 69 | 161.1[(b)] | 1 | 100 | 435 | 433 | 4 |
| 70 | | 0.8 | 100 | 395 | 393 | 4 |
| 71 | 185.4[(b)] | 0.88 | 100 | 407 | 405 | 4 |
| 72 | 188.2[(b)] | 0.91 | 100 | 411 | 409 | 4 |
| 73 | | 1.67 | 98.24 | 409 | 407 | 1 |
| 74 | 192.1[(b)] | 1.49 | 100 | 359 | 359 | 1 |
| 75 | 196.9[(b)] | 1.64 | 98.18 | 379 | 377 | 1 |
| 76 | 220.5[(b)] | 0.76 | 100 | 361 | 359 | 4 |
| 77 | | 0.95 | 100 | 411 | 409 | 4 |
| 78 | | 0.96 | 100 | 411 | 409 | 4 |
| 79 | | 0.88 | 96.93 | 397 | 395 | 4 |
| 80 | 195.7[(b)] | 1.9 | 100 | 425 | 423 | 1 |
| 81 | | 0.85 | 96.28 | 377 | 375 | 4 |
| 82 | | 0.92 | 94.52 | 397 | 395 | 4 |
| 83 | 221.8[(b)] | 1.85 | 97.73 | 369 | 367 | 7 |
| 83 | | 1.85 | 97.73 | 369 | 367 | 7 |
| 84 | | | | | | 7 |

(continued)

| Co. No. | Mp (°C) | Rt (min) | UV Area % | $[M+H]^+$ | $[M-H]^-$ | LCMS Method |
|---|---|---|---|---|---|---|
| 85 | | 1.29 | 95.32 | 368 | | 1 |
| 86 | | 1.75 | 95.91 | 390 | 388 | 7 |
| 86 | | 1.75 | 95.91 | 390 | 388 | 7 |
| 87 | | 1.18 | 100 | 368 | 366 | 1 |
| 88 | | 0.64 | 98.4 | 385 | | 4 |
| 89 | | 1.58 | 98.87 | 390 | 388 | 1 |
| 90 | | 1.22 | 100 | 324 | 322 | 1 |
| 114 | | 1.44 | 96.8 | 370 | 368 | 1 |

PHARMACOLOGICAL EXAMPLES

1) OGA - BIOCHEMICAL ASSAY

The assay is based on the inhibition of the hydrolysis of fluorescein mono-β-D-N-Acetyl-Glucosamine (FM-GlcNAc) (Mariappa et al. 2015, Biochem J 470:255) by the recombinant human Meningioma Expressed Antigen 5 (MGEA5), also referred to as O-GlcNAcase (OGA). The hydrolysis FM-GlcNAc (Marker Gene technologies, cat # M1485) results in the formation of β-D-N-glucosamineacetate and fluorescein. The fluorescence of the latter can be measured at excitation wavelength 485 nm and emission wavelength 538nm. An increase in enzyme activity results in an increase in fluorescence signal. Full length OGA enzyme was purchased at OriGene (cat # TP322411). The enzyme was stored in 25 mM Tris.HCl, pH 7.3, 100 mM glycine, 10% glycerol at -20 °C. Thiamet G and GlcNAcStatin were tested as reference compounds (Yuzwa et al. 2008 Nature Chemical Biology 4:483; Yuzwa et al. 2012 Nature Chemical Biology 8:393). The assay was performed in 200mM Citrate/phosphate buffer supplemented with 0.005% Tween-20. 35.6 g $Na_2HPO_4$ 2 $H_2O$ (Sigma, # C0759) were dissolved in 1 L water to obtain a 200 mM solution. 19.2 g citric acid (Merck, # 1.06580) was dissolved in 1 L water to obtain a 100 mM solution. pH of the sodiumphosphate solution was adjusted with the citric acid solution to 7.2. The buffer to stop the reaction consists of a 500 mM Carbonate buffer, pH 11.0. 734 mg FM-GlcNAc were dissolved in 5.48 mL DMSO to obtain a 250 mM solution and was stored at -20 °C. OGA was used at a 2 nM concentration and FM-GlcNAc at a 100uM final concentration. Dilutions were prepared in assay buffer.

50 nl of a compound dissolved in DMSO was dispensed on Black Proxiplate TM 384 Plus Assay plates (Perkin Elmer, #6008269) and 3 μl fl-OGA enzyme mix added subsequently. Plates were pre-incubated for 60 min at room temperature and then 2 μl FM-GlcNAc substrate mix added. Final DMSO concentrations did not exceed 1%.

Plates were briefly centrifuged for 1 min at 1000rpm and incubate at room temperature for 6 h. To stop the reaction 5 μl STOP buffer were added and plates centrifuge again 1 min at 1000rpm. Fluorescence was quantified in the Thermo Scientific Fluoroskan Ascent or the PerkinElmer EnVision with excitation wavelength 485 nm and emission wavelength 538 nm.

For analysis a best-fit curve is fitted by a minimum sum of squares method. From this an $IC_{50}$ value and Hill coefficient was obtained. High control (no inhibitor) and low control (saturating concentrations of standard inhibitor) were used to define the minimum and maximum values.

2) OGA - CELLULAR ASSAY

HEK293 cells inducible for P301L mutant human Tau (isoform 2N4R) were established at Janssen. Thiamet-G was used for both plate validation (high control) and as reference compound (reference $EC_{50}$ assay validation). OGA inhibition is evaluated through the immunocytochemical (ICC) detection of O-GlcNAcylated proteins by the use of a monoclonal antibody (CTD110.6; Cell Signaling, #9875) detecting O-GlcNAcylated residues as previously described (Dorfmueller et al. 2010 Chemistry & biology, 17:1250). Inhibition of OGA will result in an increase of O- GlcNAcylated protein levels resulting in an increased signal in the experiment. Cell nuclei are stained with Hoechst to give a cell culture quality control and a rough estimate of immediate compounds toxicity, if any. ICC pictures are imaged with a Perkin Elmer Opera Phenix plate microscope and quantified with the provided software Perkin Elmer Harmony 4.1. Cells were propagated in DMEM high Glucose (Sigma, #D5796) following standard procedures. 2 days before the cell assay cells are split, counted and seeded in Poly-D-Lysine (PDL) coated 96-wells (Greiner, #655946) plate at a cell density of 12,000 cells per $cm^2$ (4,000 cells per well) in 100μl of Assay Medium (Low Glucose medium is used to reduce basal levels of

GlcNAcylation) (Park et al. 2014 The Journal of biological chemistry 289:13519). At the day of compound test medium from assay plates was removed and replenished with 90μl of fresh Assay Medium. 10μl of compounds at a 10fold final concentration were added to the wells. Plates were centrifuged shortly before incubation in the cell incubator for 6 hours. DMSO concentration was set to 0.2%. Medium is discarded by applying vacuum. For staining of cells medium was removed and cells washed once with 100 μl D-PBS (Sigma, #D8537). From next step onwards unless other stated assay volume was always 50μl and incubation was performed without agitation and at room temperature. Cells were fixed in 50μl of a 4% paraformaldehyde (PFA, Alpha aesar, # 043368) PBS solution for 15 minutes at room temperature. The PFA PBS solution was then discarded and cells washed once in 10mM Tris Buffer (LifeTechnologies, # 15567-027), 150mM NaCl (LifeTechnologies, #24740-0110, 0.1% Triton X (Alpha aesar, # A16046), pH 7.5 (ICC buffer) before being permeabilized in same buffer for 10 minutes. Samples are subsequently blocked in ICC containing 5% goat serum (Sigma, #G9023) for 45-60 minutes at room temperature. Samples were then incubated with primary antibody (1/1000 from commercial provider, see above) at 4°C overnight and subsequently washed 3 times for 5 minutes in ICC buffer. Samples were incubated with secondary fluorescent antibody (1/500 dilution, Lifetechnologies, # A-21042) and nuclei stained with Hoechst 33342 at a final concentration of 1μg/ml in ICC (Lifetechnologies, # H3570) for 1 hour. Before analysis samples were washed 2 times manually for 5 minutes in ICC base buffer.

Imaging is performed using Perkin Elmer Phenix Opera using a water 20x objective and recording 9 fields per well. Intensity readout at 488nm is used as a measure of O-GlcNAcylation level of total proteins in wells. To assess potential toxicity of compounds nuclei were counted using the Hoechst staining. $IC_{50}$-values are calculated using parametric non-linear regression model fitting. As a maximum inhibition Thiamet G at a 200uM concentration is present on each plate. In addition, a concentration response of Thiamet G is calculated on each plate.

TABLE 3. Results in the biochemical and cellular assays.

| Co. No. | Enzymatic hOGA; $pIC_{50}$ | Enzymatic Emax (%) | Cellular hOGA; $pEC_{50}$ | Cellular $E_{max}$ (%) |
|---|---|---|---|---|
| 1 | 8.31 | 103 | 7.51 | 109 |
| 2 | 8.24 | 103 | 7.5 | 79 |
| 3 | 6.16 | 93 | | |
| 4 | 7.24 | 101 | 6.44 | 68 |
| 5 | 8.56 | 103 | 7.55 | 89 |
| 6 | 8.53 | 102 | 8.29 | 96 |
| 7 | 7.65 | 102 | 7.19 | 88 |
| 8 | 5.96 | 98 | | |
| 9 | 6.41 | 95 | | |
| 10 | 6.71 | 97 | | |
| 11 | 6.41 | 99 | | |
| 12 | 6.28 | 95 | | |
| 13 | 6.31 | 95 | <6 | 15 |
| 14 | 7.26 | 99 | 6.23 | 55 |
| 15 | 7.51 | 102 | ~6.2 | 68 |
| 16 | 7.69 | 104 | 6.85 | 86 |
| 17 | 7.85 | 101 | 6.83 | 79 |
| 18 | 8.53 | 104 | 7.91 | 86 |
| 19 | 8.2 | 102 | 7.22 | 80 |
| 20 | 8.11 | 100 | 7.49 | 91 |
| 21 | 8.29 | 101 | 7.82 | 77 |
| 22 | 8.39 | 103 | 7.8 | 86 |
| 23 | 7.85 | 103 | 7.09 | 98 |

(continued)

| Co. No. | Enzymatic hOGA; $pIC_{50}$ | Enzymatic Emax (%) | Cellular hOGA; $pEC_{50}$ | Cellular $E_{max}$ (%) |
|---|---|---|---|---|
| 24 | 8.06 | 101 | 7.26 | 87 |
| 25 | 8.28 | 101 | 7.47 | 87 |
| 26 | 8.38 | 103 | 7.93 | 81 |
| 27 | 8.44 | 104 | 8 | 87 |
| 28 | 7.63 | 100 | 6.48 | 76 |
| 29 | 8.15 | 102 | 7.24 | 85 |
| 30 | 6.93 | 100 | | |
| 31 | 6.32 | 97 | | |
| 32 | 8.33 | 104 | 7.54 | 98 |
| 33 | 6.54 | 98 | | |
| 34 | 6.44 | 97 | | |
| 35 | 7.09 | 100 | <6 | 39 |
| 36 | 8.3 | 103 | 7.69 | 78 |
| 37 | 8.88 | 103 | 7.44 | 85 |
| 38 | 7.18 | 100.385 | 6.06 | 50.1581 |
| 39 | 8 | 103.935 | 7.29 | 89.7319 |
| 40 | 8.25 | 99.01 | ~7.58 | 81.23045 |
| 41 | 7.71 | 98.05 | 7.34 | 69.456 |
| 42 | 8.09 | 101.475 | 7.71 | 93.5478 |
| 43 | 8.18 | 101.11 | 7.57 | 80.4125 |
| 44 | 7.86 | 103.07 | 7.17 | 82.3391 |
| 45 | 7.14 | 102.53 | 6.81 | 74.4194 |
| 46 | 7.44 | 102.665 | 6.86 | 75.29775 |
| 47 | 7.56 | 98.735 | 6.32 | 65.072 |
| 48 | 7.57 | 99.24 | 7.22 | 73.5737 |
| 49 | 7.58 | 99.39 | 6.87 | 84.96725 |
| 50 | 7.93 | 100.07 | 7.42 | 76.7245 |
| 51 | 7.98 | 104.22 | 7.18 | 93.48935 |
| 52 | 8.45 | 101.845 | 7.8 | 94.5654 |
| 53 | 8.48 | 94.735 | ~8.12 | 106.011 |
| 54 | 8.52 | 100.945 | 7.83 | 85.60435 |
| 55 | 7.81 | 98.76 | 7.47 | 98.55725 |
| 56 | 7.84 | 101.04 | 7.3 | 72.76705 |
| 57 | 7.93 | 99.23 | 7.53 | 76.22965 |
| 58 | 8 | 100.8 | 7.14 | 87.22285 |
| 59 | 8.12 | 101.56 | 7.21 | 87.3268 |
| 60 | 8.27 | 100.365 | 7.58 | 83.3741 |
| 61 | 8.29 | 99.75 | 7.76 | 94.6773 |

(continued)

| Co. No. | Enzymatic hOGA; $pIC_{50}$ | Enzymatic Emax (%) | Cellular hOGA; $pEC_{50}$ | Cellular $E_{max}$ (%) |
|---|---|---|---|---|
| 62 | 8.66 | 100.75 | 8.03 | 102.6281 |
| 63 | 8.14 | 99.395 | 8.04 | 81.1544 |
| 64 | 8.32 | 101.945 | 7.31 | 78.643 |
| 65 | 8.5 | 99.195 | ~8.22 | 89.13485 |
| 66 | 8.34 | 100.67 | 7.82 | 84.383 |
| 67 | 8.56 | 101.305 | 7.8 | 85.87875 |
| 68 | 7.78 | 101.375 | 6.85 | 79.9009 |
| 69 | 8.55 | 101.48 | 8.32 | 97.6203 |
| 70 | 8.07 | 98.98 | 7.54 | 94.8158 |
| 71 | 8.38 | 101.05 | ~8 | 85.80715 |
| 72 | 8.42 | 99.97 | 7.72 | 77.6232 |
| 73 | 7.69 | 101.67 | 6.74 | 85.3271 |
| 74 | 7.89 | 99.335 | 7.4 | 81.3139 |
| 75 | 8.22 | 99.96 | 7.41 | 93.926 |
| 76 | 8.26 | 101.72 | 7.43 | 79.8092 |
| 77 | 8.49 | 99.685 | 7.82 | 80.2154 |
| 78 | 8.43 | 96.635 | 7.43 | 106.0394 |
| 79 | 8.51 | 97.04 | 7.77 | 92.4264 |
| 80 | 8.51 | 98.81 | 8.18 | 89.9233 |
| 81 | 8.67 | 100.41 | 8.28 | 103.9332 |
| 82 | 8.81 | 95.03 | ~8.17 | 106.7803 |
| 83 | 8.41 | 103.835 | 7.97 | 102.6916 |
| 84 | 8.61 | 100.96 | 7.76 | 83.0277 |
| 85 | 7.37 | 101.165 | | |
| 86 | 7.66 | 100.445 | 7.15 | 65.18655 |
| 87 | 7.81 | 99.935 | 7.09 | 88.6943 |
| 88 | 7.84 | 101.495 | 7.62 | 93.07625 |
| 89 | 8.22 | 100.56 | 7.49 | 89.52315 |
| 90 | 7.81 | 100.86 | 6.31 | 58.03765 |
| 91 | 6.32 | 94.315 | <6 | 41.8697 |
| 92 | 6.37 | 94.605 | <6 | 22.40395 |
| 93 | 6.84 | 97.715 | <6 | 41.49375 |
| 94 | 7.75 | 99.365 | 7.2 | 70.7898 |
| 95 | 8.16 | 99.185 | 7.79 | 79.90805 |
| 96 | 8.11 | 101.42 | 7.95 | 91.97595 |
| 97 | 7.38 | 101.275 | 6.81 | 73.01495 |
| 98 | 7.41 | 99.82 | 6.18 | 50.6643 |
| 99 | 7.48 | 98.395 | 6.96 | 80.02295 |

(continued)

| Co. No. | Enzymatic hOGA; $pIC_{50}$ | Enzymatic Emax (%) | Cellular hOGA; $pEC_{50}$ | Cellular $E_{max}$ (%) |
|---|---|---|---|---|
| 100 | 7.67 | 100.785 | ~7.11 | 81.38875 |
| 101 | 7.83 | 101.14 | 7.79 | 80.4162 |
| 102 | 7.73 | 99.945 | 7.69 | 73.4892 |
| 103 | 8.27 | 100.845 | ~6.99 | 81.90755 |
| 104 | 8.32 | 100.005 | 7.33 | 80.6562 |
| 105 | 8.3 | 102.84 | | |
| 106 | 8.28 | 100.88 | ~7.26 | 89.78595 |
| 107 | 8.29 | 100.085 | 7.86 | 90.239 |
| 108 | 7.98 | 101.425 | ~7.74 | 81.7214 |
| 109 | 7.93 | 98.84 | 7.67 | 79.5067 |
| 110 | 7.81 | 98.82 | 6.56 | 78.788 |
| 111 | 7.96 | 102.71 | 7.16 | 85.0824 |
| 112 | 8.47 | 99.935 | 7.89 | 82.8988 |
| 113 | 8.09 | 102.695 | 7.57 | 83.01505 |
| 114 | 8.37 | 102.02 | 7.11 | 85.24545 |

## Claims

**1.** A compound of Formula (I)

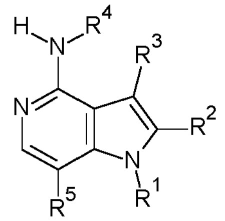

(I),

or a tautomer or a stereoisomeric form thereof, wherein

$R^1$ is $-C_{1-6}alkyl-C(O)-NR^xR^y$, wherein
$R^x$ and $R^y$ are each independently selected from the group consisting of hydrogen and $C_{1-3}alkyl$; or $R^x$ and $R^y$ together with the nitrogen atom to which they are attached form a heterocyclyl ring selected from the group consisting of azetidinyl, pyrrolidinyl, piperidinyl, piperazinyl and morpholinyl;
$R^2$, $R^3$ and $R^5$ are each independently selected from the group consisting of hydrogen, halo and $C_{1-3}alkyl$;
$R^4$ is a monovalent radical selected from the group consisting of (a), (b), (c), (d), (e) and (f):

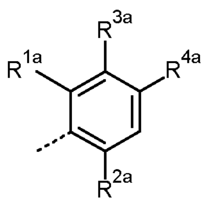

(a)

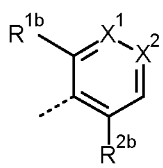

(b)

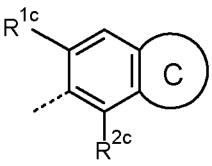 (c) 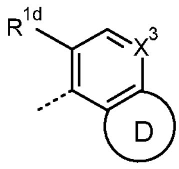 (d) 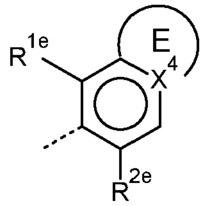 (e) 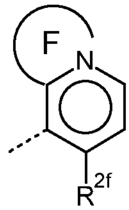 (f)

wherein

$R^{1a}$, $R^{2a}$, $R^{1b}$, and $R^{2b}$ are each independently selected from the group consisting of halo, $C_{1-3}$alkyl, monohalo$C_{1-3}$alkyl, polyhalo$C_{1-3}$alkyl, $C_{1-3}$alkyloxy, monohalo$C_{1-3}$alkyloxy, polyhalo$C_{1-3}$alkyloxy, and $C_{3-6}$cycloalkyl;

$R^{3a}$ is selected from the group consisting of hydrogen, halo, -C(O)-O$C_{1-3}$alkyl, -C(O)-NR'R", -N(R‴)-C(O)-$C_{1-3}$alkyl;

$R^{4a}$ is selected from the group consisting of hydrogen, halo, -CN, $C_{1-3}$alkyl, monohalo$C_{1-3}$alkyl, polyhalo$C_{1-3}$alkyl, $C_{1-3}$alkyloxy, monohalo$C_{1-3}$alkyloxy, polyhalo$C_{1-3}$alkyloxy,

-C(O)-O$C_{1-3}$alkyl, -C(O)-NR'R", -N(R‴)-C(O)-$C_{1-3}$alkyl, and Het;

with the proviso that $R^{3a}$ and $R^{4a}$ are not simultaneously -C(O)-O$C_{1-3}$alkyl,

-C(O)-NR'R", or -N(R‴)-C(O)-$C_{1-3}$alkyl;

R' and R" are each independently selected from the group consisting of hydrogen and $C_{1-3}$alkyl; or R' and R" together with the nitrogen atom to which they are attached form a heterocyclyl ring selected from the group consisting of azetidinyl, pyrrolidinyl,

piperidinyl, piperazinyl and morpholinyl;

R‴ is selected from the group consisting of hydrogen and $C_{1-3}$alkyl;

Het is pyrazolyl or imidazolyl, optionally substituted with one or more independently selected $C_{1-3}$alkyl substituents;

$X^1$ and $X^2$ are each independently selected from N and CH, with the proviso that at least one of $X^1$ or $X^2$ is N;

$R^{1c}$, $R^{2c}$, $R^{1d}$, $R^{1e}$, $R^{2e}$, and $R^{2f}$ are each independently selected from the group consisting of halo, $C_{1-3}$alkyl, monohalo$C_{1-3}$alkyl, polyhalo$C_{1-3}$alkyl, $C_{1-3}$alkyloxy, monohalo$C_{1-3}$alkyl, polyhalo$C_{1-3}$alkyl, and $C_{3-6}$cycloalkyl;

$X^3$ represents CH or N;

$X^4$ represents C or N;

and each of the rings represented by

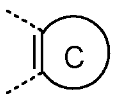, 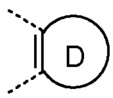, 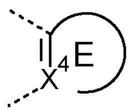, and 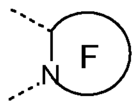,

form

(i) a 5- or 6-membered unsaturated heterocycle having one, two or three heteroatoms each independently selected from nitrogen and oxygen, and which is optionally substituted with one or more substituents, each independently selected from halo, $C_{1-3}$alkyl and oxo; or

(ii) a 5- or 6-membered aromatic heterocycle having one, two or three heteroatoms each independently selected from nitrogen, oxygen, and sulfur, and which is optionally substituted with one or more substituents, each independently selected from halo, -CN, $C_{1-3}$alkyl, monohalo$C_{1-3}$alkyl, and polyhalo$C_{1-3}$alkyl;

or a pharmaceutically acceptable addition salt or a solvate thereof.

**2.** The compound according to claim 1, wherein

$R^1$ is -$C_{1-3}$alkyl-C(O)-$NR^xR^y$, wherein

$R^x$ and $R^y$ are each independently selected from the group consisting of hydrogen and $C_{1-3}$alkyl; or $R^x$ and $R^y$ together with the nitrogen atom to which they are attached form a heterocyclyl ring selected from the group consisting of azetidinyl, pyrrolidinyl, piperidinyl, piperazinyl and morpholinyl;

$R^2$, $R^3$ and $R^5$ are each independently selected from the group consisting of hydrogen, halo and $C_{1-3}$alkyl;

$R^4$ is a monovalent radical selected from the group consisting of (a), (b), (d) and (f), wherein

$R^{1a}$, $R^{2a}$, $R^{1b}$, and $R^{2b}$ are each independently selected from the group consisting of halo, $C_{1-3}$alkyl, monohalo$C_{1-3}$alkyl, polyhalo$C_{1-3}$alkyl, $C_{1-3}$alkyloxy, monohalo$C_{1-3}$alkyloxy, polyhalo$C_{1-3}$alkyloxy, and $C_{3-6}$cycloalkyl;
$R^{3a}$ is hydrogen;
$R^{4a}$ is selected from the group consisting of hydrogen, halo, -CN, $C_{1-3}$alkyl, monohalo$C_{1-3}$alkyl, polyhalo$C_{1-3}$alkyl, $C_{1-3}$alkyloxy, monohalo$C_{1-3}$alkyloxy, and polyhalo$C_{1-3}$alkyloxy;
$X^1$ and $X^2$ are each independently selected from N and CH, with the proviso that at least one of $X^1$ or $X^2$ is N;
$R^{1d}$ and $R^{2f}$ are each independently selected from $C_{1-3}$alkyl;
$X^3$ represents CH;
and each of the rings represented by

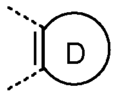 and 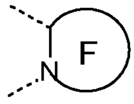

form

(i) a 5- or 6-membered unsaturated heterocycle having one or two heteroatoms each independently selected from nitrogen and oxygen, and which is optionally substituted with one or more substituents, each independently selected from halo and $C_{1-3}$alkyl; or
(ii) a 5- or 6-membered aromatic heterocycle having one, two or three heteroatoms each independently selected from nitrogen and oxygen, and which is optionally substituted with one or more substituents, each independently selected from $C_{1-3}$alkyl.

**3.** The compound according to claim 1 or 2, wherein

$R^1$ is -$C_{1-3}$alkyl-C(O)-$NR^xR^y$, wherein
$R^x$ and $R^y$ are each independently selected from the group consisting of hydrogen and $C_{1-3}$alkyl; or $R^x$ and $R^y$ together with the nitrogen atom to which they are attached form a heterocyclyl ring selected from the group consisting of azetidinyl, pyrrolidinyl, piperidinyl, piperazinyl and morpholinyl;
$R^2$, $R^3$ and $R^S$ are each independently selected from the group consisting of hydrogen, halo and $C_{1-3}$alkyl;
$R^4$ is a monovalent radical selected from the group consisting of (a), (b), (d) and (f), wherein
$R^{1a}$, $R^{2a}$, $R^{1b}$, and $R^{2b}$ are each independently selected from the group consisting of halo, $C_{1-3}$alkyl, polyhalo$C_{1-3}$alkyl, $C_{1-3}$alkyloxy, and polyhalo$C_{1-3}$alkyloxy;
$R^{3a}$ is hydrogen;
$R^{4a}$ is selected from the group consisting of hydrogen, halo, -CN, $C_{1-3}$alkyl, polyhalo$C_{1-3}$alkyl, $C_{1-3}$alkyloxy, and polyhalo$C_{1-3}$alkyloxy;
$X^1$ and $X^2$ are each independently selected from N and CH, with the proviso that at least one of $X^1$ or $X^2$ is N;
$R^{1d}$ and $R^{2f}$ are each independently selected from $C_{1-3}$alkyl;
$X^3$ represents CH;
and each of the rings represented by

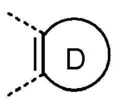 and 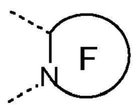

form

(i) a 5- or 6-membered unsaturated heterocycle having one or two nitrogen atoms, and which is optionally substituted with one or more substituents, each independently selected from halo and $C_{1-3}$alkyl; or
(ii) a 5- or 6-membered aromatic heterocycle having one or two nitrogen atoms, and which is optionally substituted with one or more substituents, each independently selected from $C_{1-3}$alkyl.

**4.** The compound according to any one of claims 1 to 3, wherein
$R^1$ is -$C_{1-3}$alkyl-C(O)-$NR^xR^y$, wherein -$NR^xR^y$ is selected from the group consisting of -$NH_2$, -$NHCH_3$, -$NH(CH_3)_2$, -$N(CH_3)(CH_2CH_3)$, azetidin-1-yl, and pyrrolidin-1-yl.

5. The compound according to any one of claims 1 to 4, wherein $R^2$, $R^3$ and $R^5$ are each independently selected from hydrogen and methyl.

6. The compound according to any one of claims 1 to 5, wherein $R^{1a}$, $R^{2a}$, $R^{1b}$, and $R^{2b}$ are each independently selected from the group consisting of fluoro, chloro, methyl, isopropyl, $CF_3$, $-OCH_3$ and $-OCF_3$.

7. The compound according to any one of claims 1 to 6, wherein $X^1$ is N and $X^2$ is CH, or $X^1$ is CH and $X^2$ is N, or $X^1$ and $X^2$ are both N.

8. The compound according to any one of claims 1 to 7, wherein $R^{1d}$, and $R^{2f}$ are each independently methyl or isopropyl.

9. The compound according to claim 1, wherein the compound is

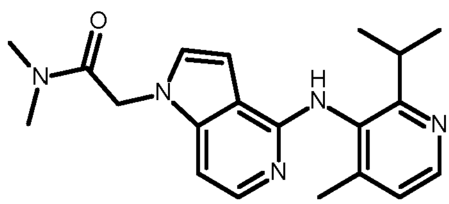

1

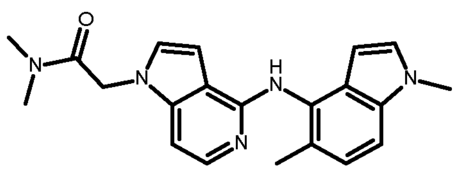

2

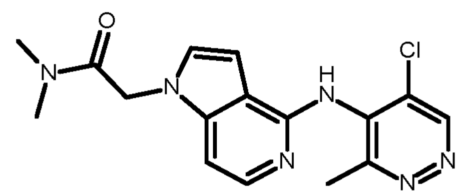

3

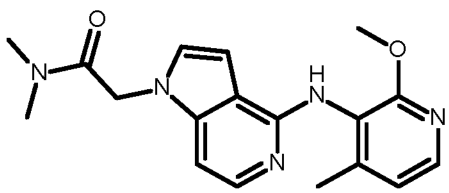

4

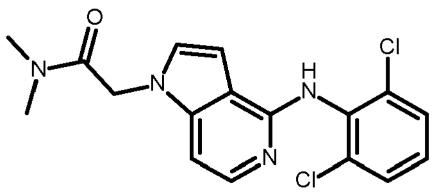

5

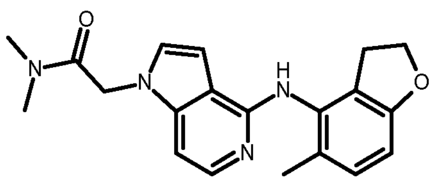

7

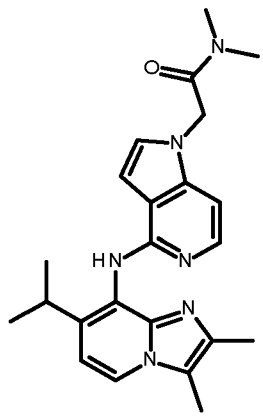

8

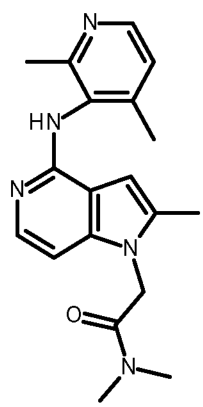

9

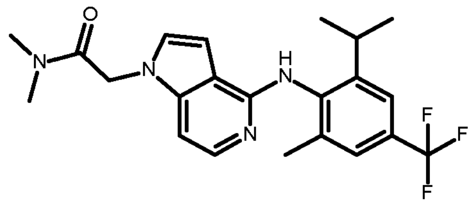

6

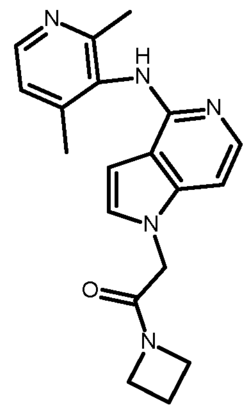

10

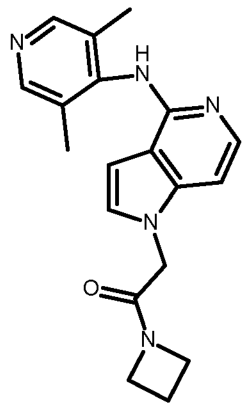

11

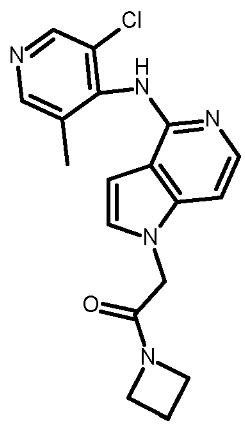

12

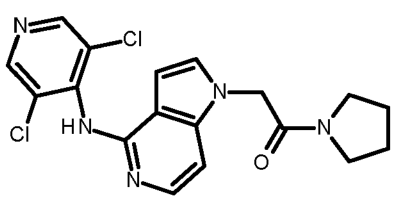

13

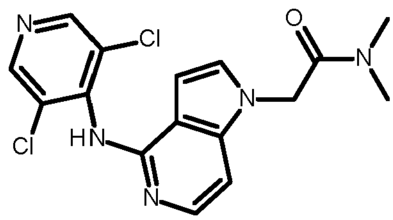

14

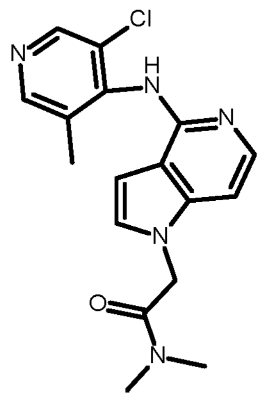

15

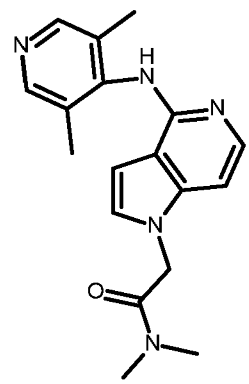

16

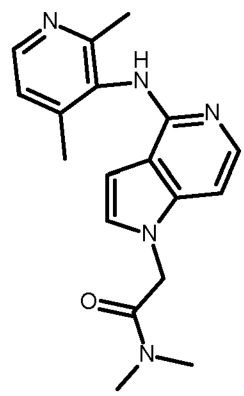

17

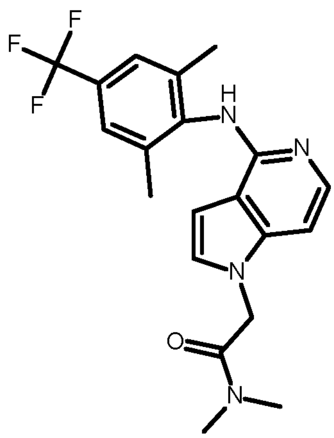

18

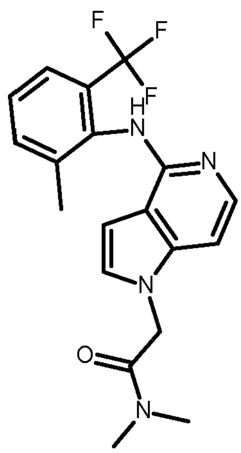

21

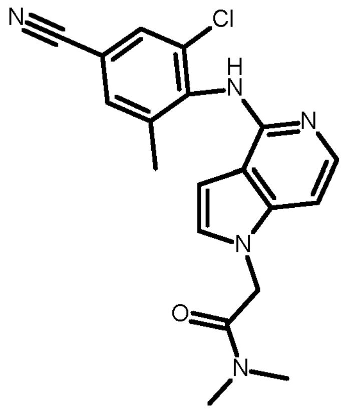

19

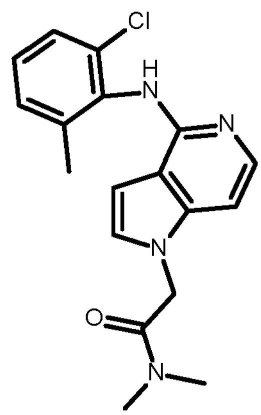

22

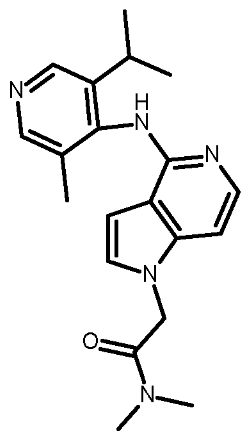

20

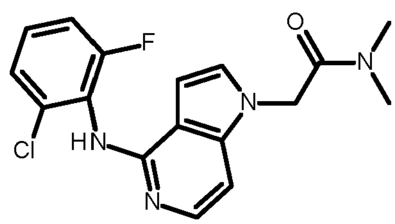

23

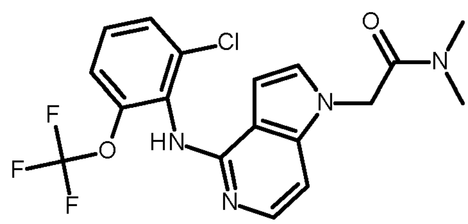

24

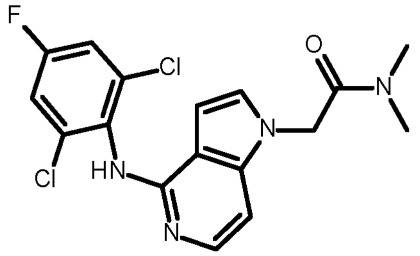

25

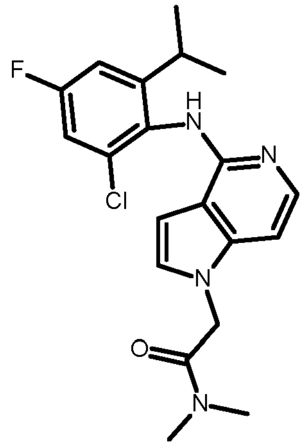

26

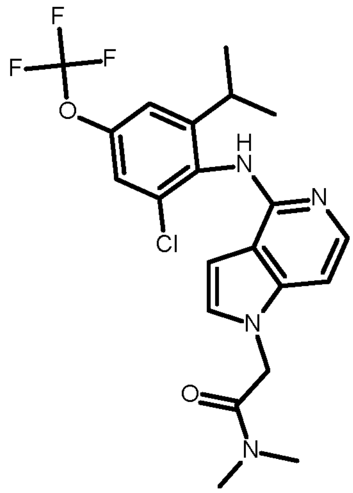

27

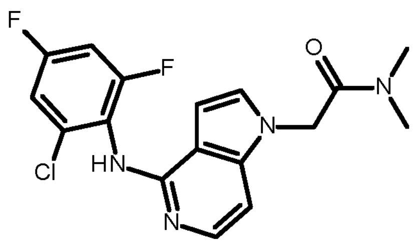

28

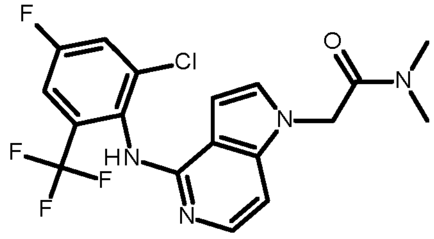

29

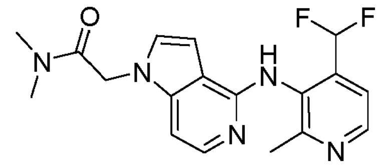

38

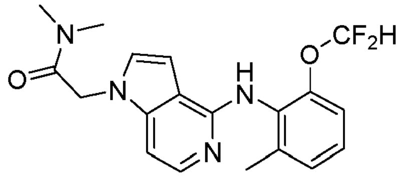

39

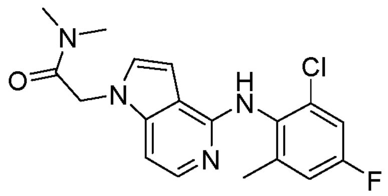

40

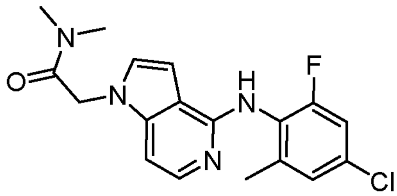

41

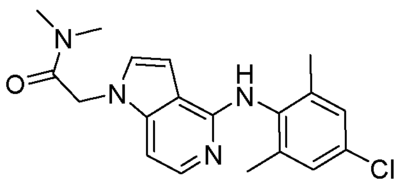

42

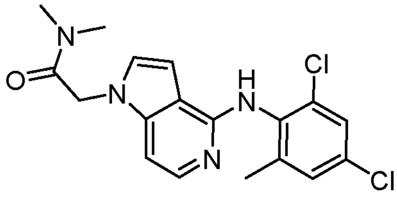

43

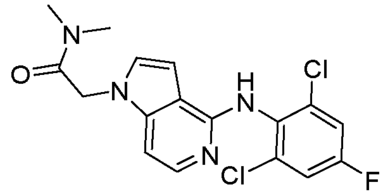

44

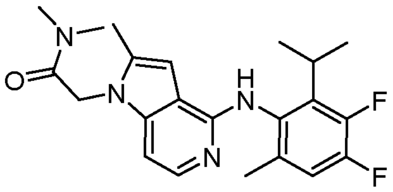

45

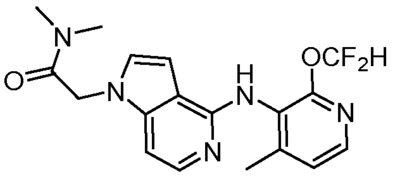

46

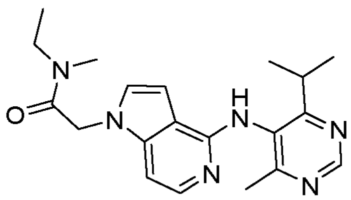

47

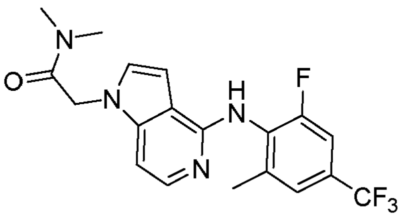

48

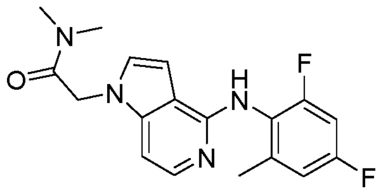

49

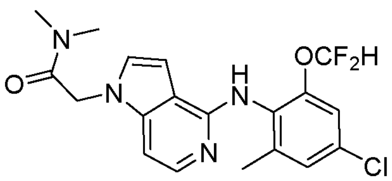

50

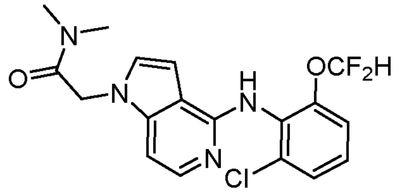

51

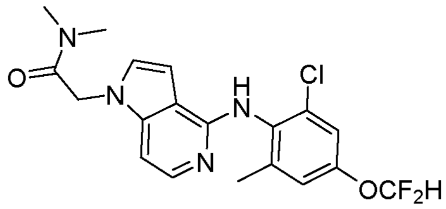

52

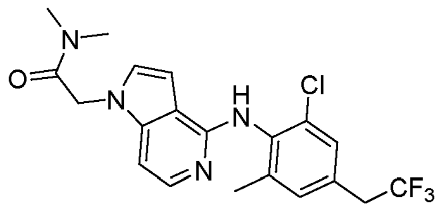

53

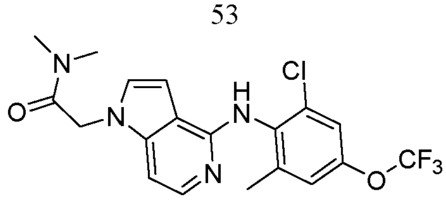

54

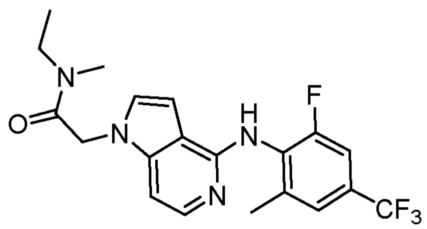

55

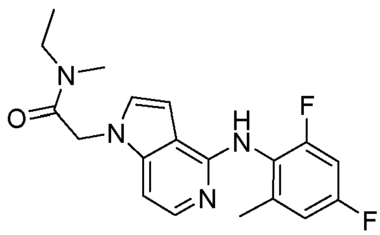

56

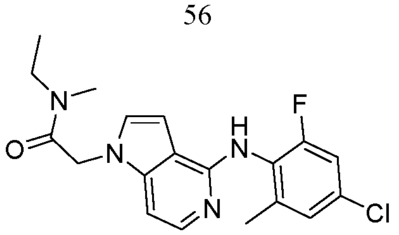

57

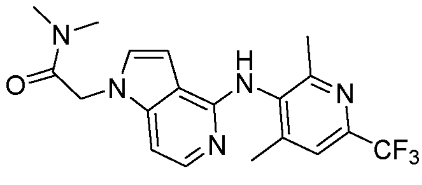

58

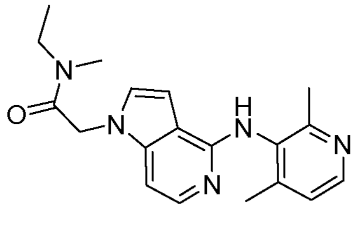

59

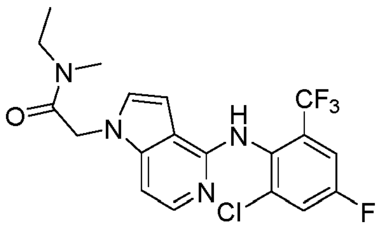

60

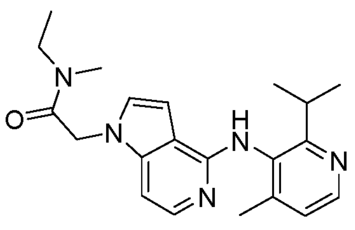

61

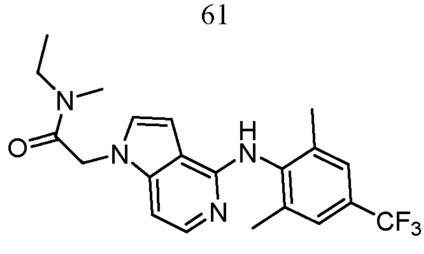

62

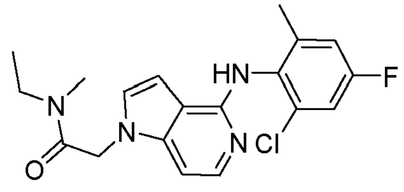

63

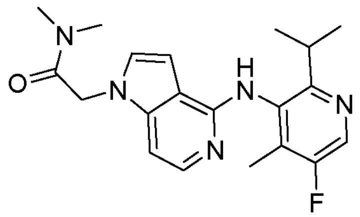

64

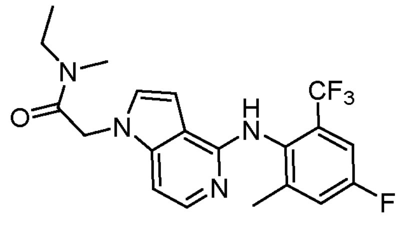

65

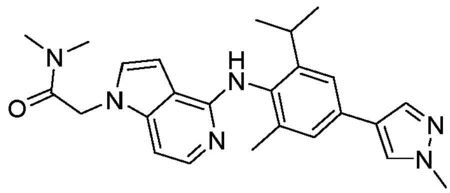

66

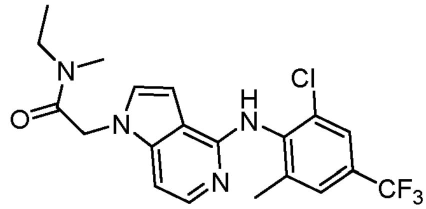

67

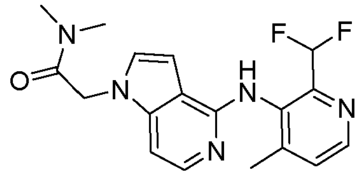

68

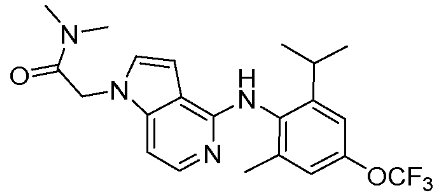

69

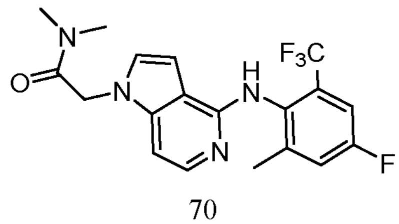

70

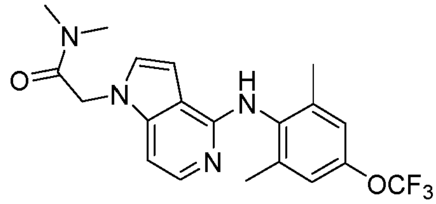

71

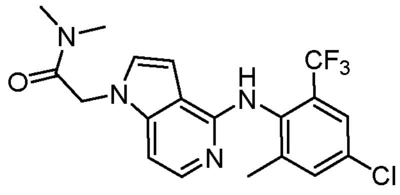

72

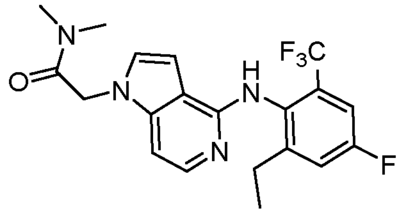

73

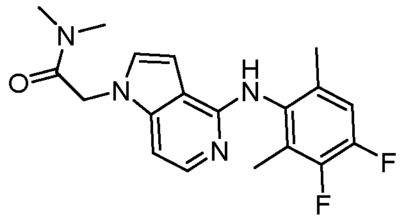

74

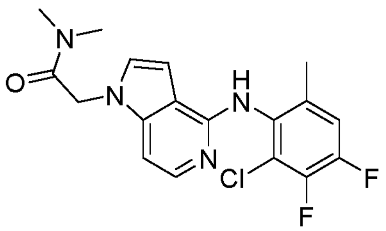

75

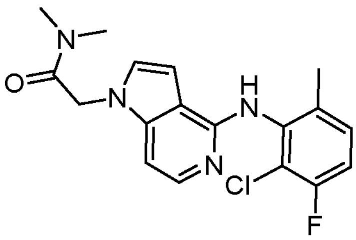

76

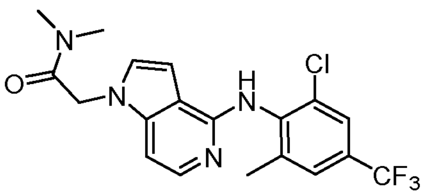

77

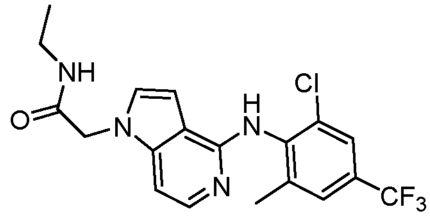

78

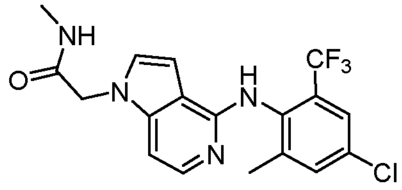

79

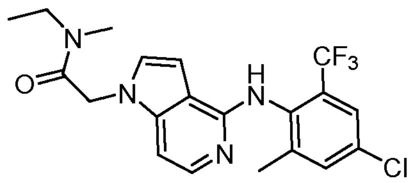

80

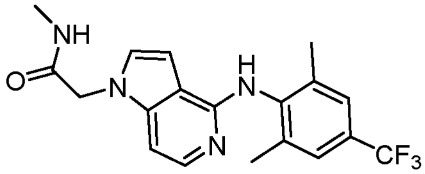

81

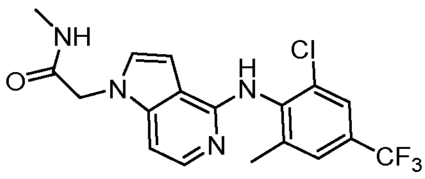

82

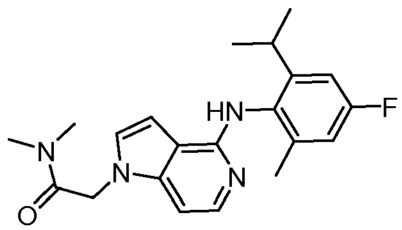

83

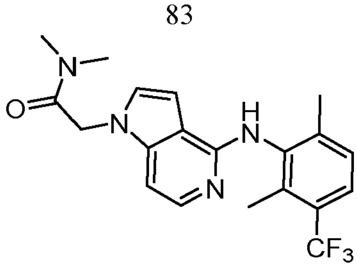

84

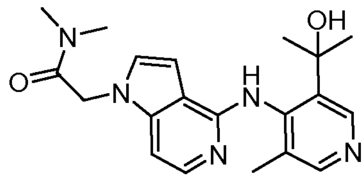

**Co. No. 85**

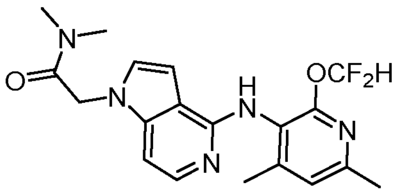

86

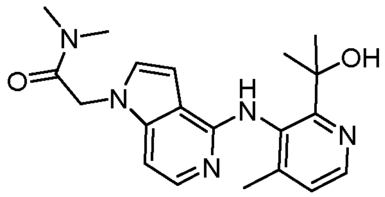

87

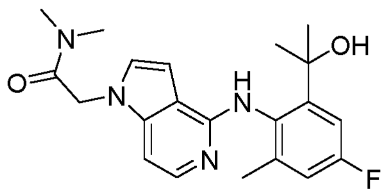

88

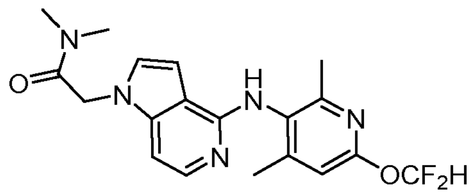

89

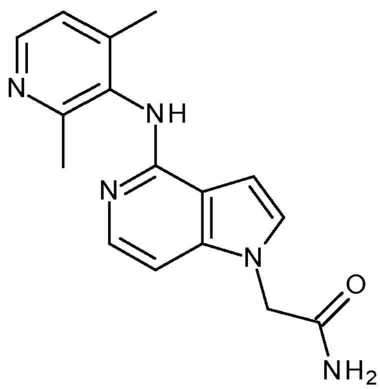

30

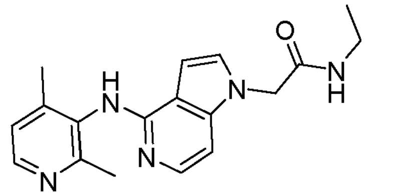

90

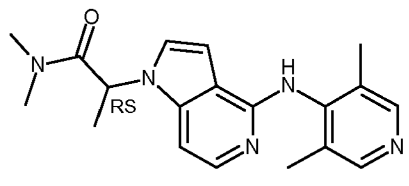

31

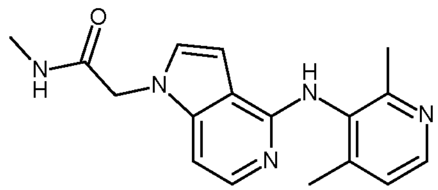

32

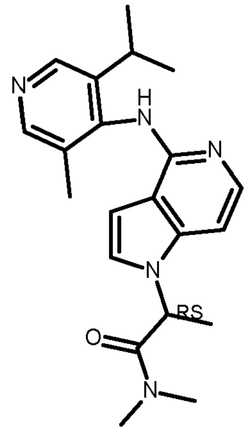

33

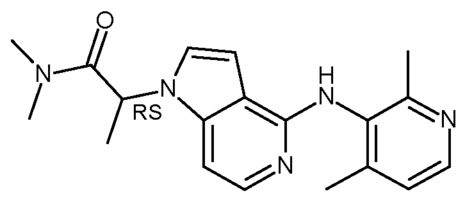

34

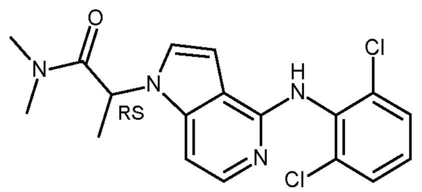

35

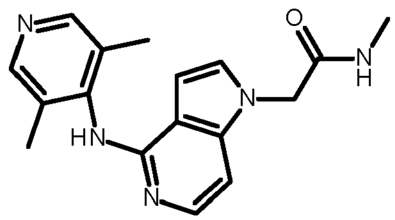

36

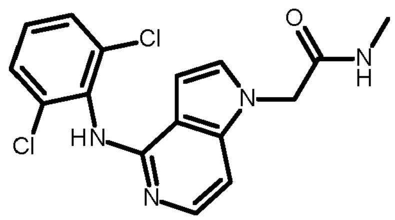

37

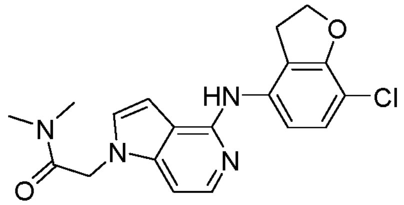

**91**

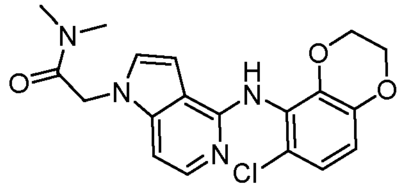

92

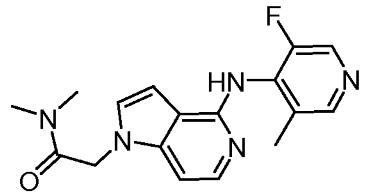

93

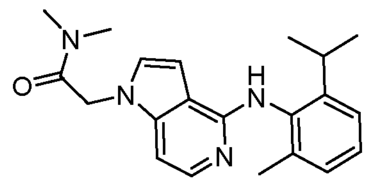

94

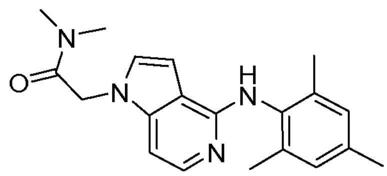

95

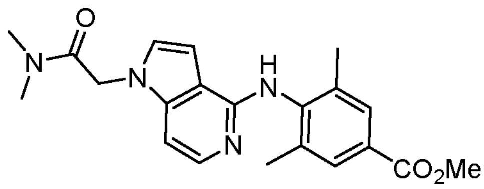

96

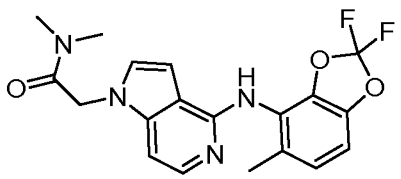

97

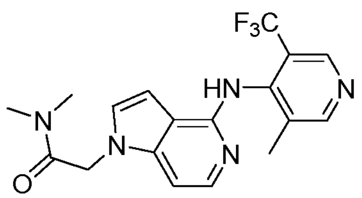

98

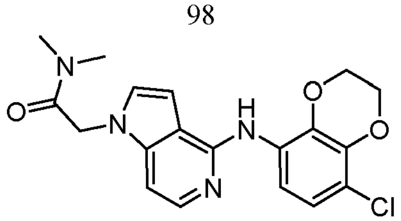

99

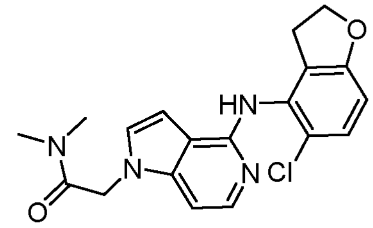

100

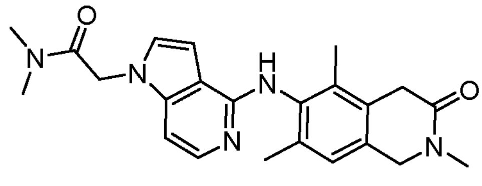

106

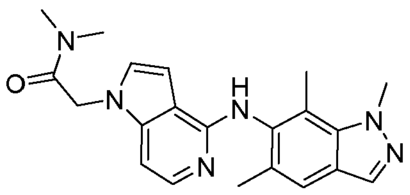

101

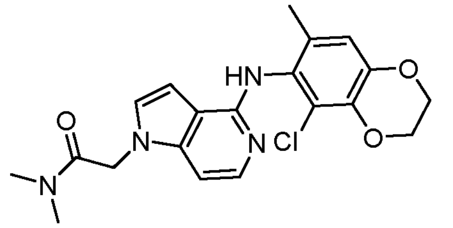

107

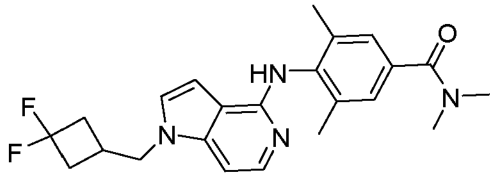

102

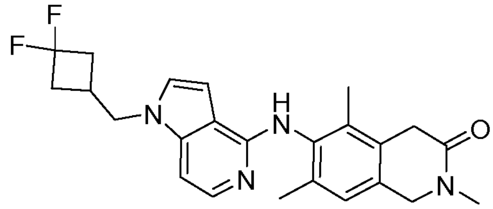

108

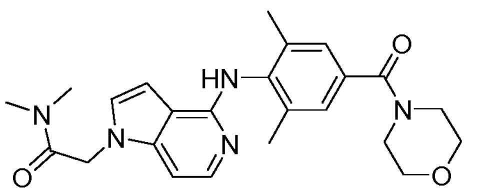

103

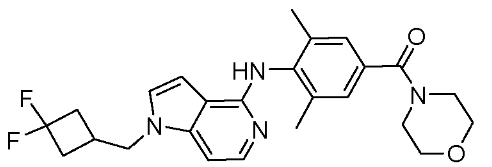

109

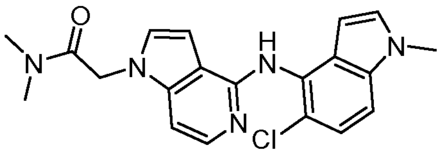

104

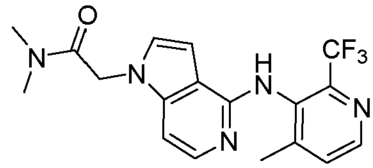

**110**

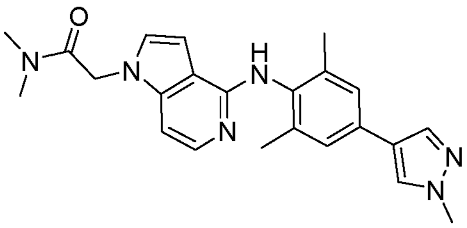

105

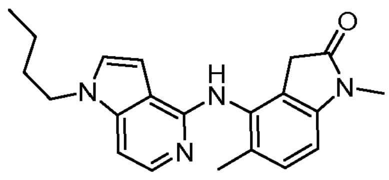

111

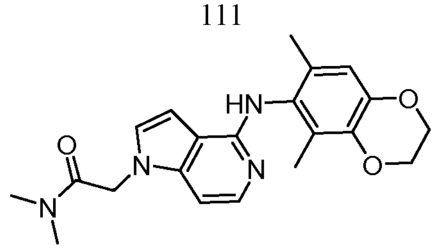

112

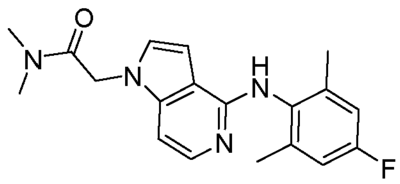

113

or

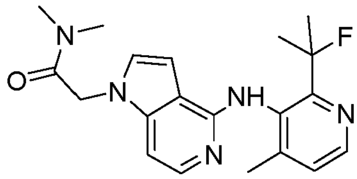

114

or a pharmaceutically acceptable salt or a solvate thereof.

**10.** The compound according to claim 9, wherein the compound is

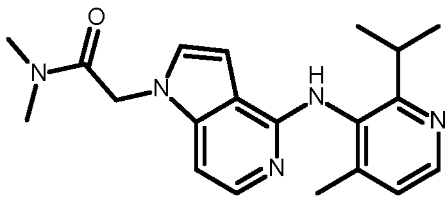

1,

or a pharmaceutically acceptable salt or a solvate thereof.

**11.** The compound according to claim 9, wherein the compound is

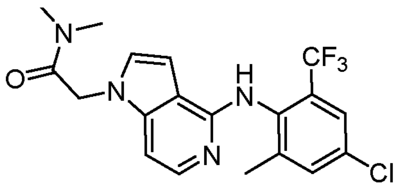

72,

or a pharmaceutically acceptable salt or a solvate thereof.

**12.** The compound according to claim 9, wherein the compound is

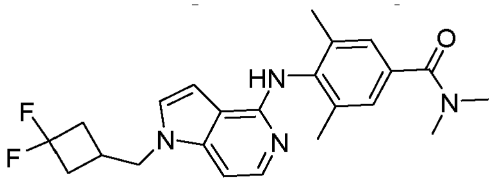

HCl
102

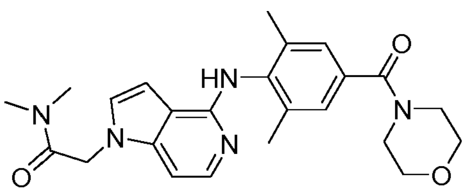

HCl
103

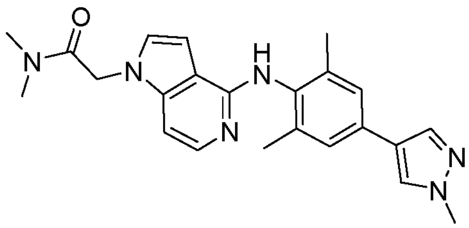

HCl
105,

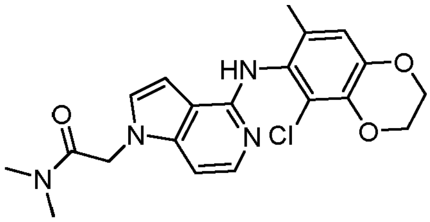

HCl
107 or

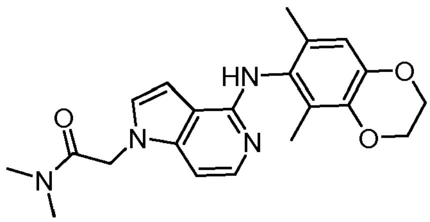

HCl
112.

**13.** A pharmaceutical composition comprising a prophylactically or a therapeutically effective amount of a compound according to any one of claims 1 to 12 and a pharmaceutically acceptable carrier.

**14.** A process for preparing a pharmaceutical composition comprising mixing a pharmaceutically acceptable carrier with a prophylactically or a therapeutically effective amount of a compound according to any one of claims 1 to 12.

**15.** A compound as defined in any one of claims 1 to 12, or the pharmaceutical composition as defined in claim 13, for use as a medicament.

**16.** A compound as defined in any one of claims 1 to 12, or the pharmaceutical composition as defined in claim 13, for use in the treatment or prevention of Alzheimer's disease, progressive supranuclear palsy, Down's syndrome, frontotemporal lobe dementia, frontotemporal dementia with Parkinsonism-17, Pick's disease, corticobasal degeneration, agryophilic grain disease,amyotrophic lateral sclerosis, or frontotemporal lobe dementia caused by C9ORF72 mutations.

## Patentansprüche

**1.** Verbindung der Formel (I)

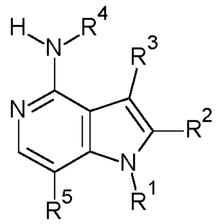

(I),

oder ein Tautomer oder eine stereoisomere Form davon, wobei

$R^1$ für $-C_{1-6}$-Alkyl-C (O) $-NR^xR^y$ steht, wobei $R^x$ und $R^y$ jeweils unabhängig aus der Gruppe bestehend aus Wasserstoff und $C_{1-3}$-Alkyl ausgewählt sind; oder $R^x$ und $R^y$ zusammen mit dem Stickstoffatom, an das sie gebunden sind, einen Heterocyclylring aus der Gruppe bestehend aus Azetidinyl, Pyrrolidinyl, Piperidinyl, Piperazinyl und Morpholinyl bilden;

$R^2$, $R^3$ und $R^5$ jeweils unabhängig aus der Gruppe bestehend aus Wasserstoff, Halogen und $C_{1-3}$-Alkyl ausgewählt sind; $R^4$ für einen einwertigen Rest aus der Gruppe bestehend aus (a), (b), (c), (d), (e) und (f) steht:

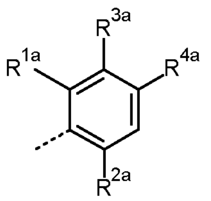

(a)

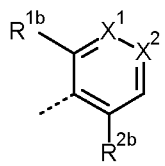

(b)

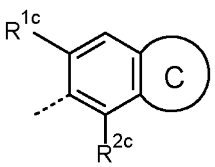

(c)

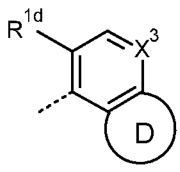

(d)

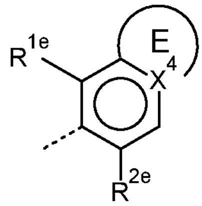

(e)

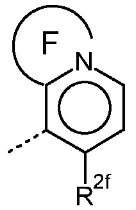

(f)

wobei

$R^{1a}$, $R^{2a}$, $R^{1b}$ und $R^{2b}$ jeweils unabhängig aus der Gruppe bestehend aus Halogen, $C_{1-3}$-Alkyl, Monohalogen-$C_{1-3}$-alkyl, Polyhalogen-$C_{1-3}$-alkyl, $C_{1-3}$-Alkyloxy, Monohalogen-$C_{1-3}$-alkyloxy, Polyhalogen-$C_{1-3}$-alkyloxy und $C_{3-6}$-Cycloalkyl ausgewählt sind;

$R^{3a}$ aus der Gruppe bestehend aus Wasserstoff, Halogen, - C(O)-O$C_{1-3}$-Alkyl, -C(O)-NR'R", -N(R''')-C(O)-$C_{1-3}$-Alkyl ausgewählt ist;

$R^{4a}$ aus der Gruppe bestehend aus Wasserstoff, Halogen, - CN, $C_{1-3}$-Alkyl, Monohalogen-$C_{1-3}$-alkyl, Polyhalogen-$C_{1-3}$-alkyl, $C_{1-3}$-Alkyloxy, Monohalogen-$C_{1-3}$-alkyloxy, Polyhalogen-$C_{1-3}$-alkyloxy, -C (O) -O$C_{1-3}$-Alkyl, -C(O)-NR'R", -N(R''')-C(O)-$C_{1-3}$-Alkyl und Het ausgewählt ist;

mit der Maßgabe, dass $R^{3a}$ und $R^{4a}$ nicht gleichzeitig für -C (O) -O$C_{1-3}$-Alkyl, -C (O) -NR'R" oder -N(R''') -C(O)-$C_{1-3}$-Alkyl stehen;

R' und R" jeweils unabhängig aus der Gruppe bestehend aus Wasserstoff und $C_{1-3}$-Alkyl ausgewählt sind; oder R' und R" zusammen mit dem Stickstoffatom, an das sie gebunden sind, einen Heterocyclylring aus der Gruppe bestehend aus Azetidinyl, Pyrrolidinyl, Piperidinyl, Piperazinyl und Morpholinyl bilden;

R''' aus der Gruppe bestehend aus Wasserstoff und $C_{1-3}$-Alkyl ausgewählt ist;

Het für Pyrazolyl oder Imidazolyl, das gegebenenfalls durch einen oder mehrere unabhängig ausgewählte $C_{1-3}$-Alkylsubstituenten substituiert ist, steht;

$X^1$ und $X^2$ jeweils unabhängig aus N und CH ausgewählt sind, mit der Maßgabe, dass mindestens eines von $X^1$ oder $X^2$ für N steht;

$R^{1c}$, $R^{2c}$, $R^{1d}$, $R^{1e}$, $R^{2e}$ und $R^{2f}$ jeweils unabhängig aus der Gruppe bestehend aus Halogen, $C_{1-3}$-Alkyl, Monohalogen-$C_{1-3}$-alkyl, Polyhalogen-$C_{1-3}$-alkyl, $C_{1-3}$-Alkyloxy, Monohalogen-$C_{1-3}$-alkyl, Polyhalogen-$C_{1-3}$-alkyl und

$C_{3-6}$-Cycloalkyl ausgewählt sind;
$X^3$ für CH oder N steht;
$X^4$ für C oder N steht;
und jeder der durch

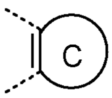 , 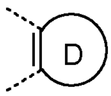 , 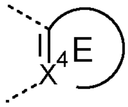 und 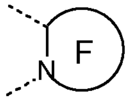

wiedergegebenen Ringe

(i) einen 5- oder 6-gliedrigen ungesättigten Heterocyclus, der ein, zwei oder drei Heteroatome, die jeweils unabhängig aus Stickstoff und Sauerstoff ausgewählt sind, aufweist und gegebenenfalls durch einen oder mehrere Substituenten, die jeweils unabhängig aus Halogen, $C_{1-3}$-Alkyl und Oxo ausgewählt sind, substituiert ist; oder
(ii) einen 5- oder 6-gliedrigen aromatischen Heterocyclus, der ein, zwei oder drei Heteroatome, die jeweils unabhängig aus Stickstoff, Sauerstoff und Schwefel ausgewählt sind, aufweist und gegebenenfalls durch einen oder mehrere Substituenten, die jeweils unabhängig aus Halogen, -CN, $C_{1-3}$-Alkyl, Monohalogen-$C_{1-3}$-alkyl, und Polyhalogen-$C_{1-3}$alkyl ausgewählt sind, substituiert ist;

bilden;
oder ein pharmazeutisch unbedenkliches Additionssalz oder ein Solvat davon.

**2.** Verbindung nach Anspruch 1, wobei

$R^1$ für -$C_{1-3}$-Alkyl-C(O) -$NR^xR^y$ steht, wobei
$R^x$ und $R^y$ jeweils unabhängig aus der Gruppe bestehend aus Wasserstoff und $C_{1-3}$-Alkyl ausgewählt sind; oder $R^x$ und $R^y$ zusammen mit dem Stickstoffatom, an das sie gebunden sind, einen Heterocyclylring aus der Gruppe bestehend aus Azetidinyl, Pyrrolidinyl, Piperidinyl, Piperazinyl und Morpholinyl bilden;
$R^2$, $R^3$ und $R^5$ jeweils unabhängig aus der Gruppe bestehend aus Wasserstoff, Halogen und $C_{1-3}$-Alkyl ausgewählt sind;
$R^4$ für einen einwertigen Rest aus der Gruppe bestehend aus (a), (b), (d) und (f) steht, wobei
$R^{1a}$, $R^{2a}$, $R^{1b}$ und $R^{2b}$ jeweils unabhängig aus der Gruppe bestehend aus Halogen, $C_{1-3}$-Alkyl, Monohalogen-$C_{1-3}$-alkyl, Polyhalogen-$C_{1-3}$-alkyl, $C_{1-3}$-Alkyloxy, Monohalogen-$C_{1-3}$-alkyloxy, Polyhalogen-$C_{1-3}$-alkyloxy und $C_{3-6}$-Cycloalkyl ausgewählt sind;
$R^{3a}$ für Wasserstoff steht;
$R^{4a}$ aus der Gruppe bestehend aus Wasserstoff, Halogen, - CN, $C_{1-3}$-Alkyl, Monohalogen-$C_{1-3}$-alkyl, Polyhalogen-$C_{1-3}$-alkyl, $C_{1-3}$-Alkyloxy, Monohalogen-$C_{1-3}$-alkyloxy und Polyhalogen-$C_{1-3}$-alkyloxy ausgewählt ist;
$X^1$ und $X^2$ jeweils unabhängig aus N und CH ausgewählt sind, mit der Maßgabe, dass mindestens eines von $X^1$ oder $X^2$ für N steht;
$R^{1d}$ und $R^{2f}$ jeweils unabhängig aus $C_{1-3}$-Alkyl ausgewählt sind;
$X^3$ für CH steht;
und jeder der durch

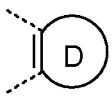 und 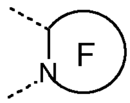

wiedergegebenen Ringe

(i) einen 5- oder 6-gliedrigen ungesättigten Heterocyclus, der ein oder zwei Heteroatome, die jeweils unabhängig aus Stickstoff und Sauerstoff ausgewählt sind, aufweist und gegebenenfalls durch einen oder mehrere Substituenten, die jeweils unabhängig aus Halogen und $C_{1-3}$-Alkyl ausgewählt sind, substituiert ist; oder
(ii) einen 5- oder 6-gliedrigen aromatischen Heterocyclus, der ein, zwei oder drei Heteroatome, die jeweils unabhängig aus Stickstoff und Sauerstoff ausgewählt sind, aufweist und gegebenenfalls durch einen oder

mehrere Substituenten, die jeweils unabhängig aus $C_{1-3}$-Alkyl ausgewählt sind, substituiert ist;

bilden.

**3.** Verbindung nach Anspruch 1 oder 2, wobei

$R^1$ für -$C_{1-3}$-Alkyl-C (O) -$NR^xR^y$ steht, wobei
$R^x$ und $R^y$ jeweils unabhängig aus der Gruppe bestehend aus Wasserstoff und $C_{1-3}$-Alkyl ausgewählt sind; oder $R^x$ und $R^y$ zusammen mit dem Stickstoffatom, an das sie gebunden sind, einen Heterocyclylring aus der Gruppe bestehend aus Azetidinyl, Pyrrolidinyl, Piperidinyl, Piperazinyl und Morpholinyl bilden;
$R^2$, $R^3$ und $R^5$ jeweils unabhängig aus der Gruppe bestehend aus Wasserstoff, Halogen und $C_{1-3}$-Alkyl ausgewählt sind; $R^4$ für einen einwertigen Rest aus der Gruppe bestehend aus (a), (b), (d) und (f) steht, wobei
$R^{1a}$, $R^{2a}$, $R^{1b}$ und $R^{2b}$ jeweils unabhängig aus der Gruppe bestehend aus Halogen, $C_{1-3}$-Alkyl, Polyhalogen-$C_{1-3}$-alkyl, $C_{1-3}$-Alkyloxy und Polyhalogen-$C_{1-3}$-alkyloxy ausgewählt sind;
$R^{3a}$ für Wasserstoff steht;
$R^{4a}$ aus der Gruppe bestehend aus Wasserstoff, Halogen, - CN, $C_{1-3}$-Alkyl, Polyhalogen-$C_{1-3}$-alkyl, $C_{1-3}$-Alkyloxy und Polyhalogen-$C_{1-3}$-alkyloxy ausgewählt ist;
$X^1$ und $X^2$ jeweils unabhängig aus N und CH ausgewählt sind, mit der Maßgabe, dass mindestens eines von $X^1$ oder $X^2$ für N steht;
$R^{1d}$ und $R^{2f}$ jeweils unabhängig aus $C_{1-3}$-Alkyl ausgewählt sind;
$X^3$ für CH steht;
und jeder der durch

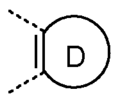 und 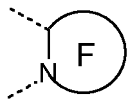

wiedergegebenen Ringe

(i) einen 5- oder 6-gliedrigen ungesättigten Heterocyclus, der ein oder zwei Stickstoffatome aufweist und gegebenenfalls durch einen oder mehrere Substituenten, die jeweils unabhängig aus Halogen ausgewählt sind, substituiert ist und
(ii) einen 5- oder 6-gliedrigen aromatischen Heterocyclus, der ein oder zwei Stickstoffatome aufweist und gegebenenfalls durch einen oder mehrere Substituenten, die jeweils unabhängig aus $C_{1-3}$-Alkyl ausgewählt sind, substituiert ist;

bilden.

**4.** Verbindung nach einem der Ansprüche 1 bis 3, wobei $R^1$ für -$C_{1-3}$-Alkyl-C(O)-$NR^xR^y$ steht, wobei -$NR^xR^y$ aus der Gruppe bestehend aus -$NH_2$, -$NHCH_3$, -$NH(CH_3)_2$,-$N(CH_3)(CH_2CH_3)$, Azetidin-1-yl und Pyrrolidin-1-yl ausgewählt ist.

**5.** Verbindung nach einem der Ansprüche 1 bis 4, wobei $R^2$, $R^3$ und $R^5$ jeweils unabhängig aus Wasserstoff und Methyl ausgewählt sind.

**6.** Verbindung nach einem der Ansprüche 1 bis 5, wobei $R^{1a}$, $R^{2a}$, $R^{1b}$ und $R^{2b}$ jeweils unabhängig aus der Gruppe bestehend aus Fluor, Chlor, Methyl, Isopropyl, $CF_3$, -$OCH_3$ und -$OCF_3$ ausgewählt sind.

**7.** Verbindung nach einem der Ansprüche 1 bis 6, wobei $X^1$ für N steht und $X^2$ für CH steht oder $X^1$ für CH steht und $X^2$ für N steht oder $X^1$ und $X^2$ beide für N stehen.

**8.** Verbindung nach einem der Ansprüche 1 bis 7, wobei $R^{1d}$ und $R^{2f}$ jeweils unabhängig für Methyl oder Isopropyl stehen.

**9.** Verbindung nach Anspruch 1, wobei es sich bei der Verbindung um

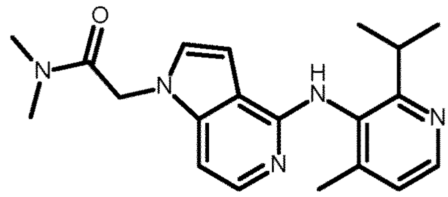

1

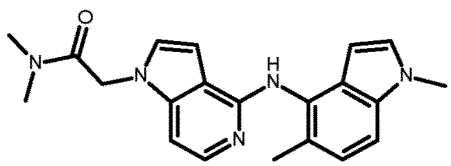

2

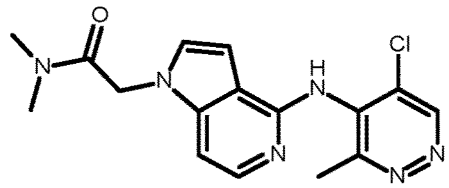

3

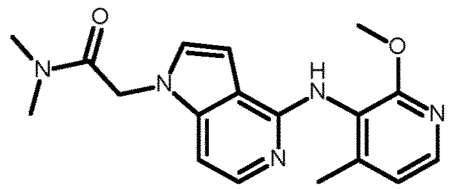

4

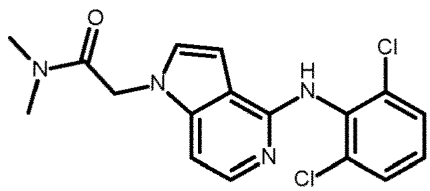

5

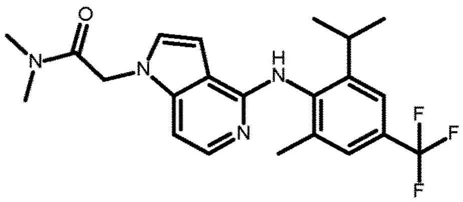

6

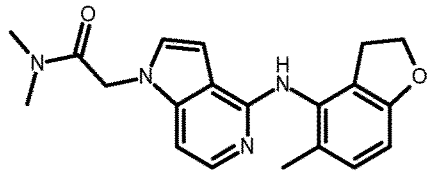

7

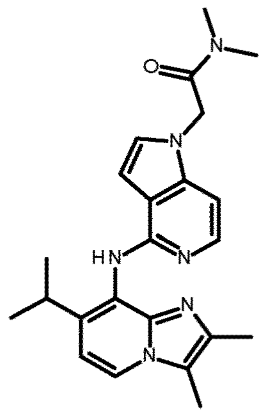

8

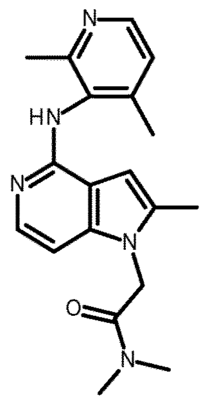

9

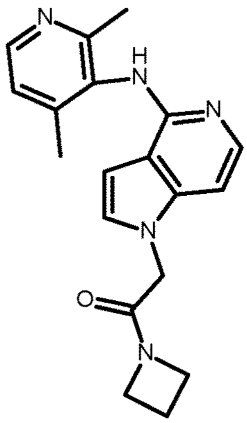

10

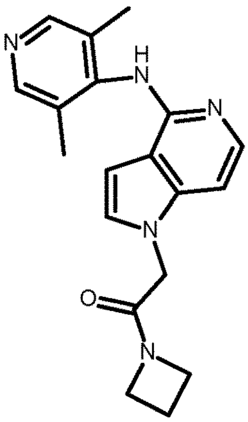

11

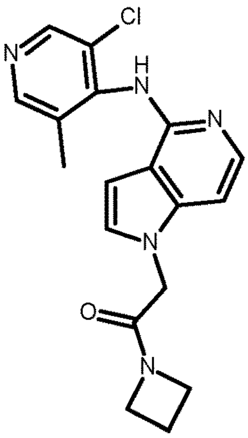

12

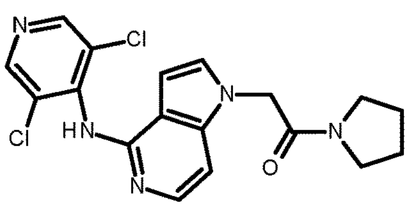

13

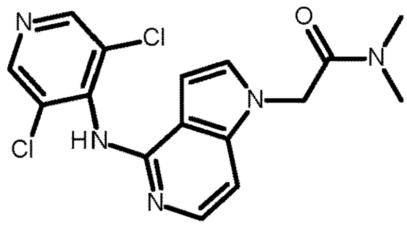

14

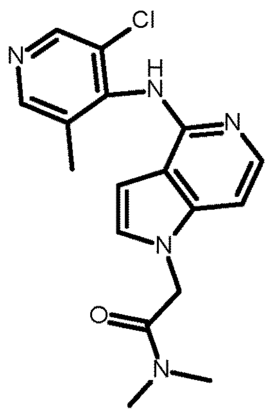

15

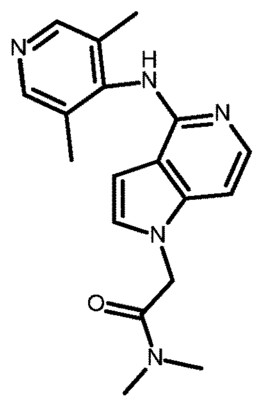

16

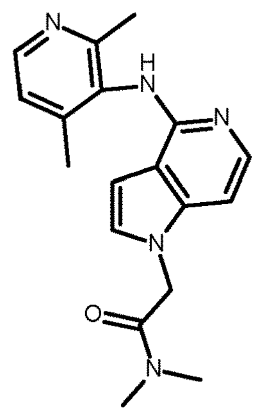

17

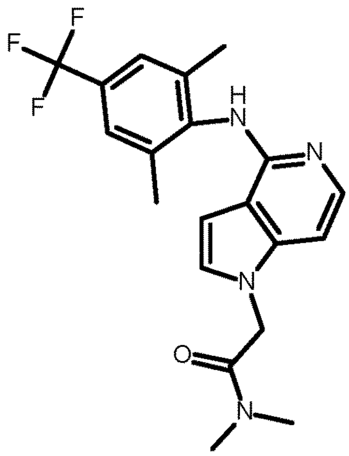

18

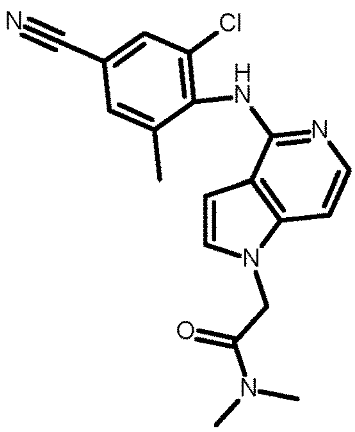

19

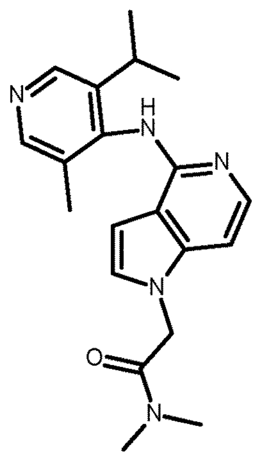

20

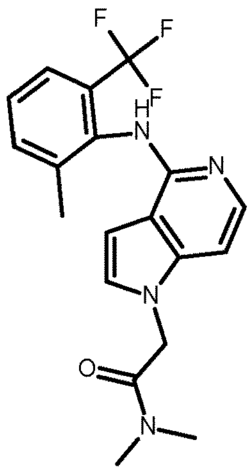

21

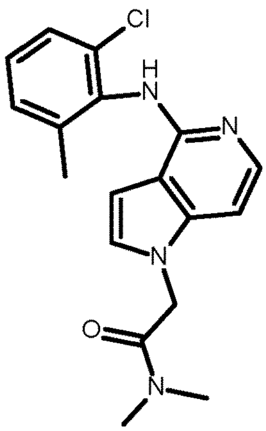

22

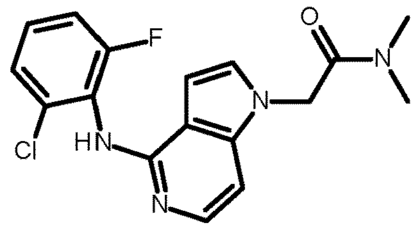

23

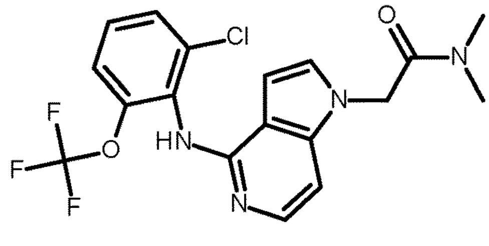

24

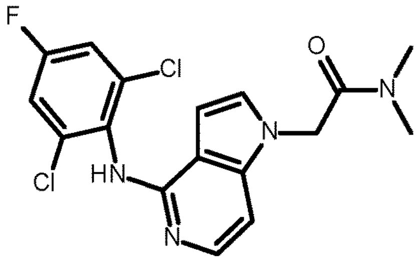

25

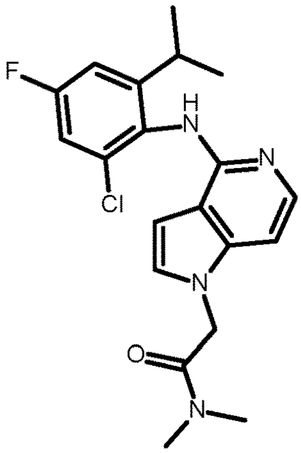

26

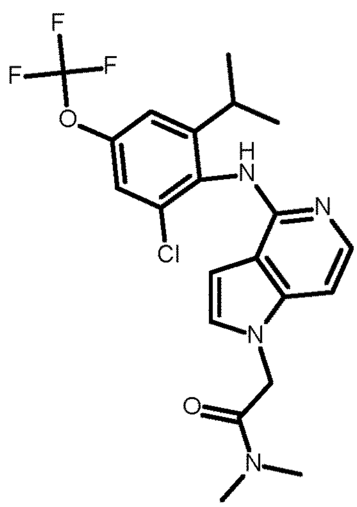

27

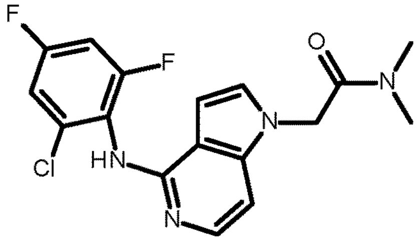

28

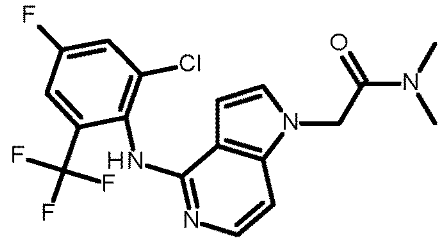

29

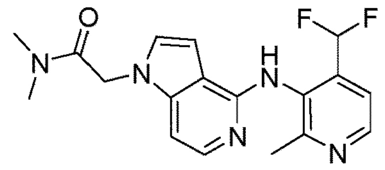

38

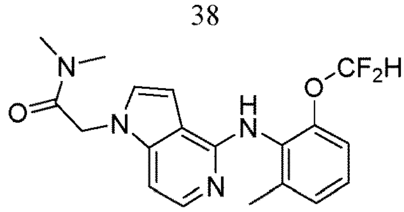

39

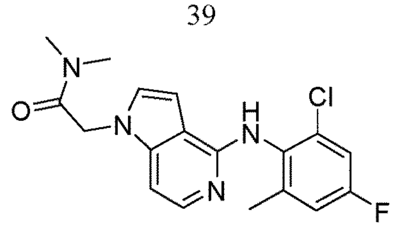

40

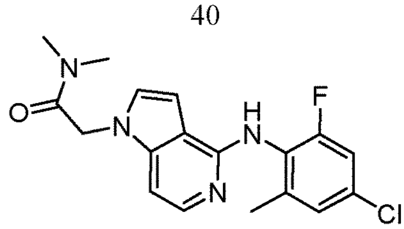

41

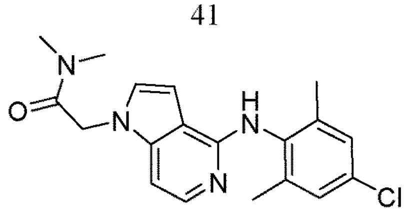

42

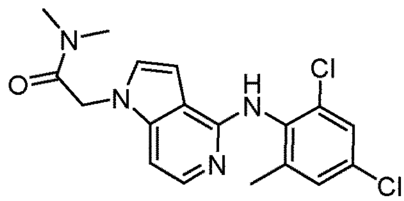

43

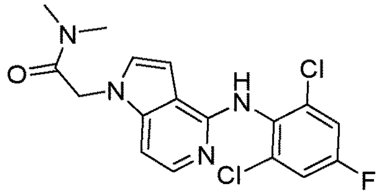

44

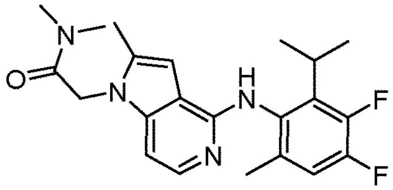

45

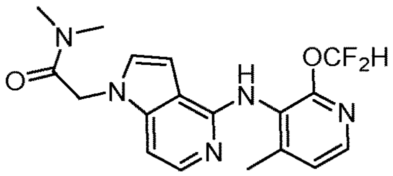

46

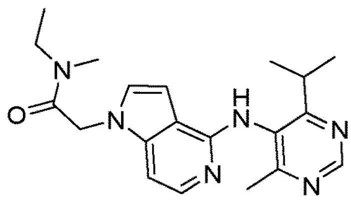

47

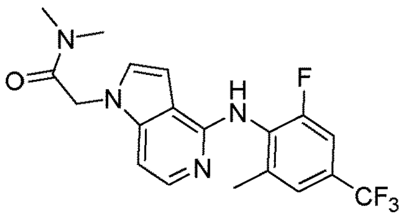

48

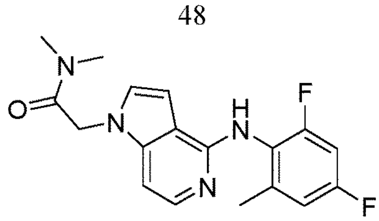

49

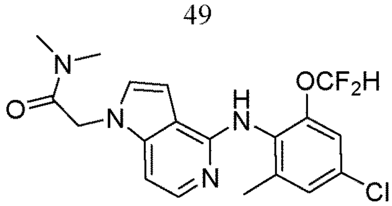

50

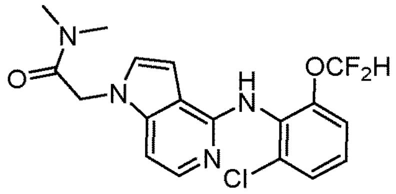

51

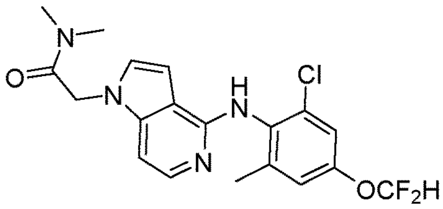

52

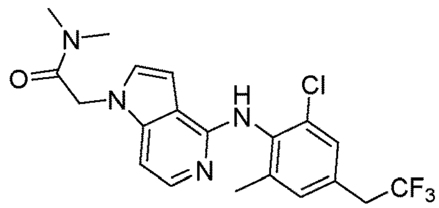

53

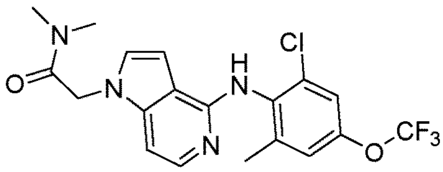

54

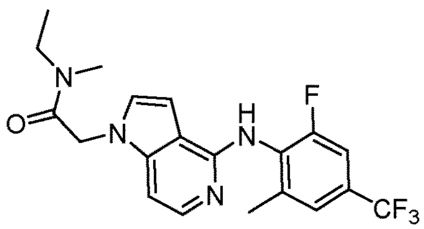

55

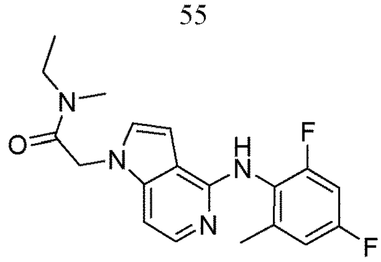

56

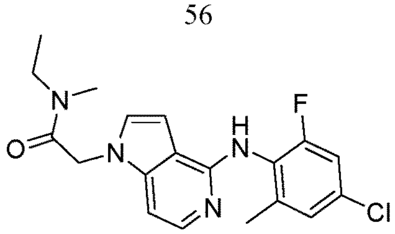

57

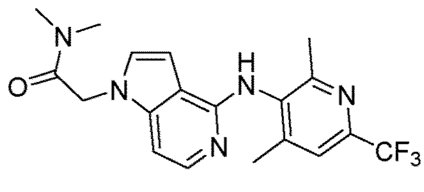

58

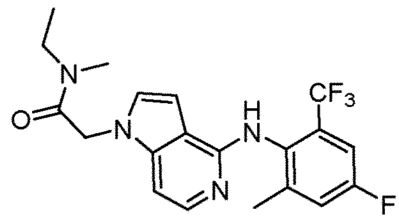

65

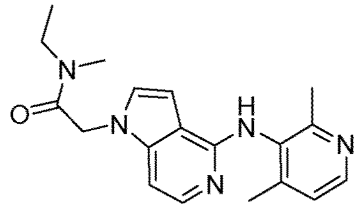

59

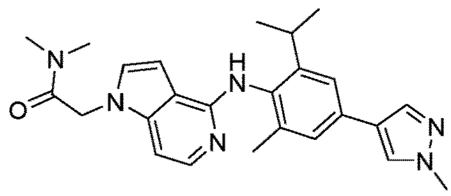

66

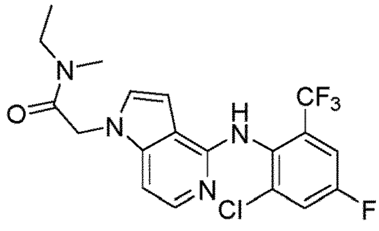

60

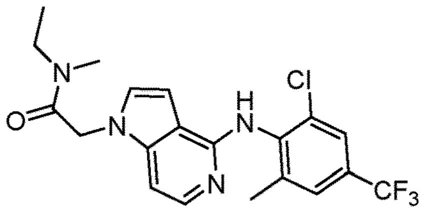

67

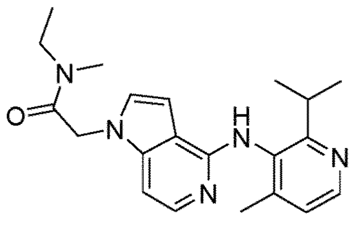

61

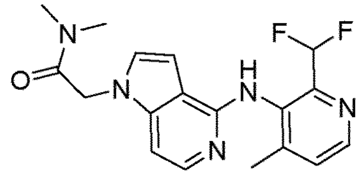

68

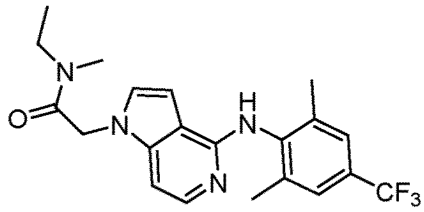

62

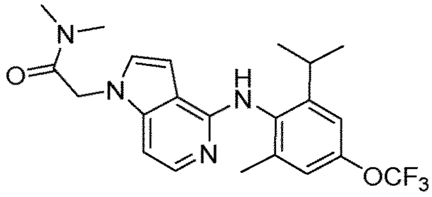

69

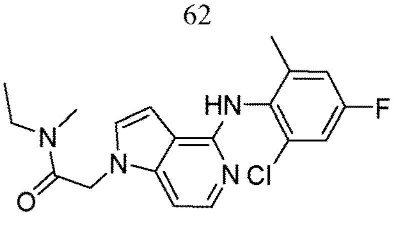

63

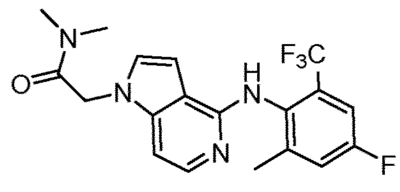

70

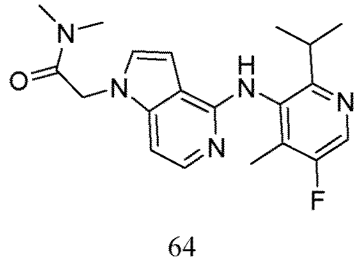

64

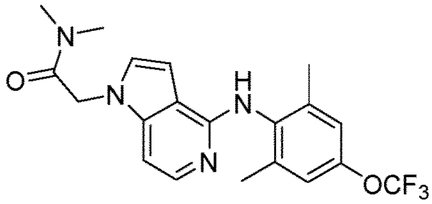

71

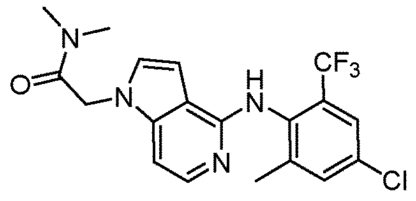

72

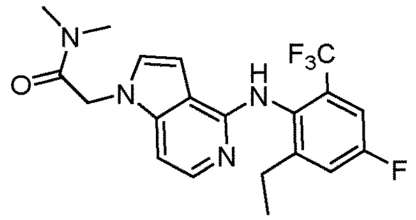

73

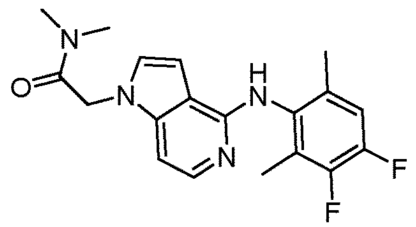

74

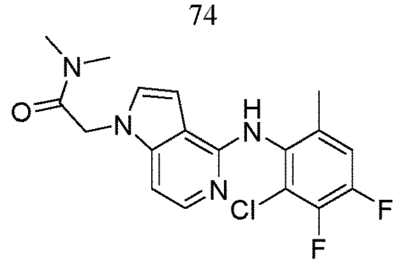

75

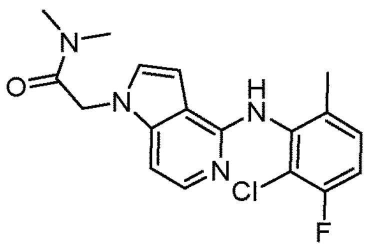

76

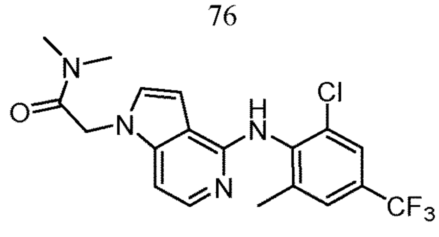

77

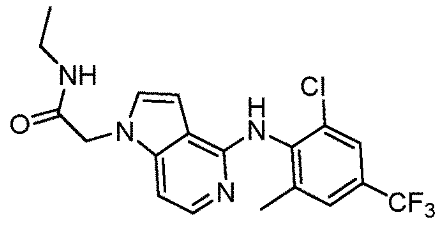

78

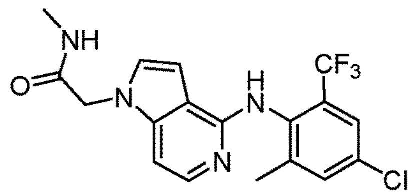

79

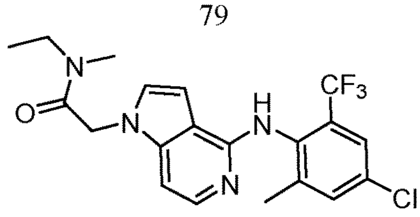

80

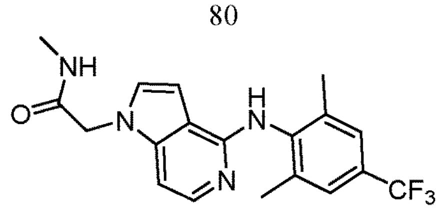

81

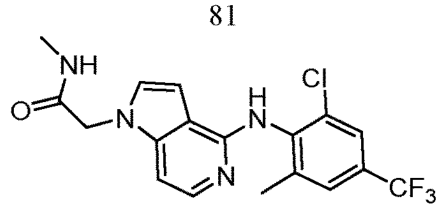

82

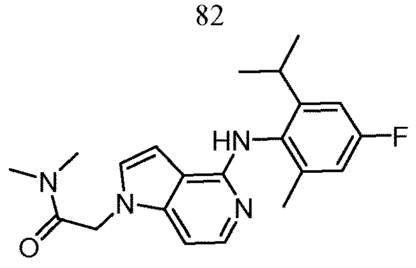

83

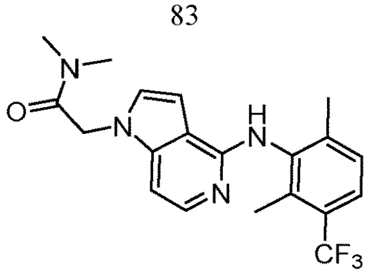

84

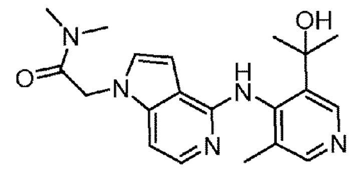

**Verb. Nr. 85**

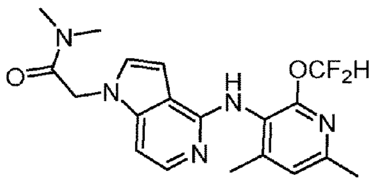

86

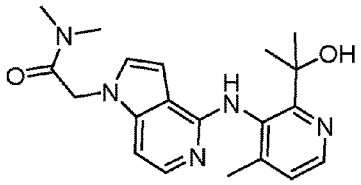

87

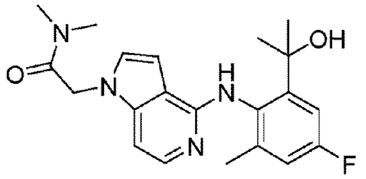

88

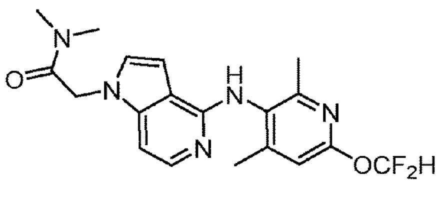

89

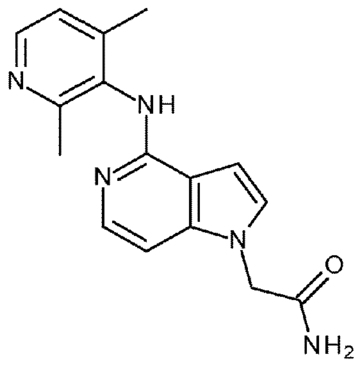

30

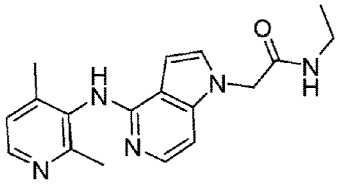

90

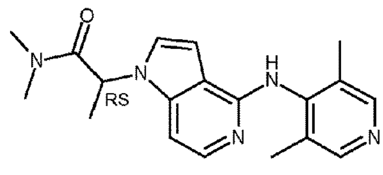

31

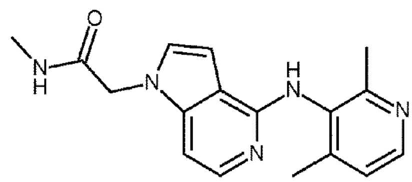

32

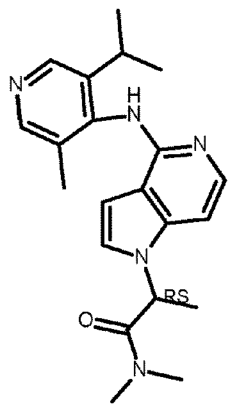

33

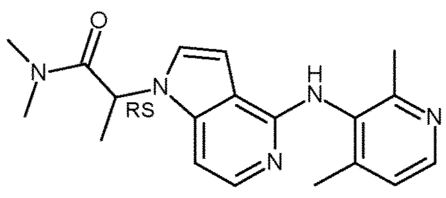

34

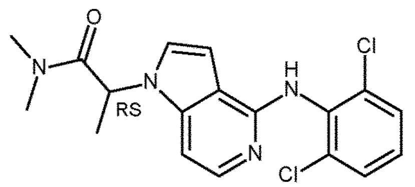

35

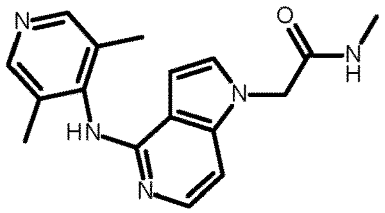

36

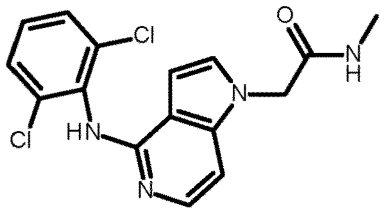

37

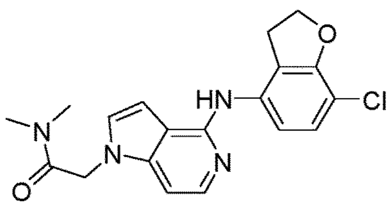

**91**

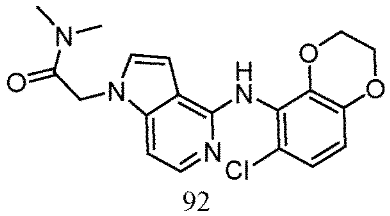

92

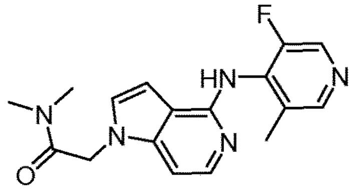

93

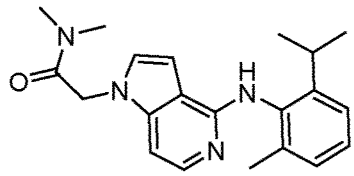

94

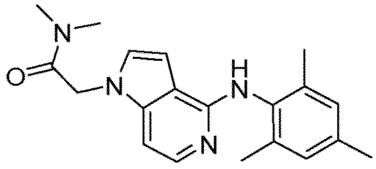

95

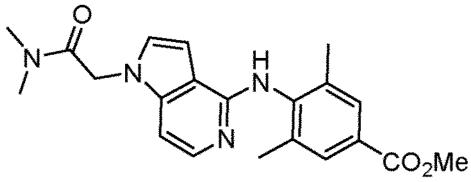

96

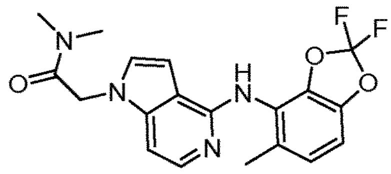

97

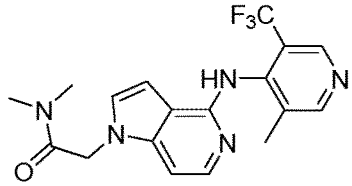

98

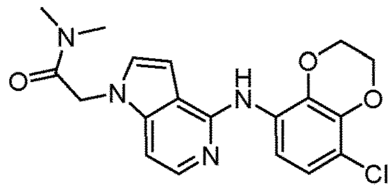

99

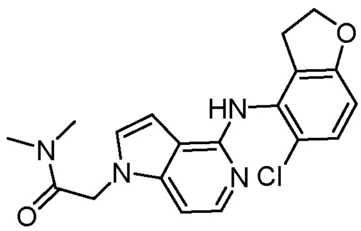

100

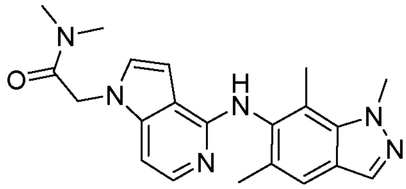

101

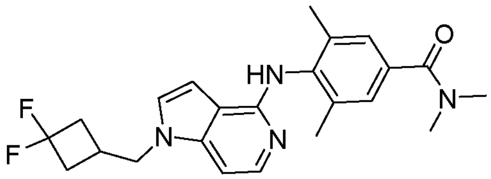

102

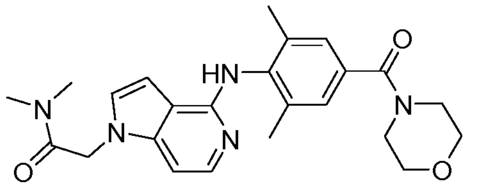

103

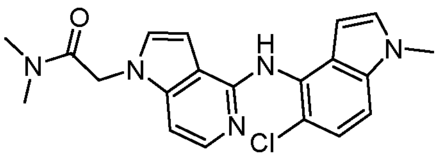

104

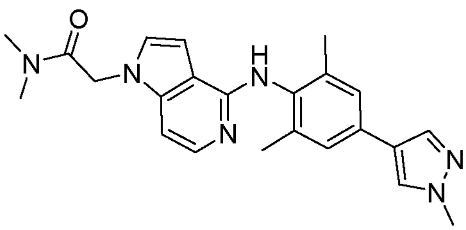

105

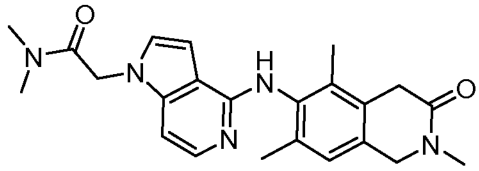

106

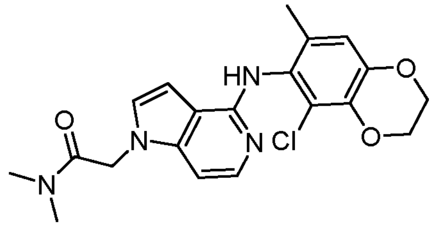

107

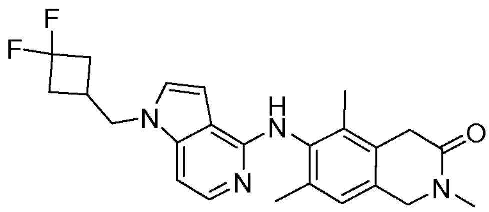

108

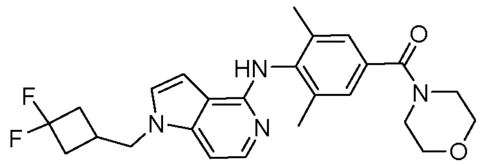

109

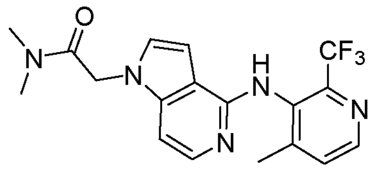

**110**

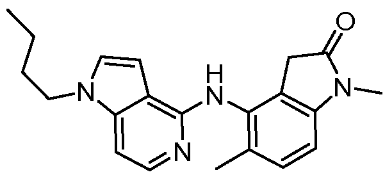

111

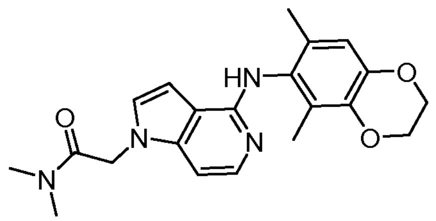

112

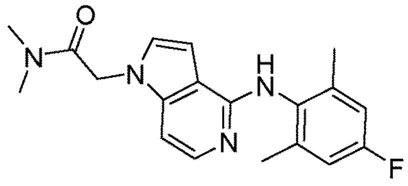

113

oder

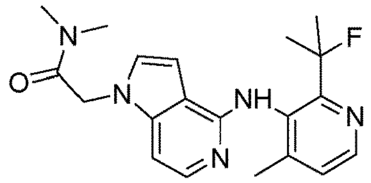

114

handelt, oder ein pharmazeutisch unbedenkliches Salz oder ein Solvat davon.

**10.** Verbindung nach Anspruch 9, wobei es sich bei der Verbindung um

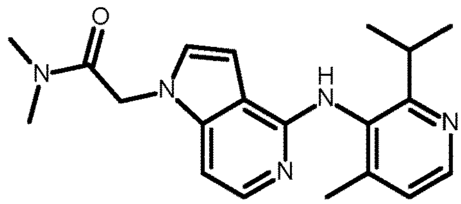

1

handelt, oder ein pharmazeutisch unbedenkliches Salz oder ein Solvat davon.

**11.** Verbindung nach Anspruch 9, wobei es sich bei der Verbindung um

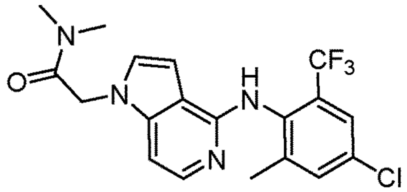

72,

handelt, oder ein pharmazeutisch unbedenkliches Salz oder ein Solvat davon.

**12.** Verbindung nach Anspruch 9, wobei es sich bei der Verbindung um

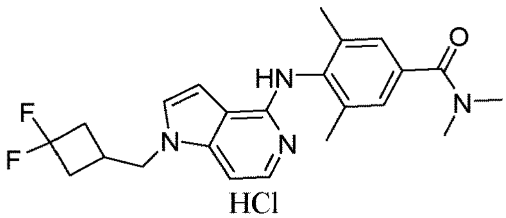

HCl

102

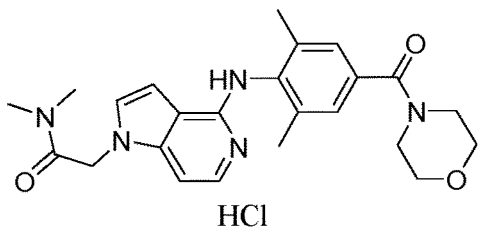

HCl

103

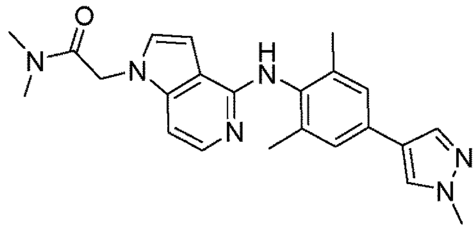

HCl
105

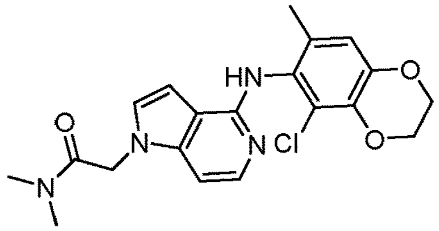

HCl
107 oder

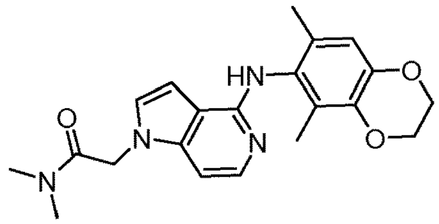

HCl
112.

handelt.

13. Pharmazeutische Zusammensetzung, umfassend eine prophylaktisch oder therapeutisch wirksame Menge einer Verbindung nach einem der Ansprüche 1 bis 12 und einen pharmazeutisch unbedenklichen Träger.

14. Verfahren zur Herstellung einer pharmazeutischen Zusammensetzung, bei dem man einen pharmazeutisch unbedenklichen Träger mit einer prophylaktisch oder therapeutisch wirksamen Menge einer Verbindung nach einem der Ansprüche 1 bis 12 mischt.

15. Verbindung gemäß einem der Ansprüche 1 bis 12 oder pharmazeutische Zusammensetzung gemäß Anspruch 13 zur Verwendung als Medikament.

16. Verbindung gemäß einem der Ansprüche 1 bis 12 oder pharmazeutische Zusammensetzung gemäß Anspruch 13 zur Verwendung bei der Behandlung oder Prävention von Alzheimer-Krankheit, progressiver supranukleärer Lähmung, Down-Syndrom, Frontotemporallappendemenz, frontotemporaler Demenz mit Parkinsonismus-17, Pick-Krankheit, kortikobasaler Degeneration, Silberkornkrankheit, amyotropher Lateralsklerose oder durch C9ORF72-Mutationen verursachter Frontotemporallappendemenz.

**Revendications**

1. Composé de formule (I)

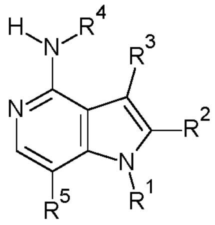

(I),

ou forme tautomère ou forme stéréoisomérique correspondante,

$R^1$ étant $-C_{1-6}$alkyl-C(O) -$NR^xR^y$,
$R^x$ et $R^y$ étant chacun indépendamment choisis dans le groupe constitué par hydrogène et $C_{1-3}$alkyle ;
ou $R^x$ et $R^y$ conjointement avec l'atome d'azote auquel ils sont fixés formant un cycle hétérocyclyle choisi dans le groupe constitué par azétidinyle, pyrrolidinyle, pipéridinyle, pipérazinyle et morpholinyle ;
$R^2$, $R^3$ et $R^5$ étant chacun indépendamment choisis dans le groupe constitué par hydrogène, halogéno et $C_{1-3}$alkyle ; $R^4$ étant un radical monovalent choisi dans le groupe constitué par (a), (b), (c), (d), (e) et (f) :

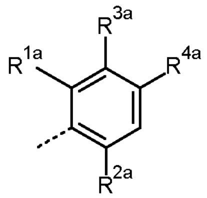

(a)

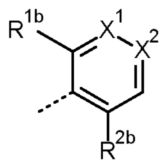

(b)

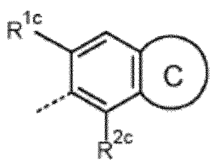

(c)

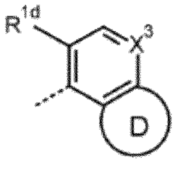

(d)

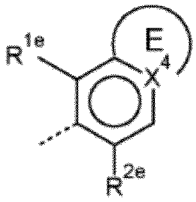

(e)

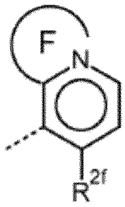

(f)

$R^{1a}$, $R^{2a}$, $R^{1b}$, et $R^{2b}$ étant chacun indépendamment choisis dans le groupe constitué par halogéno, $C_{1-3}$alkyle, monohalogéno$C_{1-3}$alkyle, polyhalogéno$C_{1-3}$alkyle, $C_{1-3}$alkyloxy, monohalogéno$C_{1-3}$alkyloxy, polyhalogéno$C_{1-3}$alkyloxy, et $C_{3-6}$cycloalkyle ;
$R^{3a}$ étant choisi dans le groupe constitué par hydrogène, halogéno, -C (O) -O$C_{1-3}$alkyle, -C(O)-NR'R", -N(R"') -C (O) - $C_{1-3}$alkyle ;
$R^{4a}$ étant choisi dans le groupe constitué par hydrogène, halogéno, -CN, $C_{1-3}$alkyle, monohalogéno$C_{1-3}$alkyle, polyhalogéno$C_{1-3}$alkyle, $C_{1-3}$alkyloxy, monohalogéno$C_{1-3}$alkyloxy, polyhalogéno$C_{1-3}$alkyloxy, -C (O) -O$C_{1-3}$alkyle,-C(O)-NR'R", -N(R‴)-C(O)-$C_{1-3}$alkyle, et Het ;
à la condition que $R^{3a}$ et $R^{4a}$ ne soient pas simultanément -C (O) -O$C_{1-3}$alkyle, -C(O)-NR'R", ou -N(R‴) -C (O) -$C_{1-3}$alkyle ;
R' et R" étant chacun indépendamment choisis dans le groupe constitué par hydrogène et $C_{1-3}$alkyle ;
ou R' et R" conjointement avec l'atome d'azote auquel ils sont fixés formant un cycle hétérocyclyle choisi dans le groupe constitué par azétidinyle, pyrrolidinyle, pipéridinyle, pipérazinyle et morpholinyle ;
R‴ étant choisi dans le groupe constitué par hydrogène et $C_{1-3}$alkyle ;
Het étant pyrazolyle ou imidazolyle, éventuellement substitué par un ou plusieurs substituants $C_{1-3}$alkyle indépendamment choisis ;
$X^1$ et $X^2$ étant chacun indépendamment choisis parmi N et CH, à la condition qu'au moins l'un parmi $X^1$ ou $X^2$ soit N ;
$R^{1c}$, $R^{2c}$, $R^{1d}$, $R^{1e}$, $R^{2e}$, et $R^{2f}$ étant chacun indépendamment choisis dans le groupe constitué par halogéno, $C_{1-3}$alkyle, monohalogéno$C_{1-3}$alkyle, polyhalogéno$C_{1-3}$alkyle, $C_{1-3}$alkyloxy, monohalogéno$C_{1-3}$alkyle, polyhalogéno$C_{1-3}$alkyle, et $C_{3-6}$cycloalkyle ;
$X^3$ représentant CH ou N ;
$X^4$ représentant C ou N ;

et chacun des cycles représentés par

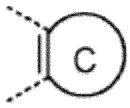 , 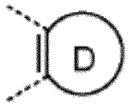 , 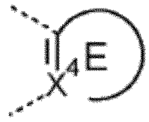 , et 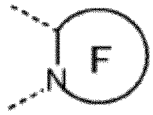 ,

formant

(i) un hétérocycle insaturé à 5 ou 6 chaînons possédant un, deux ou trois hétéroatomes chacun indépendamment choisis parmi azote et oxygène, et qui est éventuellement substitué par un ou plusieurs substituants, chacun indépendamment choisis parmi halogéno, $C_{1-3}$alkyle et oxo ; ou
(ii) un hétérocycle aromatique à 5 ou 6 chaînons possédant un, deux ou trois hétéroatomes chacun indépendamment choisis parmi azote, oxygène et soufre, et qui est éventuellement substitué par un ou plusieurs substituants, chacun indépendamment choisis parmi halogéno, -CN, $C_{1-3}$alkyle, monohalogéno$C_{1-3}$alkyle, et polyhalogéno$C_{1-3}$alkyle ;

ou sel d'addition pharmaceutiquement acceptable ou solvate correspondant.

**2.** Composé selon la revendication 1,

$R^1$ étant -$C_{1-3}$alkyl-C(O) -$NR^xR^y$,
$R^x$ et $R^y$ étant chacun indépendamment choisis dans le groupe constitué par hydrogène et $C_{1-3}$alkyle ;
ou $R^x$ et $R^y$ conjointement avec l'atome d'azote auquel ils sont fixés formant un cycle hétérocyclyle choisi dans le groupe constitué par azétidinyle, pyrrolidinyle, pipéridinyle, pipérazinyle et morpholinyle ;
$R^2$, $R^3$ et $R^5$ étant chacun indépendamment choisis dans le groupe constitué par hydrogène, halogéno et $C_{1-3}$alkyle ; $R^4$ étant un radical monovalent choisi dans le groupe constitué par (a), (b), (d) et (f),
$R^{1a}$, $R^{2a}$, $R^{1b}$, et $R^{2b}$ étant chacun indépendamment choisis dans le groupe constitué par halogéno, $C_{1-3}$alkyle, monohalogéno$C_{1-3}$alkyle, polyhalogéno$C_{1-3}$alkyle, $C_{1-3}$alkyloxy, monohalogéno$C_{1-3}$alkyloxy, polyhalogéno$C_{1-3}$alkyloxy, et $C_{3-6}$cycloalkyle ;
$R^{3a}$ étant hydrogène ;
$R^{4a}$ étant choisi dans le groupe constitué par hydrogène, halogéno, -CN, $C_{1-3}$alkyle, monohalogéno$C_{1-3}$alkyle, polyhalogéno$C_{1-3}$alkyle, $C_{1-3}$alkyloxy, monohalogéno$C_{1-3}$alkyloxy, et polyhalogéno$C_{1-3}$alkyloxy ;
$X^1$ et $X^2$ étant chacun indépendamment choisis parmi N et CH, à la condition qu'au moins l'un parmi $X^1$ ou $X^2$ soit N ;
$R^{1d}$ et $R^{2f}$ étant chacun indépendamment choisis parmi $C_{1-3}$alkyle ;
$X^3$ représentant CH ;
et chacun des cycles représentés par

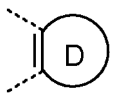 et 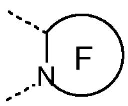 formant

(i) un hétérocycle insaturé à 5 ou 6 chaînons possédant un ou deux hétéroatomes chacun indépendamment choisis parmi azote et oxygène, et qui est éventuellement substitué par un ou plusieurs substituants, chacun indépendamment choisis parmi halogéno et $C_{1-3}$alkyle ; ou
(ii) un hétérocycle aromatique à 5 ou 6 chaînons possédant un, deux ou trois hétéroatomes chacun indépendamment choisis parmi azote et oxygène, et qui est éventuellement substitué par un ou plusieurs substituants, chacun indépendamment choisis parmi $C_{1-3}$alkyle.

**3.** Composé selon la revendication 1 ou 2,

$R^1$ étant -$C_{1-3}$alkyl-C(O) -$NR^xR^y$,
$R^x$ et $R^y$ étant chacun indépendamment choisis dans le groupe constitué par hydrogène et $C_{1-3}$alkyle ;
ou $R^x$ et $R^y$ conjointement avec l'atome d'azote auquel ils sont fixés formant un cycle hétérocyclyle choisi dans

le groupe constitué par azétidinyle, pyrrolidinyle, pipéridinyle, pipérazinyle et morpholinyle ;
$R^2$, $R^3$ et $R^5$ étant chacun indépendamment choisis dans le groupe constitué par hydrogène, halogéno et $C_{1-3}$alkyle ; $R^4$ étant un radical monovalent choisi dans le groupe constitué par (a), (b), (d) et (f),
$R^{1a}$, $R^{2a}$, $R^{1b}$, et $R^{2b}$ étant chacun indépendamment choisis dans le groupe constitué par halogéno, $C_{1-3}$alkyle, polyhalogéno$C_{1-3}$alkyle, $C_{1-3}$alkyloxy, et polyhalogéno$C_{1-3}$alkyloxy ;
$R^{3a}$ étant hydrogène ;
$R^{4a}$ étant choisi dans le groupe constitué par hydrogène, halogéno, -CN, $C_{1-3}$alkyle, polyhalogéno$C_{1-3}$alkyle, $C_{1-3}$alkyloxy, et polyhalogéno$C_{1-3}$alkyloxy ;
$X^1$ et $X^2$ étant chacun indépendamment choisis parmi N et CH, à la condition qu'au moins l'un parmi $X^1$ ou $X^2$ soit N ;
$R^{1d}$ et $R^{2f}$ étant chacun indépendamment choisis parmi $C_{1-3}$alkyle ;
$X^3$ représentant CH ;
et chacun des cycles représentés par

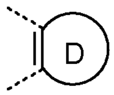 et 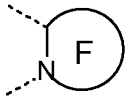 formant

(i) un hétérocycle insaturé à 5 ou 6 chaînons possédant un ou deux atomes d'azote, et qui est éventuellement substitué par un ou plusieurs substituants, chacun indépendamment choisis parmi halogéno et $C_{1-3}$alkyle ; ou
(ii) un hétérocycle aromatique à 5 ou 6 chaînons possédant un ou deux atomes d'azote, et qui est éventuellement substitué par un ou plusieurs substituants, chacun indépendamment choisis parmi $C_{1-3}$alkyle.

**4.** Composé selon l'une quelconque des revendications 1 à 3,

$R^1$ étant -$C_{1-3}$alkyl-C (O) -$NR^xR^y$, -$NR^xR^y$ étant choisi dans le groupe constitué par
-$NH_2$, -$NHCH_3$, -$NH(CH_3)_2$, -$N(CH_3)$ $(CH_2CH_3)$, azétidin-1-yle, et pyrrolidin-1-yle.

**5.** Composé selon l'une quelconque des revendications 1 à 4,
$R^2$, $R^3$ et $R^5$ étant chacun indépendamment choisis parmi hydrogène et méthyle.

**6.** Composé selon l'une quelconque des revendications 1 à 5,
$R^{1a}$, $R^{2a}$, $R^{1b}$, et $R^{2b}$ étant chacun indépendamment choisis dans le groupe constitué par fluoro, chloro, méthyle, isopropyle, $CF_3$, -$OCH_3$ et -$OCF_3$.

**7.** Composé selon l'une quelconque des revendications 1 à 6,
$X^1$ étant N et $X^2$ étant CH, ou $X^1$ étant CH et $X^2$ étant N, ou $X^1$ et $X^2$ étant tous deux N.

**8.** Composé selon l'une quelconque des revendications 1 à 7, $R^{1d}$, et $R^{2f}$ étant chacun indépendamment méthyle ou isopropyle.

**9.** Composé selon la revendication 1, le composé étant

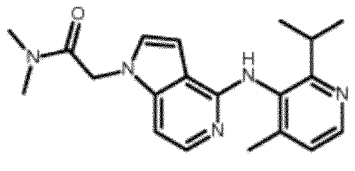

1

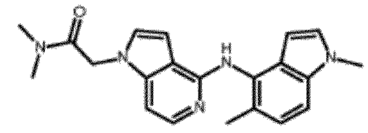

2

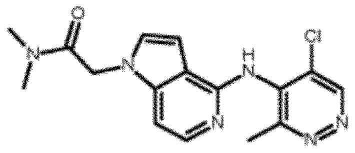

3

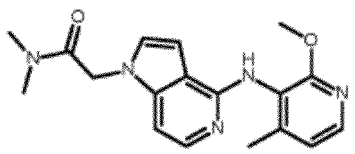

4

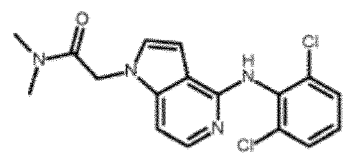

5

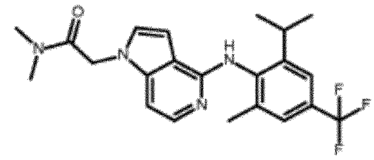

6

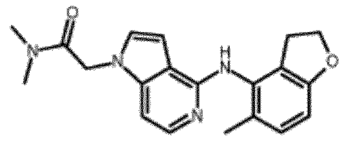

7

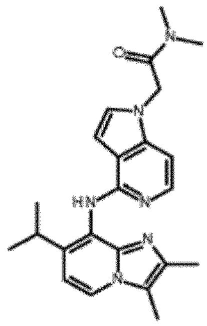

8

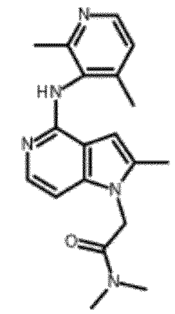

9

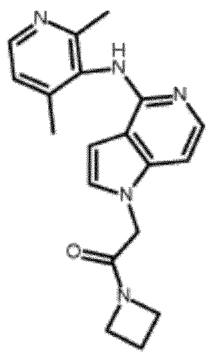

10

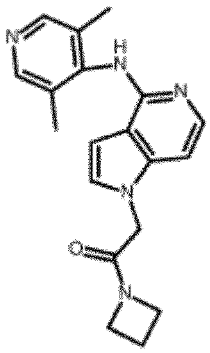

11

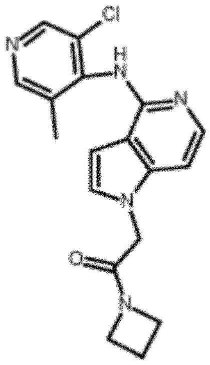

12

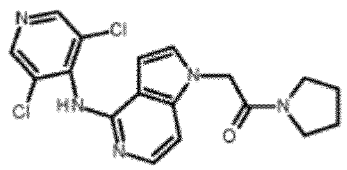

13

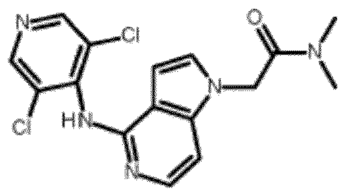

14

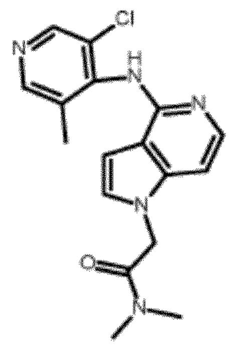

15

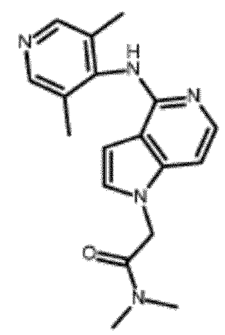

16

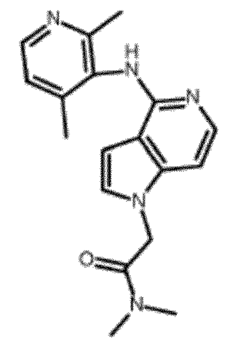

17

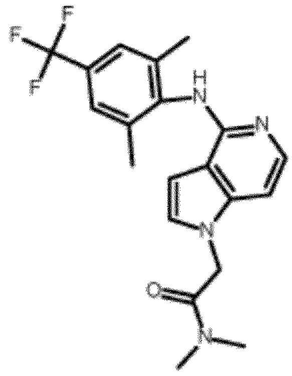

18

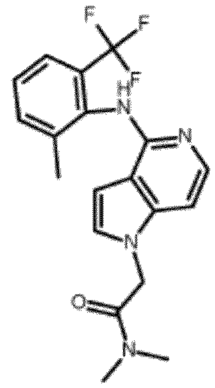

21

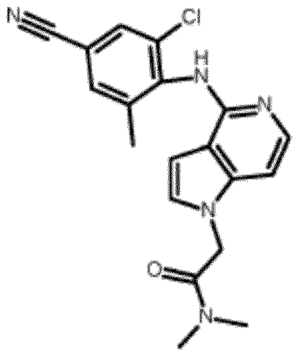

19

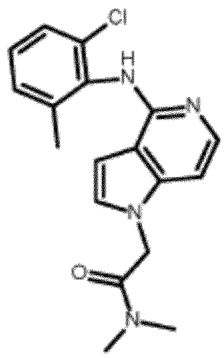

22

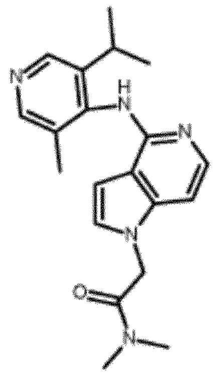

20

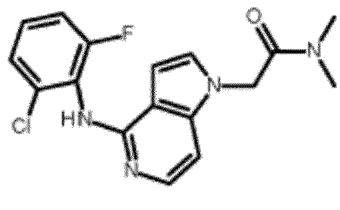

23

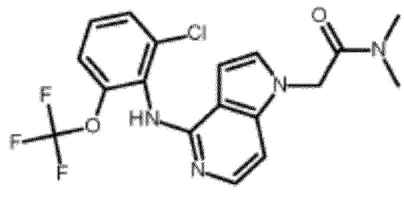

24

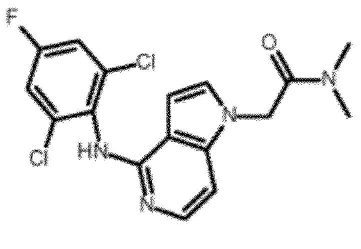

25

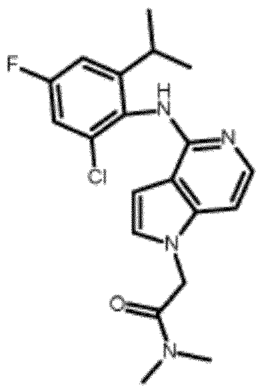

26

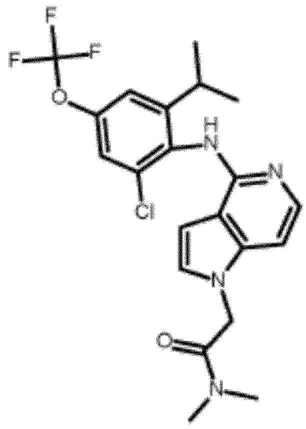

27

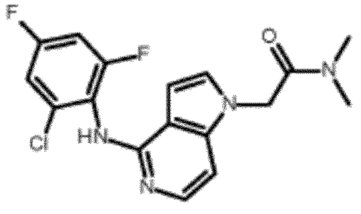

28

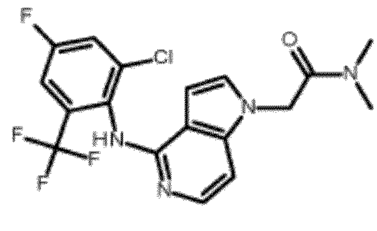

29

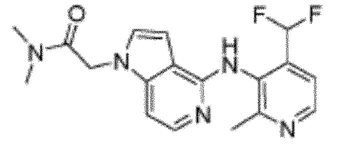

38

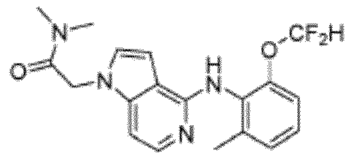

39

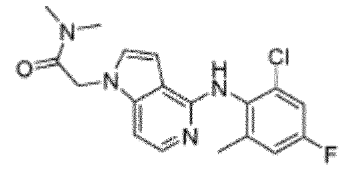

40

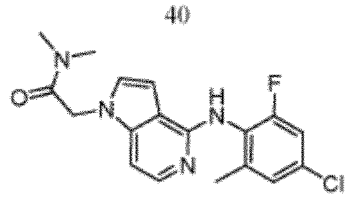

41

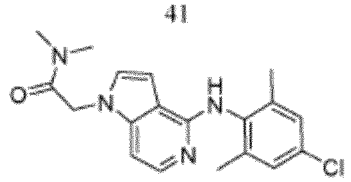

42

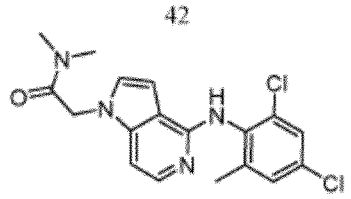

43

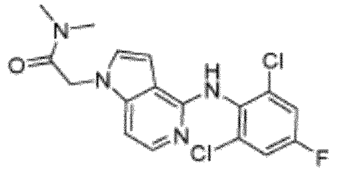

44

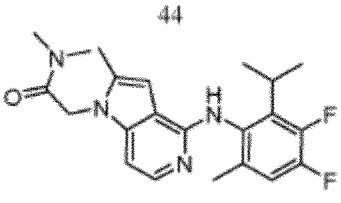

45

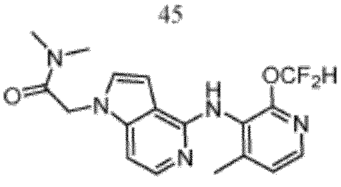

46

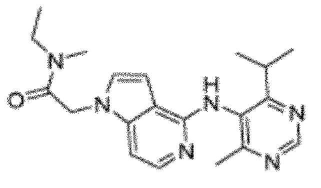

47

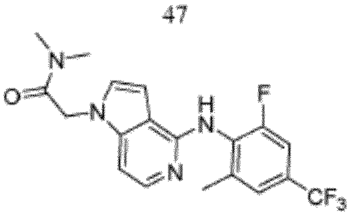

48

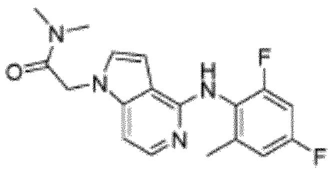

49

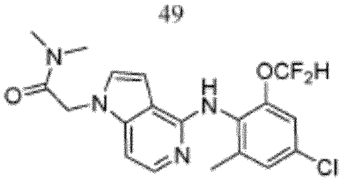

50

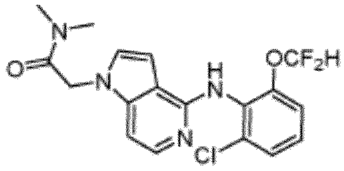

51

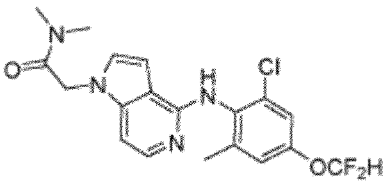

52

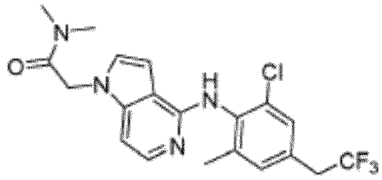

53

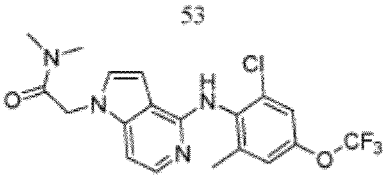

54

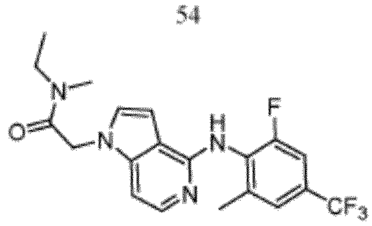

55

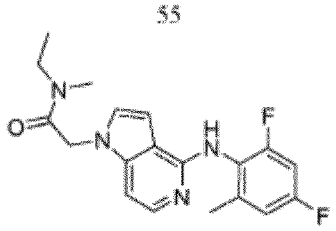

56

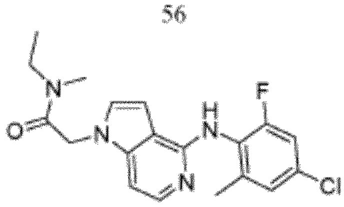

57

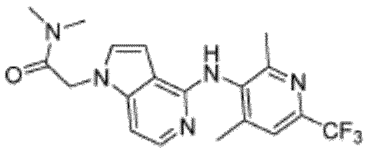

58

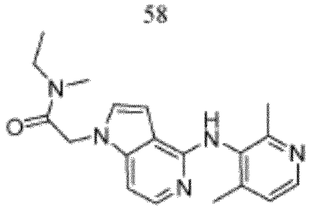

59

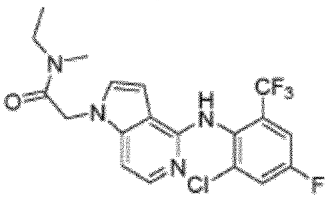

60

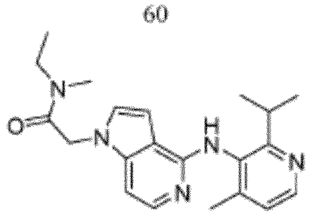

61

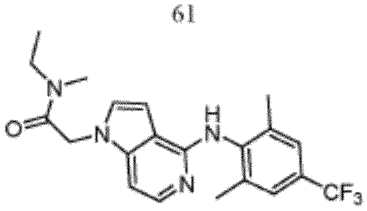

62

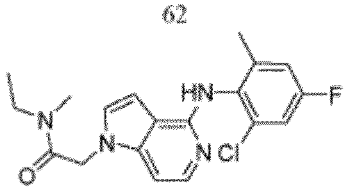

63

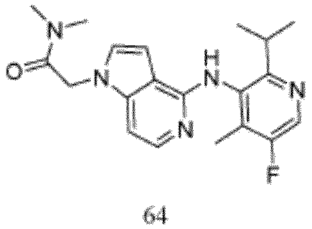

64

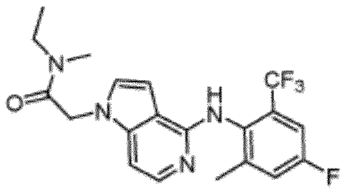

65

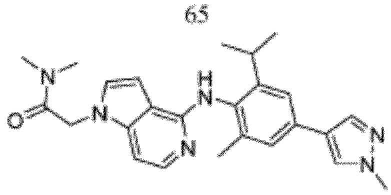

66

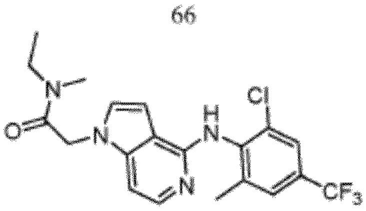

67

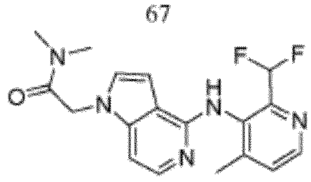

68

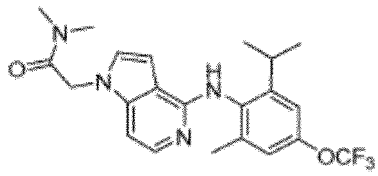

69

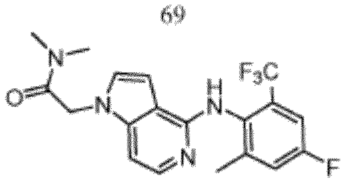

70

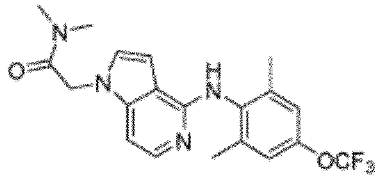

71

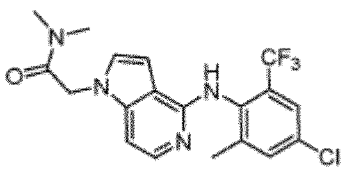

72

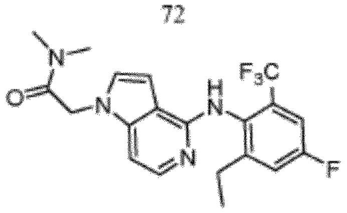

73

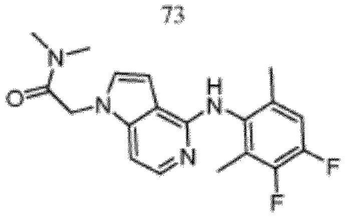

74

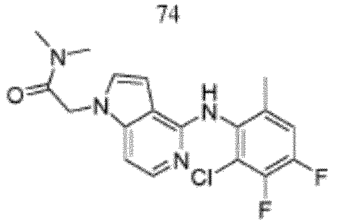

75

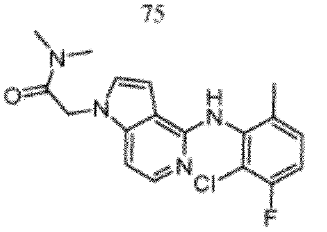

76

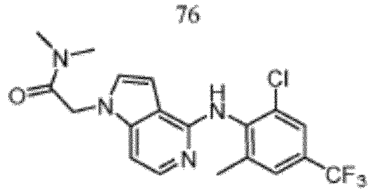

77

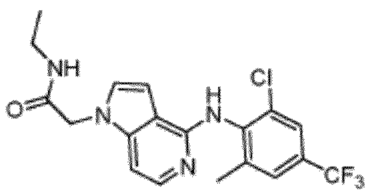

78

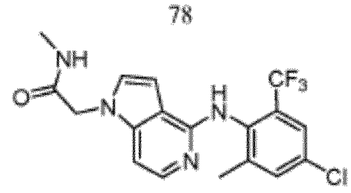

79

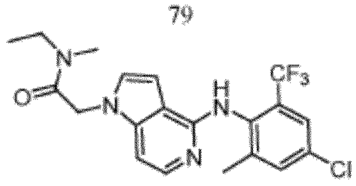

80

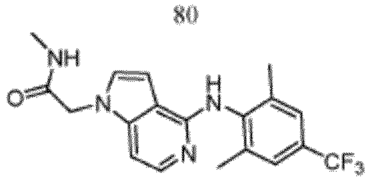

81

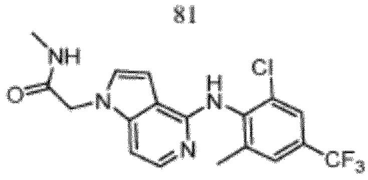

82

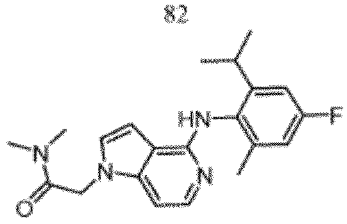

83

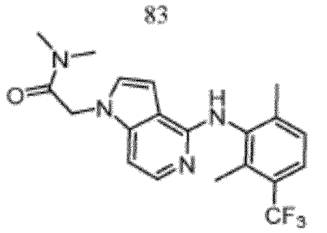

84

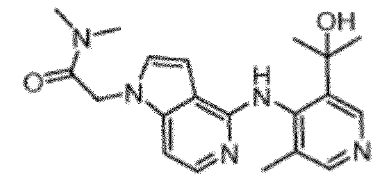

**Composé n° 85**

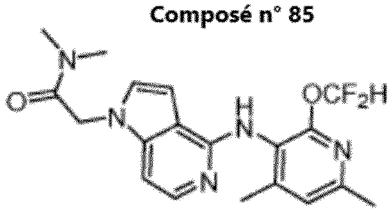

86

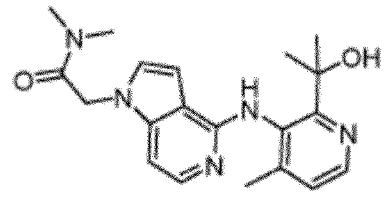

87

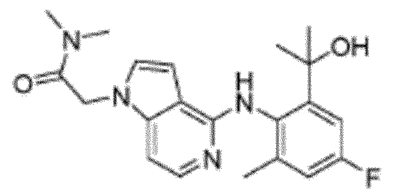

88

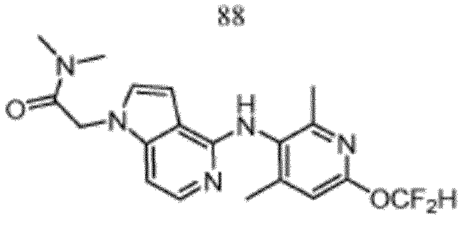

89

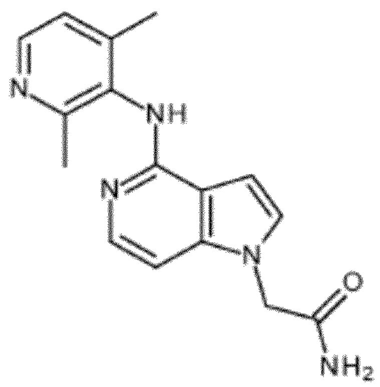

30

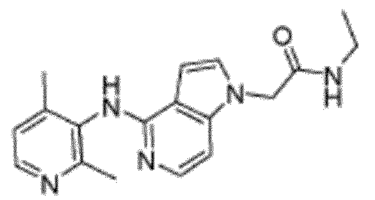

90

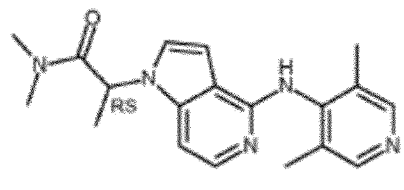

31

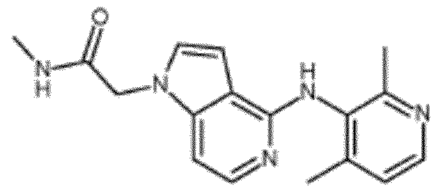

32

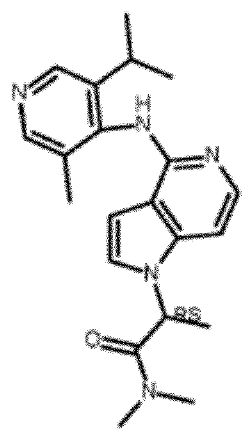

33

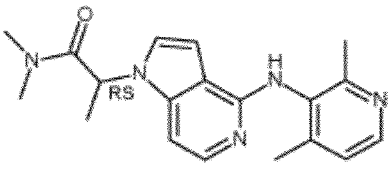
34

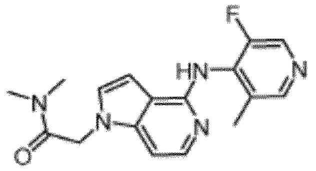
93

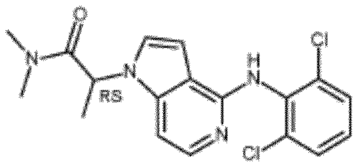
35

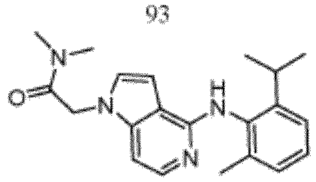
94

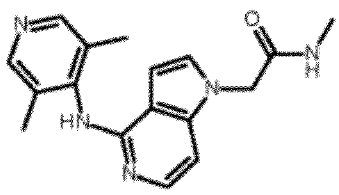
36

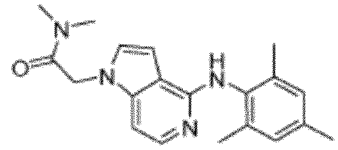
95

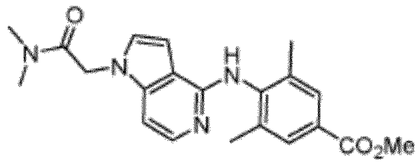
96

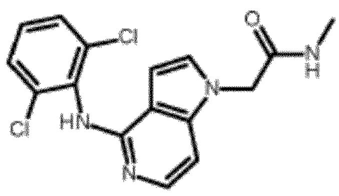
37

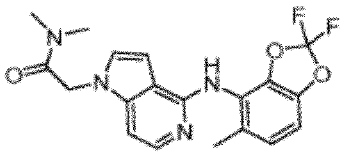
97

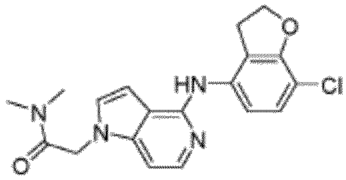
**91**

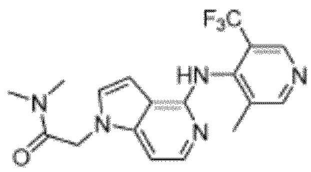
98

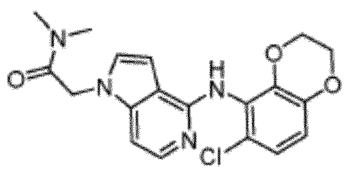
92

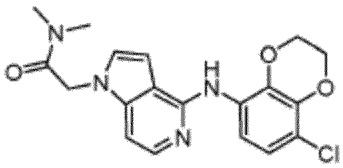
99

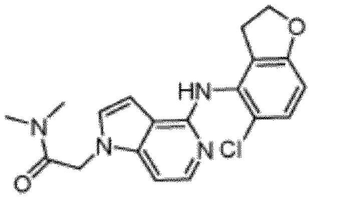
100

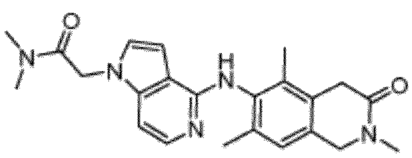
106

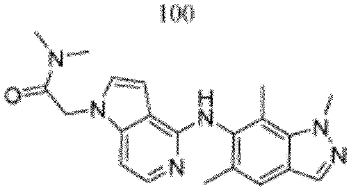
101

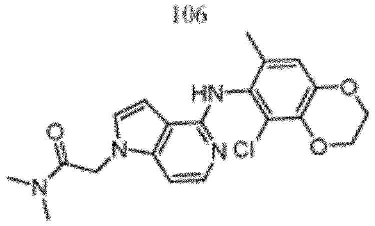
107

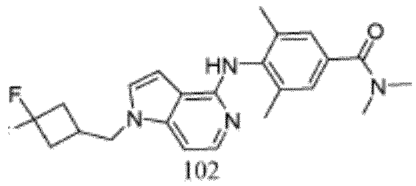

102

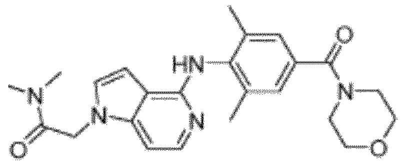

103

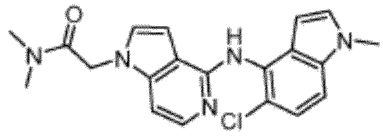

104

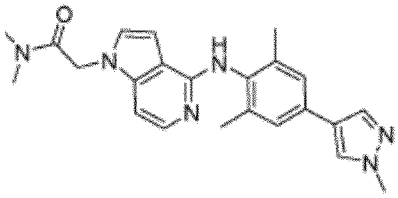

105

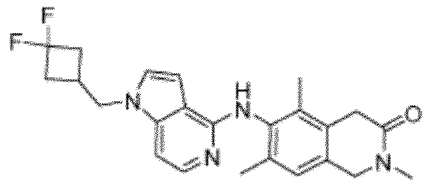

108

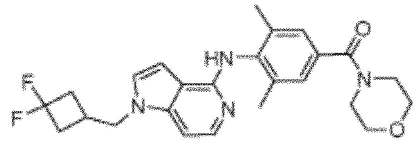

109

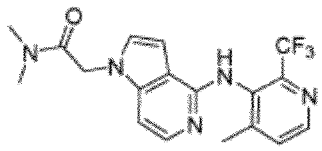

110

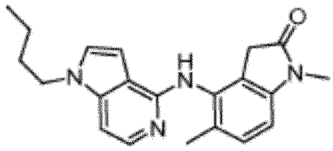

111

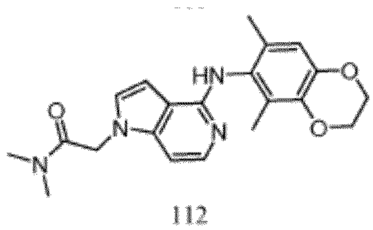

112

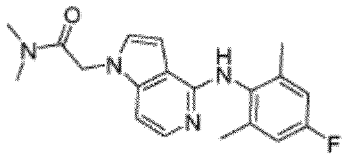

113

ou

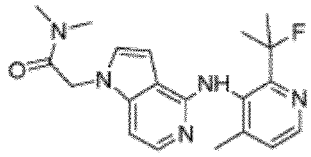

114

ou un sel pharmaceutiquement acceptable ou un solvate correspondant.

**10.** Composé selon la revendication 9, le composé étant

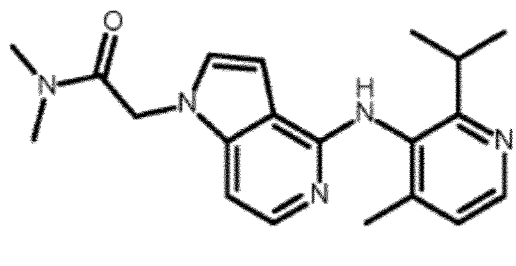

1,

ou un sel pharmaceutiquement acceptable ou un solvate correspondant.

**11.** Composé selon la revendication 9, le composé étant

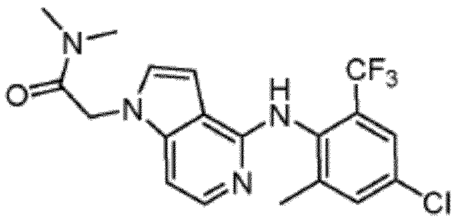 72,

ou un sel pharmaceutiquement acceptable ou un solvate correspondant.

**12.** Composé selon la revendication 9, le composé étant

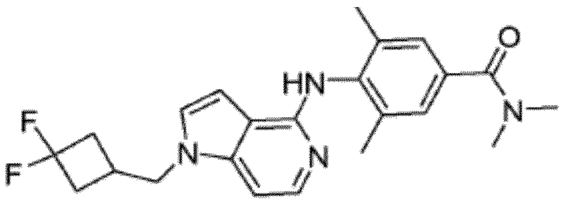

HCl
102

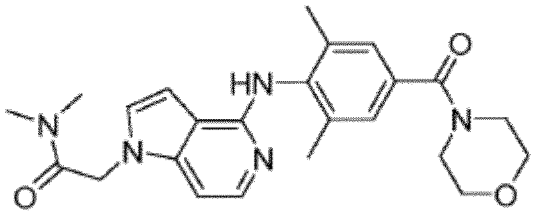

HCl
103

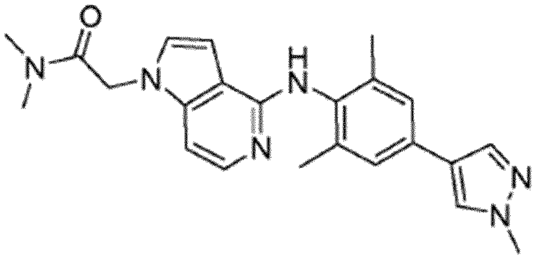

HCl
105

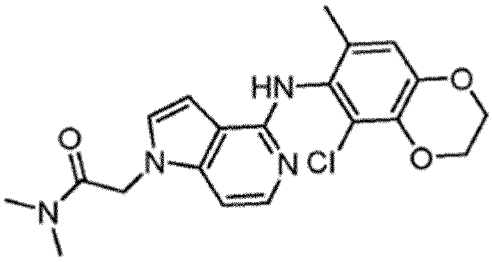

HCl
107

ou

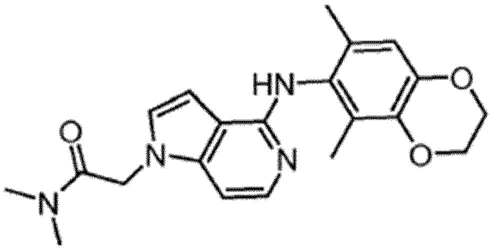

HCl
112.

13. Composition pharmaceutique comprenant une quantité efficace prophylactiquement ou une quantité efficace thérapeutiquement d'un composé selon l'une quelconque des revendications 1 à 12 et un support pharmaceutiquement acceptable.

14. Procédé pour la préparation d'une composition pharmaceutique comprenant le mélange d'un support pharmaceutiquement acceptable avec une quantité efficace prophylactiquement ou une quantité efficace thérapeutiquement d'un composé selon l'une quelconque des revendications 1 à 12.

15. Composé tel que défini dans l'une quelconque des revendications 1 à 12, ou composition pharmaceutique telle que définie dans la revendication 13, pour une utilisation en tant que médicament.

16. Composé tel que défini dans l'une quelconque des revendications 1 à 12, ou composition pharmaceutique telle que définie dans la revendication 13, pour une utilisation dans le traitement ou la prévention de la maladie d'Alzheimer, d'une paralysie supranucléaire progressive, du syndrome de Down, d'une démence du lobe frontotemporal, d'une démence frontotemporale avec parkinsonisme-17, de la maladie de Pick, d'une dégénérescence corticobasale, d'une maladie à grains argyrophiles, d'une sclérose latérale amyotrophique, ou d'une démence du lobe frontotemporal causée par des mutations C9ORF72.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2012117219 A
- WO 2014159234 A
- WO 20160300443 A
- WO 2017144633 A
- WO 20170114639 A
- WO 2017144637 A
- WO 2017106254 A
- WO 2018055316 A


### Non-patent literature cited in the description


- **DUBOIS B et al.** *Lancet Neurol,* 2014, vol. 13, 614-629
- **SPERLING, RA et al.** *Alzheimers Dement.,* 2011, vol. 7, 280-292
- *Alzheimer's & Dementia,* 2016, vol. 12, 292-323
- **MARIAPPA et al.** *Biochem J,* 2015, vol. 470, 255
- **YUZWA et al.** *Nature Chemical Biology,* 2008, vol. 4, 483
- **YUZWA et al.** *Nature Chemical Biology,* 2012, vol. 8, 393
- **DORFMUELLER et al.** *Chemistry & biology,* 2010, vol. 17, 1250
- **PARK et al.** *The Journal of biological chemistry,* 2014, vol. 289, 13519